(19) **Europäisches Patentamt**

**European Patent Office**

**Office européen des brevets**

(11) **EP 1 295 121 B1**

(12) **EUROPEAN PATENT SPECIFICATION**

(45) Date of publication and mention
of the grant of the patent:
**08.03.2006 Bulletin 2006/10**

(51) Int Cl.:
***G01N 33/53*** (2006.01)

(21) Application number: **00986922.3**

(86) International application number:
**PCT/CA2000/001516**

(22) Date of filing: **22.12.2000**

(87) International publication number:
**WO 2001/046691 (28.06.2001 Gazette 2001/26)**

(54) **A BIOLUMINESCENCE RESONANCE ENERGY TRANSFER (BRET) SYSTEM WITH BROAD SPECTRAL RESOLUTION BETWEEN DONOR AND ACCEPTOR EMISSION WAVELENGTHS AND ITS USE**

EIN BIOLUMINESZENZ RESONANZ ENERGIE TRANSFER (BRET) SYSTEM MIT BREITEM SPEKTRALEN RESOLUTION ZWISCHEN DONATOR- UND AKZEPTOR-EMISSION WELLENLÄNGE UND IHRE ANWENDUNG

SYSTEME DE TRANSFERT D'ENERGIE PAR RESONANCE BIOLUMINESCENTE (BRET) A RESOLUTION SPECTRALE LARGE ENTRE DES LONGUEURS D'ONDES D'EMISSION DE DONNEUR ET D'ACCEPTEUR, ET UTILISATION ASSOCIEE

(84) Designated Contracting States:
**AT BE CH CY DE DK ES FI FR GB GR IE IT LI LU MC NL PT SE TR**

(30) Priority: **22.12.1999 CA 2291968**
**02.08.2000 CA 2314861**

(43) Date of publication of application:
**26.03.2003 Bulletin 2003/13**

(73) Proprietor: **PerkinElmer BioSignal Inc.**
**Montreal, QC H3J 1R4 (CA)**

(72) Inventor: **JOLY, Erik**
**Blainville, Quebec J7C 5R1 (CA)**

(74) Representative: **Lee, Nicholas John et al**
**Kilburn & Strode,**
**20 Red Lion Street**
**London WC1R 4PJ (GB)**

(56) References cited:
- **KENDALL ET AL: "Aequorea victoria bioluminescence moves into an exciting new era" TRENDS IN BIOTECHNOLOGY, vol. 16, May 1998 (1998-05), pages 216-224, XP004117786**
- **XU, YAO ET AL: "A bioluminescence resonance energy transfer (BRET) system: Application to interacting circadian clock proteins" PROC. NATL. ACAD. SCI., vol. 96, no. 1, January 1999 (1999-01), pages 151-151-156, XP002127270**
- **WU AND BRAND: "Resonance Energy Transfer: Methods and Applications" BIOCHEMISTRY, no. 218, 1994, pages 1-13, XP002033685**

**Description**

**FIELD OF THE INVENTION**

The present invention pertains to the field of *in vitro* detection methods.

**BACKGROUND**

Interactions between proteins and other molecules play a key regulatory role in almost every biological process. Thus, many techniques have been developed to identify and characterise these interactions. One tool, using bioluminescence resonance energy transfer (BRET) is based on the use of Renilla luciferase (Rluc) as a donor and a Green Fluorescent protein (GFP) as an acceptor molecule in a fluorescence resonance energy transfer (FRET)-like assay configuration.

One BRET system has been developed in which Rluc and EYFP (Enhanced Yellow Fluorescent Protein) were used. Rluc is an enzyme that produces blue light (peak at 480 nm) in the presence of its substrate wild type coelenterazine (Lorenz *et al*, 1996 ; Lorenz *et al*, 1991; Matthews *et al*, 1977a). When in the appropriate spatial orientation the EYFP is able to absorb a portion of the blue light and subsequently emits light at a different wavelength. This phenomenon, therefore, can be adapted to monitor interactions between molecules.

Currently, various coelenterazine derivatives are commercially available (Molecular Probes Eugene OR ; Bio- Synth Napierville IL). Compared to native coelenterazine, most of the coelenterazine derivatives have improved brightness when they are processed by a bioluminescent enzyme such as Rluc or a photoprotein like Aequorin. Other derivatives (e.g. *hcp*) change the position of the emission peak. EYFP is a red-shifted GFP (Tsien, 1998). When excited at 513nm, it emits yellow light at 527 nm. Coupled together (e. g. as a fusion protein and in a protein-protein interaction assay), Rluc and EYFP perform BRET where part of the energy (non-radiative) from Rluc excites the EYFP. Therefore on addition of the substrate coelenterazine, EYFP emits yellow light if the Rluc and EYFP proteins are close together(<100 Å). If Rluc and EYFP are too far apart, energy is not efficiently transferred and only the blue light of Rluc is detected. Hence, to quantify energy transfer efficiencies light outputs are measured at the donor wavelength and acceptor wavelength after addition of the coelenterazine. Subsequently the ratio of acceptor light output to donor light output is calculated in order to quantify BRET.

The present applicant and others have demonstrated, using Rluc and EYFP (see Xu *et al*, 1999 and Angers *et al*, 2000 ; US Patent 5976796) that the use of BRET in drug discovery applications is feasible and that BRET systems have the potential to become excellent detection systems for live cell assays. This BRET system, however, exhibits a relatively low signal-to-base (S/B) and dynamic range (or DR : the difference between the maximal ratio and the minimal ratio), approximately 1.4 and 0.5, respectively. Furthermore, because of the small difference between donor and acceptor emission wavelengths, there is always some donor light which is emitted in the wavelength range of the acceptor emission, which causes an increase in background light.

This background information is provided for the purpose of making known information believed by the applicant to be of possible relevance to the present invention. No admission is necessarily intended, nor should be construed, that any of the preceding information constitutes prior art against the present invention.

SUMMARY OF THE INVENTION

An object of the present invention is to provide a bioluminescence resonance energy transfer (BRET) system with broad spectral resolution between donor and acceptor emission wavelengths and its use. In one aspect, the present invention provides a bioluminescence resonance energy transfer (BRET) system comprising:

   (a) a bioluminescent donor protein (BDP) attached to a first molecule or modulator;
   (b) a fluorescent acceptor molecule (FAM) attached to a second molecule or modulator, wherein said FAM can accept the energy from the BDP when they are associated, in the presence of the appropriate substrate; and

wherein a physical change in the modulator(s) influences the energy transfer efficiency between the BDP and the FAM and wherein said system has a broad spectral resolution of at least 80 nm between the peaks of the BDP and FAM emission spectra.

In one embodiment, the modulator may comprise two separate molecular entities attached to the FAM and BDP, respectively. In this embodiment, the interaction between the molecular entities may be dependent on the activity of another molecule, and may be constitutive. The two separate molecular entities may be the same or may be distinct. One or both of the molecular entities may be a receptor (which may be an orphan receptor), receptor subunit or fragment thereof.

In another embodiment, one of the molecular entities may be a receptor binding molecule that will bind to an activated receptor and the other may be a receptor, receptor subunit or fragment thereof. The receptor binding molecule may be β-arrestin, a G-protein or ubiquitin.

In a further embodiment of the first aspect, the modulator, FAM and BDP may comprise one fusion molecule.

In another embodiment, the BRET system of the first aspect additionally comprises an instrument for detecting BRET signal capable of determining specifically BDP and/or FAM emissions. One or more optical filters may be incorporated into said instrument to reduce overlap between the emission spectra and produce the broad spectral resolution. The instrument may be a microplate plate reader, or an imaging system, and one or more monochromators or prisms may be incorporated into said instrument to reduce overlap between said emission spectra and produce said broad spectral resolution.

The instrument may be a spectrophotometer or a spectrofluorometer, and an algorithm may be used to mathematically subtract BDP emission present in the FAM peak and produce the broad spectral resolution.

In a further embodiment, the BRET system according to the first aspect may have luciferase activity in the presence of an appropriate substrate. The luciferase can be *Renilla* luciferase or firefly luciferase, Gaussia luciferase, Aequorin, and a broad spectral resolution can be attained by using a non-naturally occurring luciferase.

An appropriate substrate can be used to activate the luminescent activity of the BDP, which may be a non-naturally occurring substrate, such as a derivative of coelenterazine, (e.g. coel400a).

The broad spectral resolution of the first aspect may be attained by using a non-naturally occurring FAM, which may be a mutant green fluorescent protein or a mutant red fluorescent protein. The mutant green fluorescent protein may comprise the following substitutions:

(a) phenylalanine-64 to leucine;
(b) phenylalanine-64 to leucine, serine-147 to proline;
(c) phenylalanine-64 to leucine and serine-202 to phenylalanine
(d) phenylalanine-64 to leucine, serine-147 to proline, and serine-202 to phenylalanine;
(e) phenylalanine-64 to leucine, serine-147 to proline, and tyrosine-203 to isoleucine; or
(f) phenylalanine-64 to leucine, serine-147 to proline, serine-202 to phenylalanine and tyrosine-203 to isoleucine.

In a second aspect, the invention provides the use of the BRET system of the first aspect to monitor protein-protein interactions *in vitro.*

In a third aspect, the invention provides a method of producing a BRET system of the first aspect comprising:

(a) selecting a bioluminescent donor protein (BDP) and a fluorescent acceptor molecule (FAM) such that the FAM can accept the energy from the BDP when they are associated, in the presence of the appropriate substrate; and
(b) either

(i) using an algorithm to mathematically subtract BDP emission from the FAM peak,
(ii) reducing overlap between the BDP and FAM emission spectra by using optical filters,
(iii) using site-directed mutagenesis to alter the emission spectrum of the BDP,
(iv) using a coelenterazine derivative, luciferin or bioluminescent substrate that causes a shift in the BDP emission spectrum, or
(v) using site-directed mutagenesis to alter the excitation and/or emission spectrum of the FAM,

wherein said system has a broad spectral resolution of at least 80nm between the peaks of the BDP and FAM emission spectra.

## BRIEF DESCRIPTION OF THE FIGURES

Figure 1 is a pictorial description of one embodiment of a BRET system wherein the BDP and the FAM are attached to different proteins or peptides. In this embodiment, there are generally two entities comprising the modulator, wherein the BDP is covalently attached to one modulator entity and the FAM is covalently attached to the second modulator entity. The interaction between the two modulator entities influences the energy transfer efficiency between the BDP and the FAM, and therefore the light emission from the system.

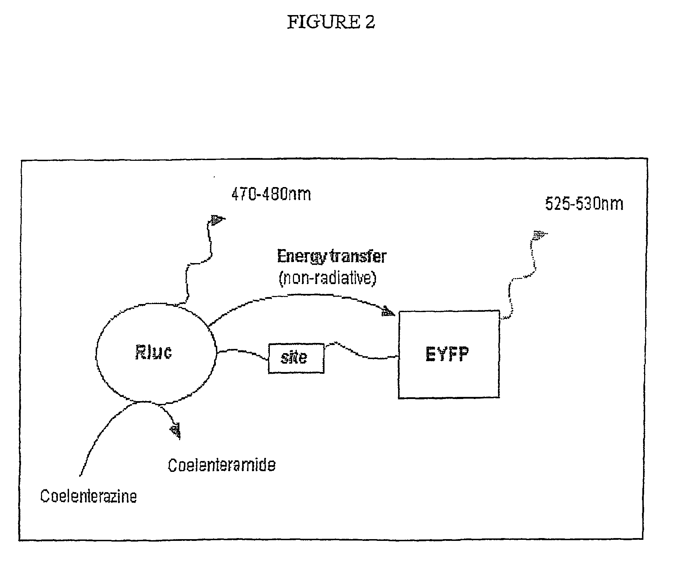

Figure 2 is a pictorial description of another embodiment of BRET wherein the BDP and the FAM are attached to the same protein or peptide. In this embodiment, the BDP and the FAM are attached to a common linker comprising the modulator component of the system. One typical example of this embodiment is the use of the modulator as a sensor that will detect changes in a component in the environment, causing the sensor to undergo a change that affects the energy transfer efficiency between the BDP and the FAM, that is reflected in the light emission from the system.

Figure 3 presents the results of an experiment demonstrating a calcitonin dose response on CHO-K1 cell transfected with the cAMP sensor (CBP:CREB constructs).

Figure 4 shows an isoproterenol dose response on CHO-K1 cell transfected with the cAMP sensor (CBP:CREB constructs). In this case, the ratio has been corrected using the alogrithm formula presented in Example II.

Figure 5 presents spectral analysis of the Coel 400A. Cells were transiently transfected with the appropriate constructs using LipofectAMINE. Two days after transfection, cells were used to perform emission scans using a spectrofluorometer with the excitation light turns off.

Figure 6 shows BRETCount analysis of the coelenterazine 400A. Two days after transfection, cells were distributed into microplate and analysed in the BRETCount. (—■— Rluc 400a; —▲— Rluc-sph 400a; and —▼— Rluc-GFPuv 400a)

Figure 7 shows BRETCount analysis of the Coel 400A using black plate. (—■— Rluc 400a; —▲— Rluc-sph 400a; and —▼— Rluc-GFPuv 400a)

Figure 8 shows the luminescence at t=0 (i.e. immediately after addition of the coelenterazine) for the various coelenterazine 400 A stocks.

Figure 9 shows the luminescence at t=5 (5 min. after addition of the coelenterazine) for the various Coel 400 A stocks.

Figure 10 demonstrates a comparison study between wt Rluc and hRluc in live cell. Cells were transfected with the appropriate constructs using LipofectAMINE along with an internal standard. Two days after transfection, cells were harvested and used to performed emission scans using a spectrofluorometer. (—hRLuc # 4; ----- hRLuc # 6; and-RLuc)

Figure 11 shows BRETCount analysis of the hRluc expression in live cell. (—■—pRLuc ; —◇—phRLuc clone # 4; and —▲—phRLuc clone #6)

Figure 12 shows BRETCount analysis of the GFP mutants. Cells were transfected with the appropriate constructs using LipofectAMINE. Two days after transfection, cells were harvested and seeded in microplate before analyzing them in the BRETCount. (—■— pRLuc; —○— pRLuc-GFPuv; —▲— pRLuc-GFP1; —□— pRLuc-GFP6; and —•— pRLuc-GFP9)

Figure 13 shows BRETCount analysis of the GFP mutants. Cells were transfected with the appropriate constructs using LipofectAMINE. Two days after transfection, cells were harvested and seeded in microplate before analyzing them in the BRETCount. (—■— pRLuc; —▼— pRLuc-GFPuv; —▽— pRLuc-GFP9; —♦— pRLuc-GFP10; and —○— pRLuc-GFP11)

Figure 14 shows BRETCount analysis of the GFP mutants. Cells were transfected with the appropriate constructs using LipofectAMINE. Two days after transfection, cells were harvested and seeded in microplate before analyzing them in the BRETCount. Effect of DTT on the ratios. (—■— pRLuc; —○— pRLuc-GFPuv; —▲— pRLuc-GFP1; —♦— pRLuc-GFP9; —•— pRLuc-GFP 10; and —□— pRLuc-GFP11)

Figure 15 shows a comparison between *hcp* and Coe1400a derivatives in CHO cells when analyzed using the BRETCount. (—■— pRLuc Coe1400a; —□— pRLuc-GFP 10 Coel 400a; —▲— pRLuc *hcp*; and —▼— pRLuc-GFP10 *hcp*)

Figure 16 shows spectral analysis of GFP10. Cells were transfected with either pRluc::GFP10 (GFP#10) or pRluc:: GFP11 (GFP#11). Two days after transfection, cells were used to perform emission scans using the coe1400a. (—GFP # 10 and ——— GFP # 11)

Figure 17 demonstrates a comparison between cells expressing Rluc, Rluc+GFP10 and the fusion Rluc::GFP10. a) cells were not lysed, b) cells were lysed. (—■— Rluc; —▲— Rluc + GFP10; and —▼— Rluc::GFP10)

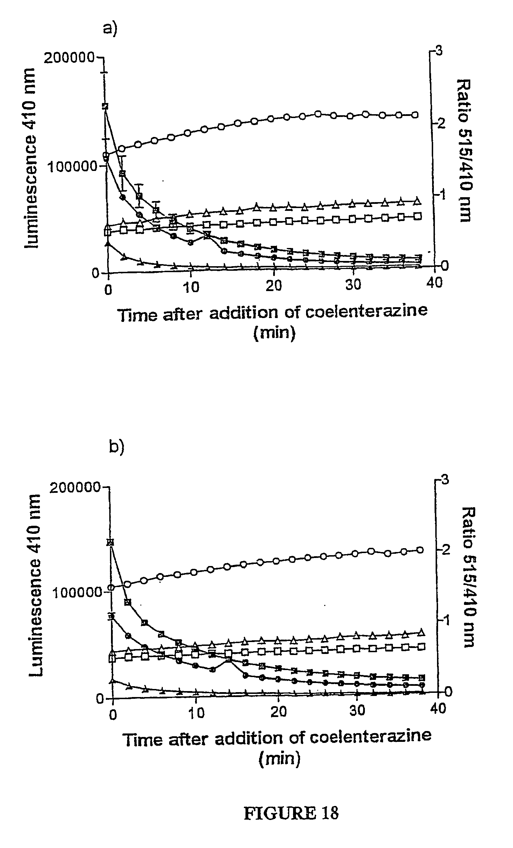

Figure 18 demonstrates the use of the BRET system of the present invention in protein-protein interaction assays. Cells were transfected or co-transfected with the mentioned constructs. Forty-eight hours post-transfection, cells were harvested and seeded in 96-well plate (Optiplate white) at a density of 50 000 cells per well in BRET buffer. a) assays was performed without DTT. B) assay was performed with DTT. (—■— Rluc::kaiB (luminescence); —•— Rluc::kaiB/GFP::kaiB (luminescence); —▲— Rluc;;kaiB/GFP::kaiA (luminescence); —□— ratio Rluc::kaiB; —○— ratio Rluc::kaiB/GFP::kaiB; and —△— ratio Rluc::kaiB/GFP::kaiA)

Figure 19 shows the result of the use of the emission filter pair GG475 (acceptor) ana D410/80 (donor) using cells expressing Rluc, Rluc::GFP1 or Rluc::GFP10 fusion constructs. (—■— Rluc; —◆— Rluc::GFP1; and —▲— Rluc:: GFP10)

Figure 20 shows the result of the use of the emission filter pair D515/30 (acceptor) and D410/80 (donor) using cells expressing Rluc, Rluc::GFP1 or Rluc::GFP10 fusion constructs. (—■— Rluc; —◆— Rluc::GFP1; and —▲— Rluc:: GFP10)

Figure 21 shows the results of the use of the a BRET system of the present invention in the constitutive protein-protein interaction Kai system.

Figure 22 provides results from β-Adrenergic Receptor- and Vasopressin2-β-arrestin BRET assays. A) BRET ratios were determined in presence (+) or in absence (-) a saturating dose of agonists (iso : isoproterenol for β2 and β3 receptors; AVP for V2 receptor). B) Dose response curve of AVP for the V2 receptor only.

Figure 23 provides a comparison of the different coelenterazine derivatives. Cells were transfected with the negative and positive control and used in BRET[2] assays.

Figure 24 provides a comparison of the emission scan of the Rluc mutants and wild type Rluc. In all cases, DeepBlueC was used as substrate.

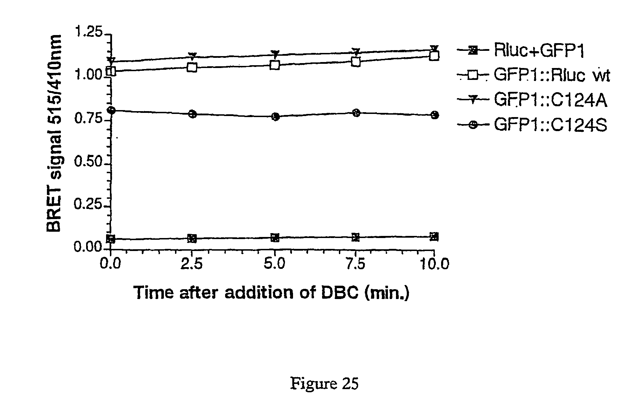

Figure 25 provides a comparison of the Rluc mutants to Rluc wild type in BRET assay.

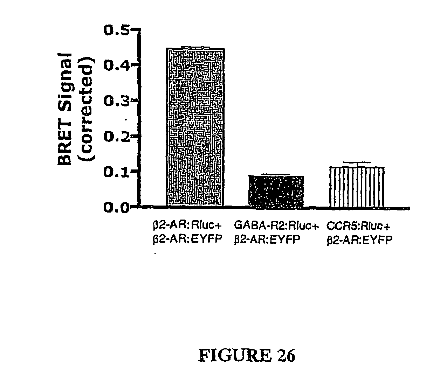

Figure 26 demonstrates results of GPCR oligomerization in crude membranes using BRET. β2-AR:EYFP was co-transfected with either β2-AR:Rluc or CCR5:Rluc or GABA-R2:Rluc. Membranes were prepared from transfected cells and used in BRET assays to determine GPCR oligomerization.

Figure 27 shows a DNA sequence (SEQ ID NO:) encoding the GFP:Rluc fusion protein containing a unique 14 amino acid linker region between the GFP and the Rluc.

Figure 28 shows the DNA sequence for GFP1:Caspase-3:Rluc construct (SEQ ID NO: ).

Figure 29 demonstrates apoptosis induction using staurosporine in HeLa cells transfected with the GFP1:Caspase-3:Rluc sensor.

Figure 30 shows effects of $Ca^{2+}$ on the interaction of Rluc:p53 and S100b:GFPuv fusion proteins. His-tag purified Rluc:p53 fusion protein was mixed with his-tag purified S100b:GFPuv fusion protein at different molecular ratio (1: 1 or 1:3) in presence (+) or absence (-) of 1mM $Ca^{2+}$.

Figure 31 demonstrates results of a comparison between the β-arrestin2:GFP1 and GFP1:βarrestin2 fusion constructs in BRET assay.

Figure 32 presents Scheme I: Synthesis of pyrazines

Figure 33 presents Scheme II: Synthesis of benzylglyoxal starting from phenylacetylchloride

Figure 34 presents Scheme III: Synthesis of phenylcoelentrazine.

Table 1: Molecular components of the first (BRET) and second BRET generation (BRET²).

Table 2: Other donor/substrate/acceptor combinations different from the one found in Table 1 having a > 50 nm spectral separation between donor and acceptor emission wavelengths. Minor peaks are shown in parenthesis. The spectral position peak can be dependent on the type of assay. For mammalian cells expressing Rluc, Rluc emits light between 470 - 480 nm when it processes wild type coelenterazine. In bacteria, Rluc emits light at lower wavelengths (460 - 470 nm) for the same substrate.

Table 3: Other bioluminescent donors that can be used according to the present invention. (* according to literature it was formerly called Cypridina; ** new derivative)

Table 4: Class 1 GFP mutants tested.(*allelic mutations were not considered ; EGFP is already codon humanised ;** (h) means codon humanised)

Table 5: Effect of the mutations. In all humanised version, a valine residue has been inserted after the first methionine in order to create a Kozak sequence.

Table 6: Light output measured in BRETCount and TopCount.(* mean of eight measurements)

Table 7: Examples of calculated ratios for both constructs using the different equations.

Table 8: Comparison of the bioluminescence activities of Rluc mutants.

Table 9: PCR amplification of human p53.

Table 10: RT-PCR amplification of human S100B.

## DETAILED DESCRIPTION OF THE INVENTION

In one aspect, the present invention provides a bioluminescence resonance energy transfer (BRET) system which has been modified to exhibit a broad spectral resolution between the donor and acceptor emission wavelengths. The purpose of the broad spectral separation that characterises the BRET system is to minimise the overlap between the BDP and FAM emission spectra and thereby reduce the affect of the BDP emission on the FAM emission. This, in turn, results in an improved signal-to-base ratio(S/B) and dynamic range (DR) in the BRET system of the present invention over a basic BRET system.

*Definitions*

Unless defined otherwise, all technical and scientific terms used herein have the same meaning as commonly understood by one of ordinary skill in the art to which this invention belongs. Generally, the nomenclature used herein and the laboratory procedures in spectroscopy, drug discovery, cell culture, molecular genetics, plastic manufacture, polymer chemistry, diagnostics, amino acid and nucleic acid chemistry, and sugar chemistry described below are those well known and commonly employed in the art. Standard techniques are typically used for preparation of plastics, signal detection, recombinant nucleic acid methods, polynucleotide synthesis, and microbial culture and transformation (e. g., electroporation, Calcium Chloride-heat shock).

The techniques and procedures are generally performed according to conventional methods in the art and various general references (see generally, Sambrook et al. Molecular Cloning: A Laboratory Manual, 2d ed. (1989) Cold Spring Harbor Laboratory Press, Cold Spring Harbor, N. Y., and Lakowicz, J. R. Principles of Fluorescence Spectroscopy, New York: Plenum Press (1983) for fluorescence techniques) which are provided throughout this document. Standard techniques are used for chemical syntheses, chemical analyses, and biological assays.

As employed throughout the disclosure, the following terms, unless otherwise indicated, shall be understood to have the following meanings:

"Bioluminescent donor protein (BDP)"refers to any protein capable of acting on a suitable substrate to generate luminescence. There are a number of different bioluminescent donor proteins that can be employed in this invention.

"Fluorescent acceptor molecule (FAM)" refers to any molecule which can accept energy emitted as a result of the activity of a bioluminescent donor protein, and re-emit it as light energy. There are a number of different fluorescent acceptor molecules that can be employed in this invention. The FAM may be proteinaceous or non-proteinaceous.

"Substrate" refers to any molecule that is employed by the bioluminescent donor protein to generate luminescence.

"Modulator" means, in its broadest sense, a molecule or molecules (proteins, lipids, polysaccharides, nucleic acids, organic molecules, inorganic molecules, etc.) that will undergo a physical change (eg. cleavage, phosphorylation, ligation, including orientation,conformational, and energy change) in response to an interaction with another factor suchas a molecule (called interacting factor), thereby affecting the energy transfer efficiency between the BDP and the FAM.

"Interacting factor (IF)," in its broadest sense, refers any "substance" including energy or a molecule (such as ions, second messenger, protein, protein domain, polypeptide or peptide) capable of interacting with the modulator resulting in a physical change in the modulator, ultimately affecting the energy transfer efficiency between the BDP and the FAM.

It is understood in the art that the BDP is an enzyme which converts a substrate into an activated product which then releases energy as it relaxes. Although the specification refers to the transfer of energy between the BDP and the FAM, it is understood that, technically, the activated product (generated by the activity of the BDP on the substrate) is the source of the BDP-generated luminescence that is transferred to the FAM. For the purpose of this invention, the orientation of the BDP relative to the FAM when it converts the substrate into the activated product is a crucial factor for the appropriate function of the invention.

"Protein" refers to a whole protein, or fragment thereof, such as a protein domain or a binding site for a second messenger, co-factor, ion, etc. It can be a peptide or an amino acid sequence that functions as a signal for another protein in the system, such as a proteolytic cleavage site.

"Binding pair" refers to two moieties (e. g. chemical or biochemical) that have an affinity for one another. Examples of binding pairs include homo-dimers, hetero-dimers, antigen/antibodies, lectin/avidin, target polynucleotide/ probe, oligonucleotide, antibody/anti-antibody, receptor/ligand, enzyme/ligand and the like. "One member of a binding pair" refers to one moiety of the pair, such as an antigen or ligand.

"Membrane-permeant derivative" refers to a chemical derivative of a compound that has enhanced membrane permeability compared to an underivativized compound. Examples include ester, ether and carbamate derivatives. These derivatives are made better able to cross cell membranes, i.e. membrane permeant, because hydrophilic groups are masked to provide more hydrophobic derivatives. Also, masking groups are designed to be cleaved from a precursor (e. g., fluorogenic substrate precursor) within the cell to generate the derived substrate intracellularly. Because the substrate is more hydrophilic than the membrane permeant derivative it is now trapped within the cells.

"Isolated polynucleotide" refers to a polynucleotide of genomic, cDNA, RNA or synthetic origin or some combination there of, which by virtue of its origin the "isolated polynucleotide" (1) is not associated with the cell in which the "isolated polynucleotide" is found in nature, or (2) is operably linked to a polynucleotide which it is not linked to in nature.

"Isolated protein" refers to a protein of cDNA, recombinant RNA, or synthetic origin or some combination thereof, which by virtue of its origin the "isolated protein" (1) is not associated with proteins it is normally found with in nature, or (2) is isolated from the cell in which it normally occurs or (3) is isolated free of other proteins from the same cellular source, e. g. free of human proteins, or (4) is expressed by a cell from a different species, or (5) does not occur in nature. "Isolated naturally occurring protein" refers to a protein which by virtue of its origin the "isolated naturally occurring protein" (1) is not associated with proteins that it is normally found with in nature, or (2) is isolated from the cell in which it normally occurs or (3) is isolated free of other proteins from the same cellular source, e. g. free of human proteins.

"Polypeptide" as used herein as a generic term to refer to native protein, fragments, or analogs of a polypeptide sequence. Hence, native protein, fragments, and analogs are species of the polypeptide genus.

"Naturally-occurring" as used herein, as applied to an object, refers to the fact that an object can be found in nature. For example, a polypeptide or polynucleotide sequence that is present in an organism (including viruses) that can be isolated from a source in nature and which has not been intentionally modified by man in the laboratory is naturally-occurring.

"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

"Control sequence" refers to polynucleotide sequences which are necessary to effect the expression of coding and non-coding sequences to which they are ligated. The nature of such control sequences differs depending upon the host organism; in prokaryotes, such control sequences generally include promoter, ribosomal binding site, and transcription termination sequences; in eukaryotes, generally, such control sequences include promoters and transcription termination sequences. The term "control sequences" is intended to include, at a minimum, components whose presence can influence expression, and can also include additional components whose presence is advantageous, for example, leader sequences and fusion partner sequences.

"Polynucleotide" refers to a polymeric form of nucleotides of at least 10 bases in length, either ribonucleotides or deoxynucleotides or a modified form of either type of nucleotide. The term includes single and double stranded forms of DNA, RNA and combinations thereof.

"Polypeptide fragment" refers to a polypeptide that has an amino-terminal and/or carboxy-terminal deletion, but where the remaining amino acid sequence is usually identical to the corresponding positions in the naturally-occurring sequence deduced, for example, from a full-length cDNA sequence. Fragments typically are at least 5, 6, 8 or 10 amino acids long, preferably at least 14 amino acids long, more preferably at least 20 amino acids long, usually at least 50 amino acids long, and even more preferably at least 70 amino acids long.

"Plate" refers to a multi-well plate, unless otherwise modified in its context.

The term "test chemical" or "test compound" refers to a chemical to be tested by one or more screening method (s) of the invention as a putative candidate.

The terms "label" or "labeled" refers to incorporation of a detectable marker, e. g., by incorporation of a radiolabeled amino acid or attachment to a polypeptide of biotinyl moieties that can be detected by marked avidin (e. g., streptavidin containing a fluorescent marker or enzymatic activity that can be detected by optical or colorimetric methods). Various methods of labeling polypeptides and glycoproteins are known in the art and may be used. Examples of labels for polypeptides include, but are not limited to, the following :radiosotopes (e.g., $^3H$, $^{14}C$, $^{35}S$, $^{125}I$, $^{131}I$), fluorescent labels (e.g., FITC, rhodamine, lanthanide phosphors), enzymatic labels (or reporter genes) (e.g., horseradish peroxidase, .beta.-galactosidase,. beta.-latamase, luciferase, alkaline phosphatase),chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

"Analyte" refers to a substance for which the presence, absence, or quantity is to be determined. An analyte is typically a small molecule or ionic solute such as $K^+$, $H^+$, $Ca^{2+}$, $CO_2$, $Na^+$, $Cl^-$, $Mg^2$, $O_2$, $HCO_3$, NO, and ATP.

Common examples of second messengers include cAMP, cGMP, $Ca^{2+}$, $IP_3$, NO, DAG, ceramide and derivatives thereof, arachidonic acid and derivatives thereof and isoprenyl and derivatives thereof.

Other chemistry terms herein are used according to conventional usage in the art, as exemplified by The McGraw-Hill Dictionary of Chemical Terms (ed. Parker, S., 1985), McGraw-Hill, San Francisco.

In one aspect, the present invention provides a bioluminescence resonance energy transfer (BRET) system with a broad spectral resolution between the donor and acceptor emission wavelengths. The purpose of the broad spectral separation that characterises the BRET system is to minimise the overlap between the BDP and FAM emission spectra and thereby reduce the affect of the BDP emission on the FAM emission. This, in turn, results in an improved signal-to-base ratio (S/B) and dynamic range (DR) in the BRET system of the present invention over a basic BRET system.

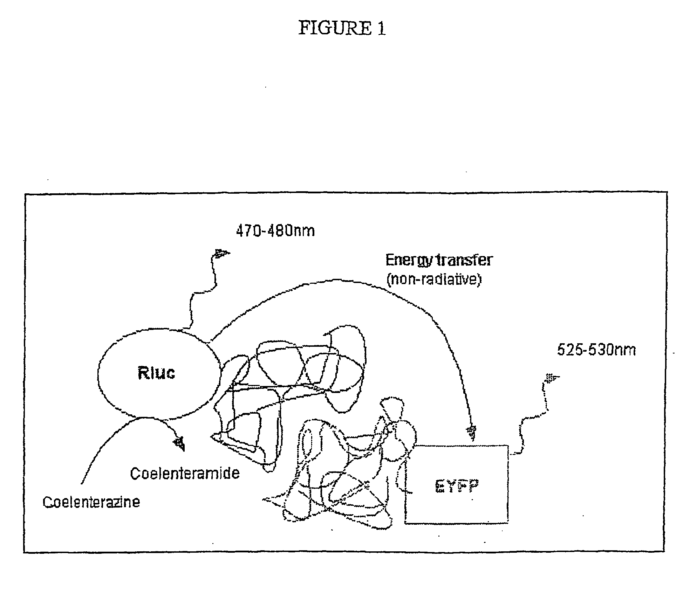

Figure 1 depicts an exemplary embodiment of a BRET system that can be used to detect the interaction (moving together, moving apart, or changing their orientation to one another) of two modulator entities. In one example, wherein this embodiment can be used for detecting binding partners, the BDP can be attached to one gene product and the FAM can be attached to an unknown gene product, such that interaction between the two gene products will be displayed by the light emission of the FAM. In another application, such as drug discovery, this embodiment is used to determine if a chemical or molecular substance will cause the molecular entities such as homo-dimers or hetero-dimers to move together or to move apart. By attaching the BDP to one of the dimer-proteins and the FAM to the other dimer-protein, changes in the spatial relationship between the dimer-proteins in response to a chemical compound such as a drug candidate, will translate to the spatial relationship between the BDP and the FAM, that will be signalled by a change in the light emission from the system.

This system can be used in *in vitro* assays to detect molecular changes in a wide variety of applications, and is amenable to automation. In particular, it is useful for assaying protein interactions, enzyme activities and the concentration of analytes or signalling molecules in cells or in solution. It is useful for drug discovery, analyte screening, second messenger screening, drug screening, diagnosis, genotoxicity, identification of gene function, gene discovery, and proteomics.

There are a wide variety of examples demonstrating the versatility and usefulness of the BRET system, including functional genomics, receptor monitoring, identification of orphan receptor ligands and orphan receptor activity, etc.

In one embodiment of the present invention the BRET system is used in *in vitro* assays. In general, the proteins are introduced by transforming or transfecting a cell *in vitro* with one or more vectors comprising recombinant DNA encoding some or all of the components of the BRET system. The cell then produces the protein and BRET occurs when the BDP, the FAM and the substrate are in the appropriate spatial relationship. The substrate can be either added exogenously following expression of the proteins or introduced in its nucleic acid form, such as via a vector with an inducible promoter.

The components of the BRET system can be produced using molecular biology techniques or isolated from natural sources. After purification, they can be used in non-cell based in vitro assays to monitor protein-protein interactions or they can be used in *in vitro* assays to monitor protein-protein interactions in cells.

The BRET system of the present invention is readily adaptable to means of automation and high-throughput screening.

## Broadening the Spectral Resolution Between Donor and Acceptor Emission Wavelengths

As stated above, current BRET systems exhibit a relatively low signal-to-base ratio (S/B) and dynamic range (DR), and due to the small difference between BDP and FAM emission wavelengths, there is always some BDP light which is emitted in the wavelength range of the FAM emission, which causes an increase in background light. Thus, there is a need to broaden the spectral resolution between BDP and FAM emission wavelengths. In one aspect, the present invention provides a bioluminescence resonance energy transfer (BRET) system with a broad spectral resolution between the BDP and FAM emission wavelengths.

There are a number of embodiments for the improved BRET system of this invention comprising: I) subtracting the background light using appropriate algorithms; 2) optimising the interference filters used for the light detection in order to reduce overlap between detected emission signals (using narrow bandwidth filters increases resolution between the donor and acceptor emission peaks); and 3) modifying the molecular components of the technology, to select for those which will provide a greater difference between donor and acceptor emission wavelengths improving both the S/B and the DR of a BRET system.

*Utilizing an Algorithm to Correct for Background Light*

One embodiment of the present invention provides the use of an algorithm to mathematically decrease the background light level during BRET assay. Although, it should be apparent to one skilled in the art that other algorithms could be used, one simple approach is to remove the portion of BDP emission in the FAM peak. For example, this could be calculated using the simple formula:

$$\frac{EmFAM - (EmBDP \times correcting\ factor)}{EmBDP} = corrected\ ratio$$

where EmFAM is the emission value (usually expressed as count per second: CPS) obtained with the filter specific for the FAM emission and EmBDP is the emission value (CPS) obtained with the filter specific for the BDP emission. The correcting factor is the ratio of EmFAM/EmBDP of the BDP in the absence of FAM. An example of the use of this algorithm is presented in Example II.

*Altering Emission Filters*

One embodiment of the present invention provides the use of BRET with a spectrophotometer, for example a TopCount™ (Packard Instrument Company, Meriden, CT), wherein interference filters are added in order to minimise the overlap between the light detected from the BDP and FAM emissions and thereby improve the S/B. Example VIII demonstrates the use of different filter pairs spectra in order to improve the BRET ratio. Changing the spectral characteristics of the filters used for the detection, it is possible to improve spectral performance of the system. In this particular example one filter pair was altered and resulted in greater than four fold improvement in the S/B.

*Altering the BDP Emission*

As discussed above, the BDP emission can be manipulated by modifications to the BDP substrate. In the case of luciferases the substrate is coelenterazine. The rationale behind altering the BDP emission is to improve the resolution between donor emission and acceptor emissions. The original BRET system uses the Rluc as donor, EYFP (or Topaz) as the acceptor and coelenterazine *h* derivative as the Rluc substrate. These components when combined in a BRET assay, generate light in the 475-480 nm range for the BDP and the 525-530 nm range for the FAM, giving a spectral resolution of 45-55nm. Unfortunately, Rluc generates a broad emission peak overlapping substantially the GFP emission, which in turn contributes to decrease the signal to noise of the system.

One embodiment of the BRET system of the present invention (see examples and below), using coel400a as the Rluc substrate, provides broad spectral resolution between donor and acceptor emission wavelengths(-105nm). Rluc with coel400a generates light between 390-400 nm and a GFP was prepared (see below) which absorbs light in this range and re-emits light at 505-508 nm. Because of this increase in spectral resolution between Rluc and GFP emissions, this BRET system provides an excellent biological tool to monitor small changes in proximity of two interacting proteins. This is a significant improvement over the system described previously using the coelenterazine h derivative and EYFP, which has a wavelength difference between donor and acceptor of approximately 51 nm.

Various coelenterazine derivatives are known in the art, including coel400a (Structure #VI described in *Biochemistry* **18**, 2204-2210, 1979), that generate light at various wavelengths (distinct from that generated by the wild type coelenterazine) as a result of Rluc activity (see Table 1 and the Examples). A worker skilled in the art would appreciate that because the light emission peak of the donor has changed, it is necessary to select an FAM which will absorb light at this wavelength and thereby permit efficient energy transfer. This can be done, for example by altering a GFP class 4 such that it becomes a class 3 or 1 GFP (see Tsien, 1998 for class description). Spectral overlapping between light emission of the donor and the light absorption peak of the acceptor is one condition among others for an efficient energy transfer (Lakowicz, 1983). Class 3 and 1 GFPs are known to absorb light at 400 nm and re-emit between 505 - 511 nm. This results in a wavelength difference between donor and acceptor emissions of approximately 111 nm.

It should be apparent to one skilled in the art that any protein capable of bioluminescence can be used in the present BRET system. Non-limiting examples of bioluminescent proteins are provided in Table 3. Alternatively, enzymes can be used in combination with appropriate substrates, as known in the art, to generate bioluminescence. Examples of such enzymes are β-galactosidase, alkaline phosphatase, and horseradish peroxidase. Regardless of the choice of BDP it is possible to further adapt the emission spectrum of the protein using the techniques outlined herein. In particular, various substrates are commercially available from companies such as Tropix Inc. and Molecular Probes, Inc., and others can be prepared using standard synthetic techniques, for use with bioluminescent proteins and other enzymes.

*Altering the FAM or BDP proteins*

Part of the rationale behind altering the FAM is to adapt its absorption spectrum to the emission of the BDP and specific coelenterazine derivative. Energy transfer efficiencies occurring in BRET are governed by the Förster laws. Förster proposed that energy transfer occurs by dipole-dipole resonance (radiation-less energy) interaction between an excited donor and an acceptor chromophore under specific conditions. One of these conditions is the physical distance between the donor and the acceptor moieties. Since the energy transfer efficiency is inversely proportional to the sixth power of the distance, the energy transfer efficiency is highly sensitive to distance changes between the donor and the acceptor. This range correlates well with most biological interactions, which makes this technique an excellent tool for monitoring protein-protein interactions. Another important condition is the relative orientation of donor and acceptor dipoles. If they are parallel, energy transfer is maximal and if they are in opposite direction, no energy is transferred. Other intrinsic parameters related to the donor and acceptor spectral characteristics are important to obtain efficient energy transfer. For example the quantum yield and the extinction coefficient of the donor and acceptor, respectively, are very important; as these values increase the energy transfer efficiency also increases. Another important parameter is the overlap between the emission spectrum of the donor and the absorption spectrum of the acceptor. This overlap must be as high as possible. These are characteristics of the FAM (and the BDP) that can be changed by altering the amino acid sequence or chemical structure.

In one embodiment, the FAM is a protein-based molecule. The absorbance and emission spectra of the FAM can be altered using standard techniques, known to a worker skilled in the art, including site directed mutagenesis or random mutagenesis. *In vitro* site-directed mutagenesis techniques are well known in the art and kits are commercially available for performing site directed mutagenesis (e.g. QuickChange™ Site-Directed Mutagenesis Kit from Stratagene). Generally, using site directed mutagenesis, the coding sequence for the FAM protein is mutated to incorporate one or more point mutations, to switch amino acids, and/or to delete or insert single or multiple amino acids. The result is a coding sequence for a mutated FAM protein. This protein is then expressed using standard molecular biological techniques, well known to those skilled in the art, and studied to determine whether its absorption and/or emission spectra have altered. The emission/excitation spectrums may be altered as a result of the mutation, such that the absorption and/or emission spectra are shifted or such that the intensity of the emission spectra has increased or decreased.

In one embodiment of the present invention the FAM is a Green Fluorescent Protein (GFP). It is well know in the field which mutations in the sequence of GFP are responsible for changes in the spectrum, quantum yield, and extinction coefficient (U.S. Patent Nos. 6,077,707, 6,054,321, 5,804,387, 6,066,476, 5,625,048 and 5,777,079).

Furthermore, since it is often important to regulate the relative gene expression of the BDP and the FAM fusion proteins, one can introduce mutations in the BDP or the FAM that will not affect the spectral characteristics of the proteins but rather will affect their cell expression levels (number of molecules per cell). Techniques for introduction of incorporation of such modifications are well known in the art. For example, it is known in the field that the mutations F64L and V163A in the GFP provide thermo-tolerance and good folding capability (U.S. Patent No. 6,027,881). Also, codon humanisation of the cDNAs coding for the Rluc and the GFP causes an increase in cell expression between six and thirty fold when expressed in mammalian cells (for GFP see U. S. Patent No. 6,020,192 and example V for Rluc). The introduction of a Kozak consensus sequence in the cDNA of the BDP or FAM is method of increasing protein expression in mammalian cells without affecting the spectral characteristics of the protein.

Other techniques can be used in order to modify the FAM. Chemical modification of amino acid residues on purified or semi-purified FAM can be performed. Also, random mutagenesis coupled to a mutant screening procedure may be used to determine modified FAMs that are more suitable or adapted to the BDP/substrate emission. Any means that changes or modifies an amino acid present in the FAM protein (both at the DNA or protein levels, using in vitro procedures) can be used to adapt the FAM to BDP/substrate emission.

In another embodiment, FAM can be a non-proteinaceous molecule (e. g. fluorescein, rhodamine, cascade blue, etc) which can be coupled to a BDP in order to build a BRET assay. There are various known non-proteinaceous FAM that can be used according to the present invention (see Table 2 for non-limiting examples of donor/acceptor pairs using non-proteinaceous FAM). Such molecules can be generated using standard chemical procedures known in the art. The non-proteinaceous FAM must have suitable spectral characteristics for efficient energy transfer with a BDP, such as overlapping of the BDP emission spectrum with the excitation spectrum of the FAM. A non-proteinaceous FAM can be chemically modified, using standard techniques known in the art, in order to adapt its spectral characteristics, improve its stability, alter is permeability, etc.

The BDP moiety can be modified using the same techniques as for the proteinaceous FAM in order to improve its spectral properties (quantum yield or its emission peak position for a specific substrate) and/or its biological (e.g. cell expression) properties. In Example V, the Rluc gene was codon humanised in order to increase its expression in mammalian cells and thereby increase light emission. Site-directed mutagenesis can be carried out on the Rluc gene to improve the quantum yield of light production when the Rluc binds the coelenterazine. For example, Liu and Escher (Gene 237 pp. 153-159, 1999) have shown that when the cysteine at position 124 (position 152 in their construct) is substituted with an alanine, a marked increase in bioluminescence activity (using native coelenterazine) of Rluc is obtained in comparison to the unmodified Rluc.

As indicated above, in one embodiment of the present invention alternative enzymes that can act on specifically designed luminescent substrates are used as BDPs. The light emission from the enzyme activity of these BDPs is dependent on the structure of the luminescent substrate. It should be apparent to one skilled in the art that in order to broaden the spectral resolution between emission wavelengths, a substrate is selected which will generate a light emission that allows a resolution of at least 60 nm, and preferably 80 nm, between the emissions.

**Monitoring the Responseof the BRET System**

In one embodiment, the energy transfer occurring between the donor (BDP) and acceptor (FAM) moieties is calculated from the emissions measured using light filters (one for the acceptor emission and the other the donor emission) that select specific wavelengths (see equation 1).

$$Ea/Ed = BRET \text{ efficiency} \qquad (1)$$

where Ea is defined as the FAM emission intensity (emission light is selected using specific filter adapted for the emission of the acceptor) and Ed is defined as the BDP emission intensity (emission light is selected using specific filter adapted for the emission of the BDP).

It should be readily appreciated by those skilled in the art that the light filters may be any type of filter that permits wavelength discrimination suitable for the BRET. For example, light filters used in accordance with the present invention can be interference filters, long pass filters, short pass filters, etc. Intensities (usually in counts per second (CPS)) of the wavelengths passing through filters are quantified using either a photo-multiplier tube (PMT) or a CCD camera. The quantified signals are subsequently used to calculate energy transfer efficiencies. The energy transfer efficiency increases with increasing intensity of the acceptor emission. Generally, a ratio of the acceptor emission intensity over the donor emission intensity is determined (see equation 1), which is an abstract number that reflects energy transfer efficiency. The ratio increases with an increase of energy transfer (see Xu *et al*, (1999) *Proc. Natl. Acad Sci. USA*. **96,** 151-156).

Energy transfer efficiencies can also be calculated using the inverse ratio of donor emission intensity over acceptor emission intensity (see equation 2). In this case, ratios decrease with increasing energy transfer efficiency.

$$Ed / Ea = BRET \text{ efficiency} \qquad (2)$$

where Ea and Ed are as defined above.

The light intensity of the BDP and FAM emission can also be quantified using a monochromator-based instrument such as a spectrofluorometer, a charged coupled device (CCD) camera or a diode array detector. Using a spectrofluorometer, the emission scan is performed such that both BDP and FAM emission peaks are detected upon addition of the substrate. The areas under the peaks represent the relative light intensities and are used to calculate the ratios, as outlined above. Any instrument capable of measuring lights for the BDP and FAM from the same sample, can be used to monitor the BRET system of the present invention.

In an alternative embodiment the FAM emission alone is suitable for effective detection and/or quantification of BRET. In this case, the energy transfer efficiency is calculated using only the acceptor emission intensity. It would be readily apparent to one skilled in the art that in order to measure energy transfer, one can use the acceptor emission intensity without making any ratio calculation. This is due to the fact that ideally the acceptor will emit light only if it absorbs the light transferred from the BDP. In this case only one light filter is necessary.

In an alternative embodiment the energy transfer efficiency is measured using only one light filter but still requires a ratiometric measurement. In this case, light intensity for the donor or the acceptor are determined using the appropriate light filter and another measurement of the samples is made without the use of any filter (intensity of the open spectrum). In this latter measurement, total light output (for all wavelengths) is quantified. Ratio calculations are then made using either equation 3 or 4. For the equation 3, only the light filter for the acceptor is required. For the equation 4, only the light filter for the donor is required.

$$E_a / E_o - E_a = \text{BRET efficiency or} = E_o - E_a / E_a \qquad (3)$$

$$E_o - E_d / E_d = \text{BRET efficiency or} = E_d / E_o - E_d \qquad (4)$$

where $E_a$ and $E_d$ are as defined above and $E_o$ is defined as the emission intensity for all wavelengths combined (open spectrum).

It should be readily apparent to one skilled in the art that further equations can be derived from equations 1 through 4. For example, one such derivative involves correcting for background light present at the emission wavelength for BDP and/or FAM.

In performing a BRET assay, light emissions can be determined from each well using the BRETCount. The BRETCount instrument is a modified TopCount™, wherein the TopCount™ is a microplate scintillation and luminescence counter sold by Packard Instrument (Meriden, CT). Unlike classical counters which utilise two photomultiplier tubes (PMTs) in coincidence to eliminate background noise, TopCount™ employs single-PMT technology and time-resolved pulse counting for noise reduction to allow counting in standard opaque microplates. The use of opaque microplates can reduce optical crosstalk to negligible level. TopCount™ comes in various formats, including 1, 2, 6 and 12 detectors (PMTs) which allow simultaneous reading of 1, 2, 6 or 12 samples, respectively. Beside the BRETCount other commercially available instruments are capable of performing BRET: the Victor 2™ (Wallac, Finland (Perking Elmer Life Sciences)] and the Fusion™ (Packard Instrument, Meriden). BRET can be performed using readers that can detect at least the FAM emission and preferably two wavelengths (for the FAM and the BDP) or more.

*The Use of BRET*

The BRET system is useful to study receptor dimerization/Multimerization. In accordance with one aspect of the present invention there is provided a system to detect the interaction (moving together, moving apart, or changing their orientation relative to one another) of the two target molecules. In one example, wherein this embodiment is used for detection of binding partners, the BDP is attached to one gene product and the FAM is attached to an unknown gene product, such that interaction between the two gene products will be displayed by the energy emission from the system.

One embodiment is used to monitor receptor, or receptor subunit, homo-multimerization or hetero-multimerization. To accomplish this one gene construct is prepared with the receptor, or receptor subunit, gene fused, in frame, to a BDP or FAM coding sequence and a second gene construct is prepared with a second receptor, or receptor subunit, gene fused, in frame, to the counterpart (FAM or BDP), and wherein the second receptor, or receptor subunit, may or may not be the same as the first. The gene fusion constructs are used to transfect or transform cells and the protein fusion products are expressed. In the presence of an appropriate BDP substrate, a BRET signal is generated as a result of association of the gene products, which may be detected and, where necessary, quantified.

In a related embodiment, the assay may be performed *in vitro*, where each of the gene fusion constructs are expressed in a cell-free system. In the presence of an appropriate BDP substrate, the association of gene products is again indicated by generation of a BRET signal, which may be detected and, where necessary, quantitated.

Alternatively, the receptor, or receptor subunit, proteins under study can be chemically attached to the BDP and FAM. In this way it is possible to make use of a non-protein FAM which is useful when studying receptor signaling under variable conditions.

Another embodiment of the present invention is used for characterization of orphan receptors. A gene construct is prepared with the receptor, or receptor subunit, gene fused, in frame, to a BDP or FAM coding sequence and a second construct is prepared with a potential ligand coding sequence fused, in frame, to the coding sequence for the counterpart (FAM or BDP). The gene fusions are used to transfect or transform cells and the gene fusions are expressed to generate the protein products. In the presence of an appropriate BDP substrate, a BRET signal is generated as a result of association of the gene products, which may be detected and, where necessary, quantitated. A BRET signal indicates that the orphan receptor binds the ligand. This assay can be used to screen a variety of potential ligands by preparing gene fusions from the coding sequences of a collection of potential ligands.

Another embodiment, also used to identify ligands for orphan receptors, makes use of changes in the target receptor upon ligand binding. A gene construct is prepared with the receptor, or receptor subunit, gene fused, in frame, to a BDP or FAM coding sequence. A second construct is prepared with a coding sequence for a protein which will bind specifically to the activated form of the target receptor (ie. the receptor bound to its ligand) fused, in frame, to the coding sequence of the counterpart (FAM or BDP). The gene fusions are used to transfect or transform cells *in vitro* and the gene fusions are expressed to generate the protein products. A potential ligand molecule is added to the transfected, or transformed, cells and upon binding of this ligand, the labeled target receptor undergoes a change (e.g. conformational change, phosphorylation, etc) such that it is recognized by the protein product of the second gene fusion. The second protein product then specifically binds the labeled, and now activated, target receptor and a BRET signal is generated. The BRET signal is detected and, where necessary, quantitated. Non-limiting examples of proteins which will specifically bind to activated receptors are effector molecules (eg. β-arrestin, G-protein, ubiquitin) and antibodies generated against the active form of the target receptor.

In a related embodiment, these assays may be performed *in vitro*, where each of the gene fusion constructs are expressed in a cell-free system. In the presence of an appropriate BDP substrate, the association of gene products is again indicated by generation of a BRET signal, which may be detected and, where necessary, quantitated.

Alternatively, the target orphan receptor, or receptor subunit, protein can be chemically attached to the BDP and FAM. Similarly, the binding partner may be labeled by chemical attachment of the counterpart (FAM or BDP). When screening non-protein ligands for receptor binding it is necessary to chemically attach the reporter molecule (FAM or BDP) to the potential ligand. In this way it is possible to make use of a non-protein FAM which is useful when studying receptor signaling under variable conditions.

Another embodiment of the invention provides a system of detecting interactions which occur as a consequence of receptor signaling, and such signaling through second messenger systems. In this embodiment the two potentially interacting partners are known and are labeled with BDP and FAM, respectively. The labeled interacting partners can be prepared using genetic methods, by fusing the gene encoding one partner, in frame, to a BDP or FAM coding sequence and fusing the gene encoding the second partner, in frame, to the counterpart (either FAM or BDP, respectively). The gene fusions are then used to transfect, or transform cells *in vitro*, for expression of the gene fusion products. These cells also express the target receptor and upon addition of a potential agonist, antagonist or inverse agonist the BRET signal is monitored as an indication of receptor activation and signaling.

In a related embodiment, the potentially interacting partners are labeled with BDP and FAM using chemical methods. This is particularly useful when one or both of the partners are non-protein molecules and when the FAM is a non-protein molecule.

Examples of interacting partners which may be used in these embodiments include, but are not limited to: adenylate cyclase which will interact with the G-protein α-subunit; G-protein α-subunit binding to the G-protein βγ-subunits; and Cap-response element binding protein (CREB) which will bind to the Cap-response element (CRE) and the Cap-response element modulator (CREM).

The BRET system and assays built thereon can be used to study the effect of additional molecules on receptor function. Once an interaction has been identified as part of the target receptor signaling, another embodiment of this invention can be used to analyze modulators of receptor function. These embodiments can be applied to situations wherein it is desirable to screen for modulator compounds or to investigate environmental effects such as pH, concentration of the modulator, role of ions, etc.

In terms of its application to modulators of receptor dimerization/multimerization, in one embodiment, one gene construct is prepared with the receptor, or receptor subunit, gene fused, in frame, to a BDP or FAM coding sequence and a second gene construct is prepared with a second receptor, or receptor subunit, gene fused, in frame, to the counterpart (FAM or BDP), and wherein the second receptor, or receptor subunit, may or may not be the same as the first. The gene fusion constructs are used to transfect or transform cells *in vitro* and the protein fusion products are expressed. In the presence of an appropriate BDP substrate, a BRET signal is generated as a result of association of the gene products, which can be monitored, before, during and after the addition of a potential modulator compound, as an indication of inhibition or enhancement of receptor, or receptor subunit, interactions.

In a related embodiment the gene products are physically linked to the reporter molecules, which allows the use of a non-peptide FAM.

In one embodiment pertaining to modulators of receptor-ligand binding, a gene construct is prepared with the receptor, or receptor subunit, gene fused, in frame, to a BDP or FAM coding sequence and a second construct is prepared with a potential ligand coding sequence fused, in frame, to the coding sequence for the counterpart (FAM orBDP). The gene fusions are used to transfect or transform cells *in vitro* and the gene fusions are expressed to generate the protein products. In the presence of an appropriate BDP substrate, a BRET signal is generated as a result of association of the gene products, which can be monitored, before, during and after the addition of a potential modulator compound, as an indication of inhibition or enhancement of receptor, or receptor subunit, interactions.

In a related embodiment the gene products are physically linked to the reporter molecules, which allows the use of a non-peptide FAM.

To gain a better understanding of the invention described herein, the following examples are set forth. It should be understood that these examples are for illustrative purposes only. Therefore, they should not limit the scope of this invention in any way.

**EXAMPLES**

**EXAMPLE I: Preparation of the BRET System**

*1. Selecting a Suitable BDP-FAM-substrate combination*

To design an effective BRET system, it is necessary to select a BDP and a FAM, either or both of which may be modified in according to the present invention. In nature some bioluminescent protein-fluorophore pairings interact directly with one another (see Ward and Cormier, 1978 for a list of naturally occurring interactions). In other cases, the pairing interaction is mediated through other proteins, in the absence of which interactive coupling occurs with a significantly lower affinity. If such a decreased affinity is present at expression levels required to achieve BRET, when the BDP and FAM are coupled respectively to other protein moieties, then such a pairing is suitable for BRET. Examples of systems which occur naturally include the *Renilla* luciferase (Rluc)/*Renilla* GFP coupling (Cormier (1978) Methods Enzymol. 57:237-244) and the Aequorin/*Aequorea* GFP coupling (Wilson and Brand (1998) Annu. Rev. Cell Dev. Biol. 14:197-230). An example of an engineered system, which is suitable for BRET is an Rluc and enhanced yellow mutant of GFP (EYFP) pairing which do not directly interact to a significant degree with one another alone in the absence of mediating proteins (Y. Xu, *et al.* (1999) *Proc. Natl. Acad Sci. USA* **96**:151-156). Ward and Cormier (1978) describes a list of coelenterate luciferase/GFP combinations, which can perform energy transfer. Other fluorophores, which are suitable pairings for Rluc include fluorophores from GFP classes 1, 2, 4 and some from 5. Specific examples of suitable fluorophores include but are not necessarily limited to: wild type GFP, Cycle 3, EGFP, Emerald, Topaz, 10C Q69K, and 10C.

Another aspect to consider when selecting a suitable BDP/FAM/substrate combination is the spectral characteristics of both the BDP and FAM. BDP generates light when it processes its substrate. The spectral characteristics of this light are dependent of the substrate derivative used. For example, when *Renilla* luciferase (Rluc) processes wild type coelenterazine light emission peaks around 460-480 nm, when Rluc processes coelenterazine *hcp* derivative, it generates light peaking around 445 nm. Therefore, Rluc can emit at various wavelength depending of what substrate it processes. Hence, according to Forster laws describing energy transfer between donor and acceptor, the FAM must be selected based on the emission wavelength of the BDP. In order to obtain efficient energy transfer between BDP and FAM upon substrate addition, FAM excitation spectrum must overlap emission spectrum of BDP.

It has been demonstrated that an Rluc/EGFP pairing is not as good as an Rluc/EYEF pairing based on observable emission spectral peaks (Y. Xu, (1999), *supra* and Wang, *et al.* (1997) in *Bioluminescence and Chemiluminescence: Molecular Reporting with Photons,* eds. Hastings et al. (Wiley, New York), pp. 419-422) even if Rluc/EGFP in theory (according to Forster laws) should be a better pair because of the better spectral overlap. The emission peak of the EGFP is too close to the emission peak of the Rluc in live mammalian cells, which increases the difficulty in specifically quantifying each emission using standard interference filters.

Using Rluc as the BDP, with wild-type coelenterazine as the substrate, and EYFP as the FAM the wavelength difference that separates donor and acceptor emissions is about 50 nm. Because the emission peak of the donor is very broad (>65nm), it partially covers the acceptor emission which results in a reduction in the signal-to-base ratio (S/B). In order to minimise this drawback, the difference between the wavelengths of the donor and acceptor emissions should be maximised. The emission wavelength difference can be maximised by carefully selecting the BDP and FAM based on their spectral characteristics. This may be further improved by altering the emission wavelength of the BDP, for example by using a different coelenterazine substrate molecule when the BDP is a luciferase, or by altering the absorbance and emission spectra of the FAM, for example by using a mutant green fluorescent protein (GFP). In one embodiment of the present invention a combination of both an altered emission wavelength of the BDP and an altered absorbance and emission spectrum of the FAM is used to maximise the distance between the donor and acceptor emissions.

There are a number of different bioluminescent donor proteins that can be employed in this invention. One very well known example is the class of proteins known as luciferases which catalyze an energy-yielding chemical reaction in which a specific biochemical substance, a luciferin (a naturally occurring fluorophore), is oxidized by an enzyme having a luciferase activity. A great diversity of organisms, both prokaryotic and eukaryotic, including species of bacteria, algae, fungi, insects, fish and other marine forms can emit light energy in this manner and each has specific luciferase activities and luciferins which are chemically distinct from those of other organisms. Luciferin/luciferase systems are very diverse in form, chemistry and function. For example, there are luciferase activities which facilitate continuous chemiluminescence, as exhibited by some bacteria and mushrooms, and those which are adapted to facilitate sporadic, or stimuli induced, emissions, as in the case of dinoflagellate algae. As a phenomenon which entails the transformation of chemical energy into light energy, bioluminescence is not restricted to living organisms, nor does it require the presence of living organisms. It is simply a type of chemiluminescent reaction that requires a luciferase activity which at one stage or another had its origins from a biological catalyst. Hence the preservation or construction of the essential activities and chemicals suffices to have the means to give rise to bioluminescent phenomena. Bioluminescent proteins with luciferase activity are thus available from a variety of sources or by a variety of means. Examples of bioluminescent proteins with luciferase activity may be found in U.S. Patent Nos. 5,229,285, 5,219,737, 5,843,746,5,196,524, 5,670,356.

Alternative BDPs that can be employed in this invention are enzymes which can act on suitable substrates to generate a luminescent signal. Specific examples of such enzymes are β-galactosidase, alkaline phosphatase, β-glucuronidase and β-glucosidase. Synthetic luminescent substrates for these enzymes are well known in the art and are commercially available from companies, such as Tropix Inc. (Bedford, MA, USA).

It should be apparent to one skilled in the art that other non-GFP fluorophores, or other BDPs may be suitable for the BRET system of the present invention if the key pairing criteria are met. Other sources for example may include pairings isolated or engineered from insects (U.S. Patent No. 5,670,356) or other marine organisms (PCT WO 99/49019) and review in Hastings, 1996)). Alternatively, it may be preferable for the bioluminescent protein with luciferase activity and the fluorophore to be derived from completely different sources (organism) in order to minimise the potential for direct and spontaneous affinity between the pairings. Table 2 lists non-limiting examples of FAMs paired with appropriate BDPs and substrates, wherein the separation between the respective emission wavelengths is greater than 50 nm. Table 3 lists potential donors that can be used as alternatives to Rluc.

There are a number of different FAMs that can be employed in this invention. One very well known example is the group of fluorophores that includes the green fluorescent protein from the jellyfish *Aequorea victoria* and numerous other variants (GFPs) arising from the application of molecular biology, eg. mutagenesis and chimeric protein technologies (R.Y. Tsien, (1998) *Ann. Rev. Biochem*. 63: 509-544). GFPs are classified based on the distinctive component of their chromophores, each class having distinct excitation and emission wavelengths: class 1, wild-type mixture of neutral phenol and anionic phenolate: class 2, phenolate anion: class 3, neutral phenol: class 4, phenolate anion with stacked π-electron system: class 5, indole: class 6, imidazole: and class 7, phenyl (R.Y. Tsien, (1998), *supra*). Further non-limiting examples of FAMs are provided in the table below, which lists examples of non-protein FAMs paired with appropriate BDPs and substrates.

It should also be apparent to one skilled in the art that the FAM can be a protein-based molecule (such as GFPs) or a non-proteinaceous compound (e.g. Fluorescein). In the latter case, the FAM must be chemically attached to the modulator. When the FAM is a protein, it can be genetically or chemically coupled to the modulator. Since the BDP is always an enzyme it follows that it is always a protein and, therefore, can be attached to the modulator using recombinant DNA or chemical techniques.

To study potential BDP - FAM pairing, protein fusions are prepared containing the BDP and FAM to be tested. This may be achieved, for example, using the method of Xu *et al.*, (1999) *Proc. Natl. Acad Sci. USA* **96**:151-6, or as described herein.

It should be confirmed that the donor and acceptor fluorophores do not spuriously associate with each other. This can be accomplished by separate co-expression of the fluorophores in the same cells and then monitoring the luminescence spectrum in order to determine if BRET occurs. This may be achieved, for example, using the method of Xu *et al.*, (1999) *Proc. Natl. Acad Sci. USA* **96**:151-6.

Finally tests should be conducted to confirm the generation of BRET from the BDP and FAM when fused to interacting proteins. This can be performed by creating fusion proteins using the BDP and FAM with test proteins, known to interact with one another, and monitoring the luminescence spectrum after association. This may be achieved, for example, using the method of Xu *et al.*, (1999) *Proc. Natl. Acad. Sci. USA* **96**:151-6.

*2. Attachment of the BDP and FAM to a Modulator and/or Protein(s) of Interest*

There are a number of methods well known in the art for chemically or genetically attaching the BDP and the FAM to the modulator or to the target proteins or polypeptides of interest.

Chemical cross-linking reagents may be used to covalently attach the reporter molecule (BDP or FAM) to the protein under study. These reagents are well known in the art and include homobifunctional and heterobifunctional reagents that have two reactive groups (the same or different, respectively) that provide a means of linking the two molecules. The reactive groups include, but are not limited to, succinimidyl esters, maleimides and iodoacetates. These reagents can further provide a spacer molecule between the reporter molecule and the protein under study and thereby reduce steric hindrance. Another type of chemical crosslinking reagent, that is well known in the art, is one which will form a chemical bond between the protein under study and the reporter molecule without itself being incorporated into the product. This type of reagent allows the reporter molecule and the protein under study to be linked without an intervening molecule.

In some cases, a non-covalent interaction of sufficient affinity can function as a crosslink between the reporter molecule (BDP or FAM) and the protein under study; examples include, but are not limited to, avidin—biotin and antibody—hapten interactions. Biotinylation and haptenylation reagents are well known in the art and can be used to effectively tag the reporter molecule or protein under study, such that it can be recognised using a labelled detection reagent. For this system the detection reagent is labelled with either the protein under study or the reporter molecule, respectively.

Methods for genetically attaching the protein under study to the reporter molecule (BDP or FAM, where FAM is a protein) are also well known in the art. Generally, these methods involve fusing the coding sequences for each protein, in frame and under the control of appropriate regulatory sequences, followed by expression of the resulting fusion genes in *vitro* (after transfection or transformation into appropriate cells).

For *in vitro* production, BDP and FAM proteins according to the invention can be produced by transformation (transfection, transduction, or infection) of a host cell with all or part of a BDP or FAM-encoding DNA fragment in a suitable expression vehicle. Suitable expression vehicles include: plasmids, viral particles, and phage. For insect cells, baculovirus expression vectors are suitable. The entire expression vehicle, or a part thereof, can be integrated into the host cell genome. In some circumstances, it is desirable to employ an inducible expression vector, e. g., the LACSWITCH™ Inducible Expression System (Stratagene, LaJolla, Calif.).

Those skilled in the field of molecular biology will understand that any of a wide variety of expression systems can be used to produce the recombinant protein. The precise host cell used is not critical to the invention. The BDP and/or FAM protein can be produced *in vitro* in a prokaryotic host (e.g., *E.coli* or *B. subtilis)* or in eukaryotic host cells (e.g., Saccharomyces or *Pichia*; mammalian cells, e.g., COS, NIH 3T3, CHO, BHK, 293, or HeLa cells; or insect cells; or plant cells).

The host cells harbouring the expression vehicle can be cultured in conventional nutrient media adapted as needed for activation of a chosen gene, repression of a chosen gene, selection of transformants, or amplification of a chosen gene.

Specific initiation signals may also be required for efficient transcription and translation of inserted nucleic acid sequences, *in vitro.* These signals include the ATG initiation codon and adjacent sequences. In cases where an entire native BDP or FAM gene orcDNA, including its own initiation codon and adjacent sequences, is inserted into the appropriate expression vector, no additional translational control signals may be needed. In other cases, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators (e.g. Bittner *et al.* (1987) *Methods in Enzymol.* **153,** 516).

In addition, a host cell may be chosen which modulates the expression of the inserted sequences, or modifies and processes the gene product in a specific, desired fashion. Such modifications (e.g., glycosylation) and processing (e.g., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells that possess the cellular machinery for proper processing of the primary transcript, glycosylation, and phosphorylation of the gene product can be used. Such mammalian host cells include, but are not limited to, CHO, VERO, BHK, HeLa, COS, MDCK, 293, 3T3, WI38, and in particular, choroid plexus cell lines.

Alternatively, any fusion protein can be readily purified by utilising a binding molecule specific for the fusion protein being expressed. For example, a system described in Janknecht *et al.* (1981) *Proc. Natl. Acad. Sci. USA* **88,** 8972, allows for the ready purification of non-denatured fusion proteins expressed in human cell lines. In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto $Ni^{2+}$ nitriloacetic acid-agarose columns, and histidine-tagged proteins are selectively eluted with imidazole-containing buffers.

Alternatively, BDP and/or FAM, can be fused to an immunoglobulin Fc domain. Such a fusion protein can be readily purified using a protein A column.

*3. Preparation of the BDP-FAMFusion Molecule*

One skilled in the art would readily appreciate that the components of the BRET system (i.e. BDP, FAM and M) and their derivatives, can be combined to form a single fusion molecule which may be produced using recombinant techniques, isolated from natural sources or chemically synthesised, or by any combination thereof. Further, the components can be produced either as a single entity or separately, with subsequent coupling using standard coupling techniques. Various configurations of the BRET fusion protein are possible and are listed below:

| | |
|---|---|
| BDP-M-FAM: | the modulator is genetically or chemically inserted in between the BDP and FAM moieties. |
| FAM-M-BDP: | inverse configuration of the BDP-M-FAM |
| BDP-M-FAM: | T variation in which the modulator is chemically attached to a linker (peptide or non-peptide) sequence attaching the BDP and FAM moieties together. |
| FAM-M-BDP: | inverse configuration of the BDP-M-FAM |
| BDP-FAM-M: | the modulator is genetically or chemically attached to the FAM moiety only |
| BDP-PAM (M): | the modulator is genetically inserted either at the amino-or the carboxy-of the FAM moiety or internally in the FAM moiety |
| M-BDP-FAM: | the modulator is genetically or chemically attached to the BDP moiety only |
| BDP (M)-FAM: | the modulator is genetically inserted either at the amino-or the carboxy-of the BDP moiety or internally in the BDP moiety |

The fusion protein of the present invention can be prepared using standard recombinant DNA techniques well known to those skilled in the art. For example, in the preparation of the BDP-M-FAM configuration, the BDP coding sequence can be genetically introduced into the multiple cloning site of a bacterial expression vector such that the necessary regulatory sequences are operatively linked to the BDP coding sequence.

Finally, the various configurations of BRET fusion protein once produced *in vitro*, are used for the BRET assay, in the same cells used to produce the fusion protein, only if the cells express (endogenously or exogenously) the interacting factor. The substrate coelenterazine or any derivative thereof, is used to start the bioluminescent reaction (BRET assay) and may be added at any time before, during or after the BRET reaction. The substrate coelenterazine needed to start the bioluminescent reaction may be bought from commercial suppliers (e.g. Molecular Probes) or prepared using standard synthetic methods.

In another embodiment, the various configurations of BRET fusion proteins are produced *in vitro* as one entity but used in an *in vitro* BRET assay. The DNA constructs for various configurations of BRET fusion proteins, are transfected/transformed in suitable cell lines (eukaryotic or prokaryotic) for its production only. The various configurations of BRET fusion proteins produced in cells *in vitro*, are then purified or semipurified from the transfected/transformed cells. The easiest procedure to purify BDP-M FAM is using affinity chromatography where a his-tag engineered in the DNA construct (at the amino-termini) once translated binds a Nickel bead. Standard biochemical techniques can be also used alone or in combination with affinity chromatography to purify to various level the various fusion proteins. Finally, these purified fusion proteins can be also chemically or enzymatically modified before their use in the BRET assay. As an example of a modification, a phosphate group can be added on an specific amino acid residue found in the modulator sequence (e.g. tyrosine). This is done in order for a given interacting factor to recognise the modulator. The phosphate group can be added using a tyrosine or serine/threonine kinase enzyme or chemically using as an example phosphoramidite reagent. Other types of modifications can be envisaged. These are, but limited to, methylation, isoprenylation, addition of palmitate, myristate, sulfate, sugar groups).

In another embodiment, the fusion protein is produced by a combination of *in vitro* methods. First a fusion protein is genetically engineered and expressed in cells using recombinant techniques. The fusion protein is then purified or semi-purified before being modified by chemically or enzymatically attaching the modulator into the linker region. In that case, modulator attached onto the linker region can be peptide-based or chemically-based. This configuration can be used in an *in vitro* (biochemical) assay or in cells *in vitro* using various techniques such as protein transfection techniques, permeabilizing agent, by micro-injection or electroporation to introduce the protein inside the cells. In all cases, the substrate coelenterazine or any derivative thereof, is used to start the bioluminescent reaction (BRET assay).

(ii) Methods for preparing the various configurations of BRET fusion protein in vitro as one recombinant entity

In one embodiment, the various configurations of BRET fusion protein can be synthesised as one entity *in vitro* using IVTT (*in vitro* transcription-translation) techniques. When produced *in vitro*, the gene coding for the various configurations of BRET fusion protein are introduced downstream of a promoter (e. g. usually viral-based promoter: T7, T3, SP6) which is recognised by the specific viral RNA polymerase added to the IVTT reaction and therefore used to produce mRNA. mRNAs are then used in an in vitro translation reaction using cell extracts (e.g. rabbit reticulocytes, wheat germ extract) to produce the protein coding by the mRNA. The *in vitro* translation reaction can be optionally coupled to the *in vitro* transcription technique, using standard methods known in the art (e.g. TnT kits from Promega). These techniques of producing large quantities of specific mRNA and proteins *in vitro* are well known in the art. Furthermore, several suppliers (Invitrogen, Lifetechnologies, Stratagen, Promega) provide kits and reagents necessary to perform these reactions.

If necessary, the configurations of BRET fusion proteins produced using IVTT can be purified or semi-purified before its use in a BRET assay, using techniques as described above for the *in vitro* produced fusion protein. The fusion protein once produced *in vitro* and purified can be further chemically or enzymatically modified before its use in a BRET assay, as described above for the *in vitro* produced fusion protein.

Finally, the unpurified fusion protein, the purified or semi-purified the fusion protein or the purified and modified the fusion protein once produced *in vitro*, can be used in an *in vitro* (biochemical) assay or in cells *in vitro* using various techniques such as protein transfection techniques, permeabilizing agent, by micro-injection or electroporation to introduce the protein inside the cells. In all cases, the substrate coelenterazine or any derivative thereof, is used to start the bioluminescent reaction (BRET assay).

(iii) Methods for preparing the various configurations of BRET fusion protein as two entities coupled together using recombinant BDP and FAM moieties

The moieties (BDP and FAM) of the various configurations of BRET fusion protein can be produced separately (two different entities) and attached together before performing the BRET assay. In this case, the BDP and FAM protein are produced and purified using recombinant technology as described previously, or isolated from natural sources. Briefly, for recombinant production of the BDP and FAM protein genes coding for the BDP and FAM are introduced separately in expression vectors. These are then used produce the BDP and FAM protein using *in vitro* techniques as described above. The BDP and FAM protein are purified or semi-purified before attaching them to each other. For example, methods for the purification of GFPs (an example of a FAM) and luciferases (an example of a BDP) are known in the art (Deschamps, *et al*, (1995) *Protein Expression and purification* **6**: 555-558 ; Matthews, J. C., *et al* (1977) *Biochemistry* **16**: 85-91; Ward, W. W. and Cormier, M. J. (1978) *Photochemistry and Photobiology* **27**: 389-396). The two moieties are bridged together using chemical means. This is preferred if the modulator is not a peptide. A number of homo-hetero-bifunctional cross-linker reagents, all well know in the art, can be used to form the modulator (such as DSP, GMBS, etc; see the Pierce Catalog and Handbook for the signification of these acronyms). Molecules and chemical groups forming the modulator or part of the modulator can be attached covalently to either the amino- or carboxy-terminus of the moieties, or using internally amino acids. The modulator region or sequences can be formed during the attachment of the two moieties or added (in the linker attaching the two moieties together) after the attachment of the two moieties using chemical means (BDP-$_M$-FAM). It should be readily apparent to one skilled in the art that the protein moieties can be modified prior the coupling in order to facilitate their attachment to each order. The chemical composition of the modulator or in some cases the linker, is not critical. The molecular distance between the donor and acceptor moieties is more important. It should be between 10 to 100Å.

In another embodiment, the two BRET moieties (BDP and FAM) are expressed using recombinant technology and purified as one entity in which both donor and acceptor are genetically attached together through a peptide linker region (forming a BDP-FAM or FAM-BDP fusion proteins), and the modulator is chemically attached to either the donor (M-BDP-FAM) or the acceptor (BDP-FAM-M) moiety after the purification or partial purification of the BDP-FAM or FAM-BDP fusion proteins.

It should be apparent to one skilled in the art that the BDP and FAM do not have to be prepared the same way before the BRET assay. As an example, the BDP can be isolated from an organism and the FAM can be produced and purified using recombinant technology or IVTT technology. Or the BDP can be produced and purified using a modified gene and recombinant technology, and the FAM can be isolated from an organism, or chemically synthesised.

**EXAMPLE II : An Algorithm for the BRET Assay**

This example made use of the following algorithm to correct for background light:

$$\underline{(Em550 - Em470 \text{ x correcting factor})} = \text{corrected ratio}$$
$$Em\ 470$$

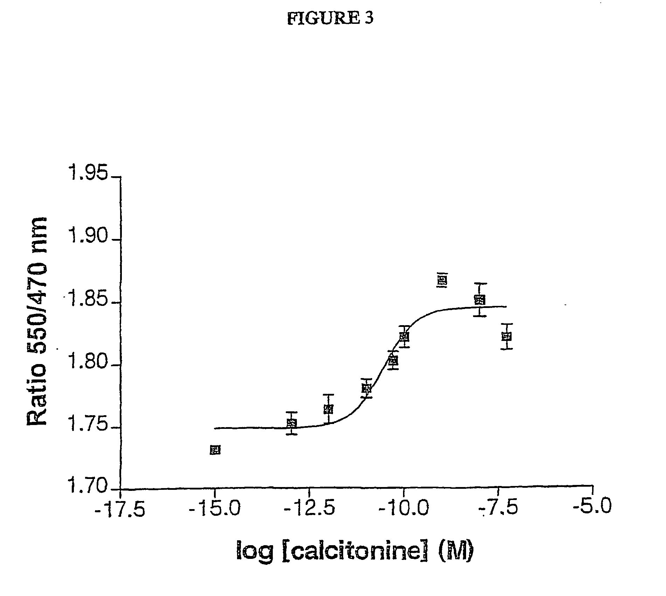

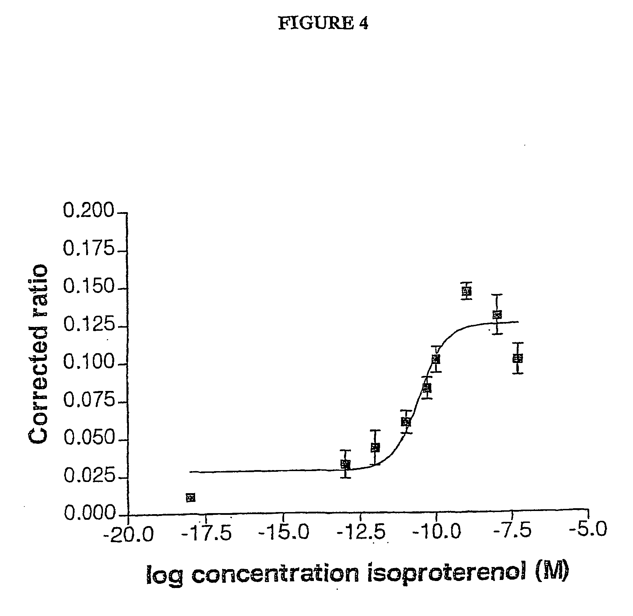

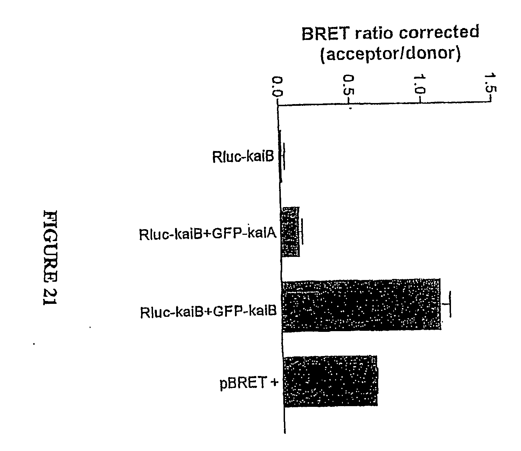

Where Em550 represents the emission at 550 nm from an FAM, expressed as CPS; and Em470 represents the emission at 470 nm from a BDP, expressed as CPS. The correcting factor was experimentally calculated using Rluc construct transfected alone (without the EYFP construct). The correcting factor is the ratio (550/470nm) produced by an Rluc construct at a given time after addition of coelenterazine. Figures 3 and 4 represent the non corrected and corrected data, respectively. The same raw data (light emissions using both filters)) were used in both figures. The only difference is that in Figure 4 the ratio has been corrected using the above formula. In this example, the above algorithm does not change the range dynamic of the assay which is still approximately 0.1 (the difference between maximal and minimal ratio) in both figures. But the S/B (signal to noise ratio) does change. S/B is the ratio between maximal and minimal BRET ratio values. In Figure 3, the S/B is 1. 07 compared to 4.77 for Figure 21. Introduction of algorithm in calculating BRET ratios, could be very useful during HTS process since it decreases background and increase S/B therefore ease result interpretation.

**EXAMPLE III : Chemical Synthesis of Coelenterazine Derivatives**

A series of papers (Hart et al, 1979; Chen et al, 1991; Chen et al, 1992; Matthews et al, 1977b; Shimomura et al, 1988; Shimomura et al, 1990; Shimomura et al, 1993; Shimomura, 1995), teach a derivative of coelenterazine, herein referred to as coel400a or phenylcoelenterazine (Structure #VI described in *Biochemistry* **18**, 2204-2210, 1979), which, when processed by Rluc, generates light at 400 nm. The use of this coel400a with Rluc, combined with a GFP absorbing light at 400 nm (e.g. class 1 and 3 GFPs) could provide a BRET system having a broad difference between emission wavelengths. In 1962 Shimomura *et al* discovered the luminescent protein from the jelly Fish *Aequorea victoria* and demonstrated that aequorin consists of a complex of apoaequorin and coelenterazine. The coelenterazine compound is responsible for the light emission in the bioluminescent reaction. Since Shimomura, the bioluminescent system was studied extensively and the synthesis of coelenterazine derivatives started.

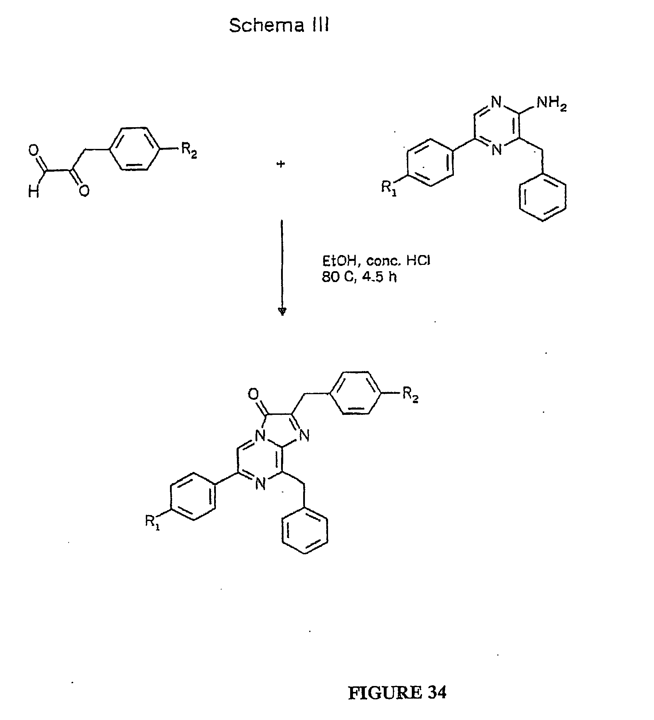

Originally the synthesis of coelenterazines was best described by Chart *et al* following a synthesis route originally published by Kishi. This synthetic route is known as the Kishi method reviewed by Shimomura and applied to prepare a large variety of coelenterazines. From the early 1970s until 1990, the methods used to prepare coelenterazines did not vary much. In one example is an intermediate demethylation method now using ethanethiol. Around 1990 some new synthetic methods to introduce Aryl groups into pyrazines appeared. Also an Italian group from the University of Bologna, Paradisi *et al.*, worked out an improved method. Synthesis of Phenylcoelenterazine (see scheme 3, Figure 34 $R_1$ =$R_2$=H)

All of the methods ultimately lead to scheme 3 (Figure 34); the coupling of an aryl pyrazine and an $\alpha$-oxoaldehyde. The resulting coelenterazine is purified by flash chromatography to obtain a compound which is sufficiently pure to be used to generate the aequorin bioluminescence. In scheme 3 (Figure 34) both the substituents $R_1$ and $R_2$ represent an hydrogen atom in the phenylcoelenterazine.

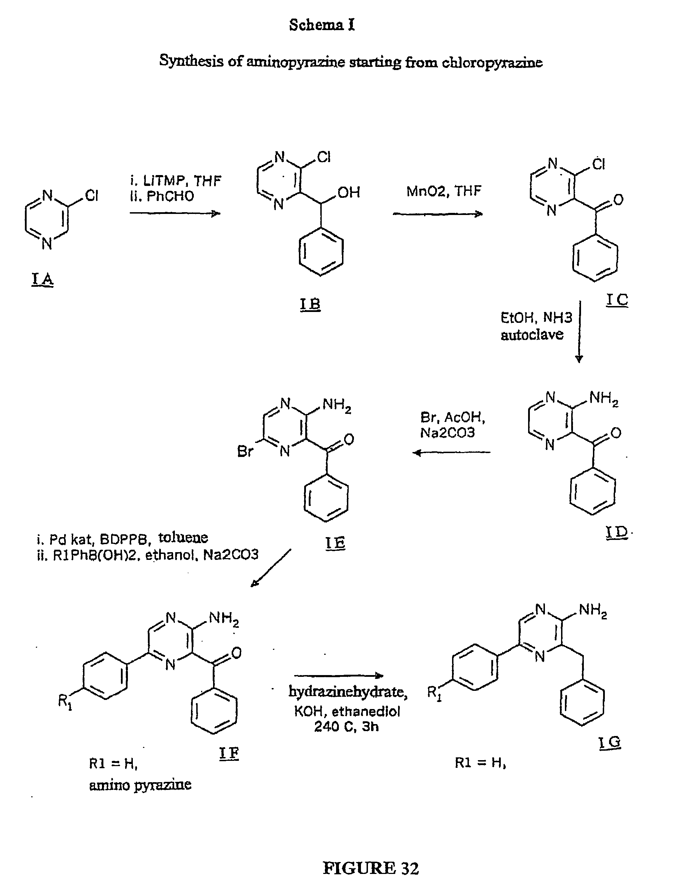

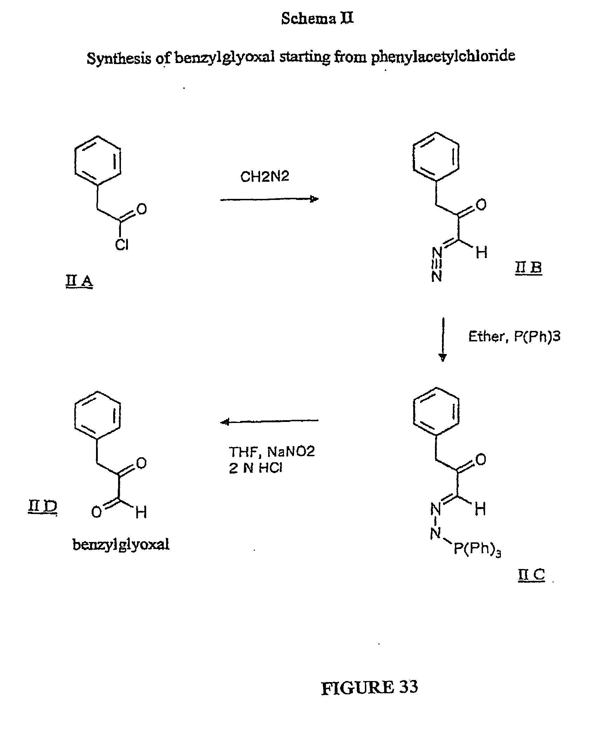

In scheme 1 (Figure 32) and scheme 2 (Figure 33) the synthesis of the intermediate aminopyrazine (sch. 1) and $\alpha$-oxoaldehyde (sch. 2) is presented. In the separate descriptions of the synthesis, for each individual step the references are provided. Arylpyrazines, one of the key compounds in the coelenterazine synthesis, (see scheme 3: Figure 34) can be made easily by the use of the Suzuki aryl boronic acid coupling reaction or a Stille coupling. The synthesis of various coelenterazines is possible using one of the published methods. However, the synthesis requires a lot of experience as the intermediates sometimes are unstable and the final end products sensitive to light and in solution the compounds are sensitive to deterioration. Purification is often troublesome, but flash chromatography is useful in obtaining sufficiently pure coelenterazines.

Scheme 1: Synthesis of amino pyrazines (Figure 32)

Preparation of 3-chloro-2-(1-hydroxybenzyl) pyrazine (I B)

Using the procedure of Turck et al. (*Communications*, 881-884, 1988), Benzaldehyde and THF (tetrahydrofuran) were freshly distilled. In an Argon atmosphere in a 3 necked flask 450 ml THF was cooled to -10°C. Next, 52,2 ml of a Buli solution (in hexane 84 mmole) was added and further cooled down to -70°C. Using a syringe 15 ml (88mmole) tetramethyl piperidine was added. Half an hour the mixture was stirred at 0°C and thereafter cooled down to -70°C again. To this 6 ml chloropyrazine was added followed by stirring during 30 min. at -70°C. Freshly distilled benzaldehyde was added in 15 minutes at a temperature between -65 and -60°C. The mixture was hydrolysed with a mixture of 30ml HCl/ 30ml ethanol/60ml THF at -60°C in approx. 15 minutes. The solution was allowed to warm to room temperature and neutralised with 150 ml saturated Sodium Bicarbonate solution. After evaporation of some of the solvents the solution was extracted with 2 × 100 ml dichloromethane and the combined layers dried on MgSO$_4$. The product was filtered on a silica column in 60 ml dichloromethane and 400 ml solution was recovered of the column. After evaporation of the solvent a slightly yellow solid was collected, Yield 12 grams (80%).

Preparation of 3-chloro-2-pyrazinylketone (I C)

Using the procedure of Turck et al., .(*Communications*, 881-884, 1988),
To a flask was added 14 g MnO$_2$ as a fine powder. While stirring a quantity of 5.5 grams of (I B) in 50 ml dry THF was added. At room temperature the mixture was allowed to react for 24 - 48 hrs. (Reaction followed by N.M.R.). The mixture was filtered through a glass tunnel and washed with 80 ml THF. Dry on MgSO$_4$. After evaporation of the solvent a light coloured oil was obtained. (5 g., 89%).

Preparation of 3-amino-pyrazinyl phenyl ketone.

Using the procedures of Jones et al. (Synlett, p. 509-510, 1996) and Thomson et al. (J. Org. Chem.,no. 53, p. 2052-2055, 1988), in an Argon atmosphere was dissolved in 50 ml ethanol (100%) 6,6 gr. 3-chloro-2-pyrazinyl ketone, while cooling the mixture to -20°C to -30°C. During 25 min. ammonia gas was introduced into the system. The solution was divided into steel tubes and after closing the tubes were heated to 120°C in an oven during the night. After cooling, the tubes were emptied in a beaker and neutralised with a sodium bicarbonate solution. After evaporation of some of the solvents the mixture was extracted with dichloromethane and dried on MgSO$_4$. Evaporation of the solvent resulted in 3 grams of the product (60% yield by NMR 80-90% pure).

Preparation of 2-amono-3-benzoyl-5-bromopyrazine (I E)

Using the procedures of Jones et al. (Synlett, p. 509-510, 1996), a mixture of 75 ml of glacial acetic acid and 6 grams of Sodium Carbonate was prepared in an Argon atmosphere. To this the crude 3 grams of aminopyrazinylphenyl ketone was added. Thereafter 2.4 g Br$_2$ was introduced and the mixture stirred for 30 minutes. The mixture was poured onto 200 grams of ice and neutralised with sodium bicarbonate solution until CO$_2$ evolution stopped. The aqueous layer was extracted with dichloromethane, the organic fraction was dried and the dichloromethane was removed by evaporation. The yield was 4 grams of product (purity 80% by N.M.R.) which was used without further purification.

Preparation of -2 amino-3-benzoyl-5 phenyl pyrazine. (I F)

Using the procedures of Jones et al. (Synlett, p. 509-510, 1996) and McKillop et al (Tetrahedron, vol. 48 (37), p. 8117-8126, 1992) the reaction was carried out in a 3-neck flask maintaining an Argon atmosphere. To 7 ml of toluene was added while stirring 100 mg bis (benzonitrile) Cl$_2$ Pd (II) and 125 mg. bis (diphenylphoshino) butane resulting in a slurry. A mixture was prepared consisting of 1 gr. crude I E 650 mg. methoxyphenyl boronic acid, 2ml ethanol, 10 ml toluene and 3,5 ml 1M Sodium Carbonate. The mixture was added to the above mentioned slurry and the composition reflected for 2,5 hrs. After cooling 20 ml of water was added, 100 ml of ethylacetate and the layers were separated. The aqueous layer was extracted twice with ethyl acetate. After washing with brine and drying (MgSO$_4$) the solvents were evaporated. Yield 1 g crude product. Column chromatography (ethylacetate-hexane 1:1) resulted in a 400 mg. (90%) pure product.

Preparation of 2-amino-3-benzyl-sphenyl pyrazine (I G)

Using the procedure of Jones et al (Synlett, p. 509-510, 1966) a mixture was prepared of 200 mg (I F) 0,55 ml 80% hydrazin hydrate, 1,3 gram KOH and 4 ml of ethyleneglycol.
The mixture is heated up to 200-220°C and remained at that temperature for at least 5 hrs. After cooling and acidifying with 2M HCl the layer was extracted with ethylacetate. After washing with brine and drying the solvents are evaporated. Column chromatography (ethylacetate : hexane 1:1) resulted in a slight yellow amino pyrazine I G. Yield 350 mg (70%).

Scheme II: (Figure 33)

Preparation of -1-phenyl-3 diazoacetone (II B)

Using the procedure of F. Arndt & J. Amende (Ber. Btsch.Chem. Ges., p. 1122-1124 and 2259-2265,1928), diazomethane was prepared in advance according to Organic Synthesis Vol. 41, p. 16. The solution of diazomethane was stored in the cold. A Diazomethane solution (0.3 mol) was decanted after storage in the cold to remove frozen water. The residual solution of diazomethane was cooled to 0 °C. Next 12 grams of Phenylacetylchloride in 30 ml of dry ether was added in approx. 20 minutes. Gas development occurred. Then the mixture was allowed to come to room temperature, where after approx. 1 hr. Argon was led across the system to remove excess diazomethane. The ether was partly distilled off and the product analysed by N.M.R. The crude product II B was used without further purification. Yield approx. 50%.

Preparation of Benzylglyoxal-1-triphenyl phosphazine (II C)

Using the procedure of Bestmann et al (Chem. Ber. 1345-1355, 1959) the Phenyl diazoacetone solution (II B) was cooled to approximately 0°C. Over the next 15 minutes a total of 25 grams of triphenylphosphine was added in portions of about 2 grams to the stirred reaction mixture. Thereafter the mixture was slowly allowed to come to room temperature and after a while slightly yellow crystals separated from the reaction mixture. The mixture was allowed to stand overnight. Next day the crystals were separated and recrystallised from toluene/ethanol 1:1. Purity: <1% of triphenyl phosphine. Compound >99% pure. Yield 99%.

Preparation of benzyl glyoxal (II D)

Using the procedure of Bestmann et al (Chem. Ber. 2259-2265, 1963) benzyl glyoxal triphenyl phosphazine (10 mmole) was dissolved in THF (30ml) sodium nitrite (1.52 g) was added and the mixture cooled in an ice bath. Over the next 15 minutes the mixture was kept at a temperature of 10 to 15 °C and 18 ml of a 2 M HCl solution was added. The cooling bath was removed and the mixture was stirred until no more nitrogen evolution was observed (1 h). The layers were separated and the organic layer dried over $MgSO_4$. The product was distilled by vacuum distillation. (0.05-0.01 mmHg) and the product collected at 90-110°C. Yield 15-25%.

Scheme III (Figure 34)

Preparation of "Phenyl coelenterazine"

Using the procedure of Cheng Fen Qi et al (J. Chem. Soc. Perkin Trams 1, 1992, p. 1607) amixture of aminopyrazine (I G) 0,173 mmole and benzylglyoxal (II D) 0,26 mmole, 4 and 50 ml aq. 36% HCl was heated at 80°C while stirring during 4 hours. After cooling to room temperature the solvent was removed under reduced pressure. The yellow solid was recrystallised from ethanol and purity checked by NMR (purity >95%). Yield 45%.

**EXAMPLE IV: Testing the Coelenterazine Derivatives**

After its synthesis and lyophilization, the coel400a (described in Example III) was dissolved in 100% ethanol to a concentration of 2 mM. The derivative was further diluted in BRET buffer (PBS $Ca^{2+}/Mg^{2+}$ glucose, aprotinin with or without 10 mM DTT) to a working concentration of 40 $\mu$M or 20 $\mu$M (pre-dilution) and a final concentration of 5 or 10 $\mu$M in the reaction.

Two different constructs were created, in which the EYFP from the original Rluc::EYFP (1st generation) was replaced by either Sapphire or GFPuv. Both constructs were tested separately. Constructs were subcloned in pCDNA3.1 (+) zeo for their expression in mammalian cells.
Constructs used :

pCDNA3.1/ Rluc negative control
pCDNA3.1/ Rluc::Sph positive control
pCDNA3.1/Rluc::GFPuv positive control

A CHO-K1 cell line was transfected using the DNA constructs and LipofectAMINE (BRL/Gibco) in 100 mm dishes (according to the manufacturer's protocol). Forty-eight hours post-transfection, transfected cells were washed, harvested and diluted in BRET buffer (PBS $Ca^{2+}/Mg^{2+}$, glucose, aprotinin with or without DTT 10 mM). Bioluminescent reactions were either analysed using the Spectrofluorometer (Fluorolog-3) or using the BRETCount with appropriate filters. The coelenterazine derivative was used at a concentration of 40 $\mu$M (final 10 $\mu$M or 5 $\mu$M) was added in each well to start the bioluminescent reaction.

When using the Fluorolog-3: $2 \times 10^6$ cells were add per assay (cuvette 1 or 2 ml). The analysis was performed at: 22 - 37 °C, slit at 10 nm, integration time 0.5 - 1 sec, step 2 nm, no excitation, scan between 400 - 600 nm

When using the BRETCount, 50 000 cells per well were added to white or black 96-well plates. The analysis was performed at 20 - 25 ºC, integration time 1 - 5 sec, luminescence mode. The filters used were: for Rluc 410DF80 (from Omega); for GFP moiety D515/50M (from Chroma). Temperature: 20 - 25 °C.

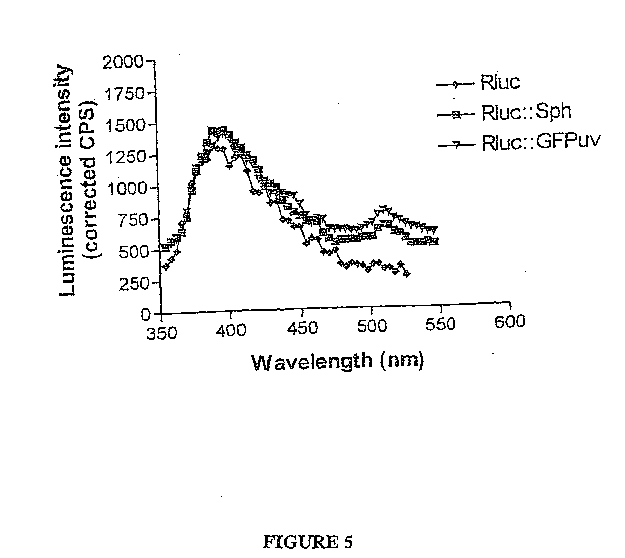

Coel400a was assayed with the BDP using the Fluorolog-3 in order to determine the emission wavelengths. Three DNA constructs were used for expression of BDP: Rluc alone, Rluc::Sph or Rluc::GFPuv, These were transfected in CHO cells. Forty-eight hours after transfection the cells were washed, harvested and put in a cuvette along with the different coelenterazine derivatives for the spectral analysis. Figure 5 represents the spectral analysis for the Coe1400A when processed by Rluc. As expected, Coel400a when processed by Rluc, resulted in an emission peak at 400 nm. Furthermore, it seemed that energy transfer between Rluc and the GFP moiety (Sph or GFPuv) was successful since an emission of approximately 510 nm was detected from the Rluc::Sph or Rluc::GFPuv constructs only. The spectral difference between Rluc and GFP moiety emissions is remarkable. GFPuv appeared to be a better acceptor than Sapphire using this configuration and substrate. During spectral analysis, the emission peak for the GFPuv was always higher compared to Sapphire for a similar Rluc peak height. This is not unusual considering that GFPuv has a higher quantum yield and extinction coefficient than Sapphire for the 400 nm peak (Tsien, 1998). This difference between GFPs means that each GFPs are different in terms of accepting energy transfer.

The new coelenterazine derivatives were tested using the BRETCount. Light filters were selected to accommodate the requirement of the new BDP and FAMs.

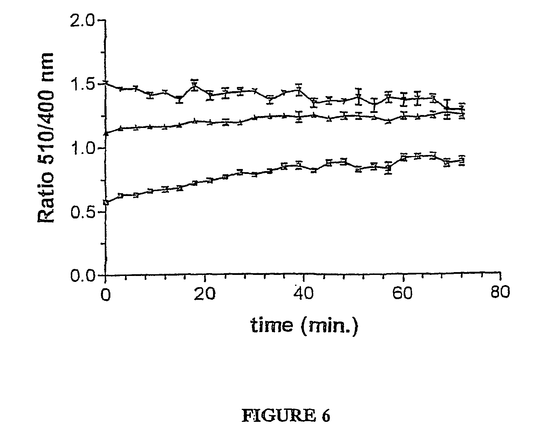

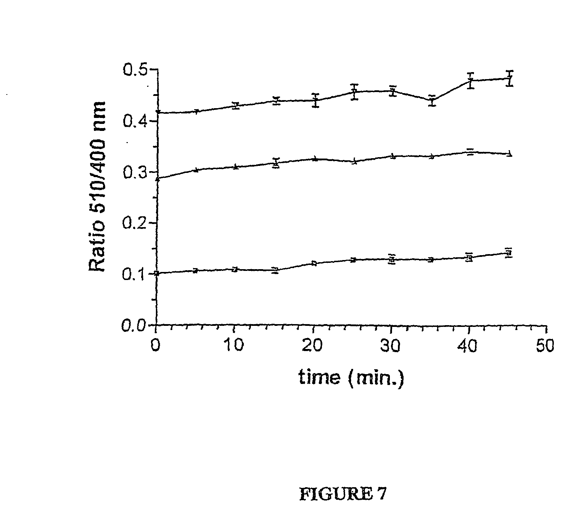

Transfected cells were distributed in either black or white 96-well plate (Optiplate, Packard BioScience, Meriden CT) at a density of 50 000 cells per well in PBS $Ca^{2+}/Mg^{2+}$, glucose, aprotinin and with or without DTT. Figures 6 and 7 represent data obtained for the Coe1400a. Ratios (510/400 nm) at the beginning of the bioluminescent reaction are about 0.6 for Rluc alone, 1.2 for Rluc::Sph and 1.5 Rluc::GFPuv (Figure 6). Rluc::Sph and Rluc::GFPuv are about $2\times$ and $2.5\times$, respectively, higher than Rluc ratio. This is a significant difference in comparison to the previous generation. Rluc::EYFP/Rluc was about 1.6x (see PCT # WO 99/66324). A worker skilled in the art would recognise that since these data are ratiometric data and any small increase is significant.

In Figure 7, the experiment demonstrated in Figure 6 was repeated except a black microplate and read length of 2 sec instead of 1 sec were used. In this case, a Rluc ratio of 0.10, a Rluc::Sph ratio of 0.29 and a Rluc::GFPuv ratio of 0.42 were obtained. This latter result demonstrates that the format used to measure BRET can be important (including the microplate type and the read length).

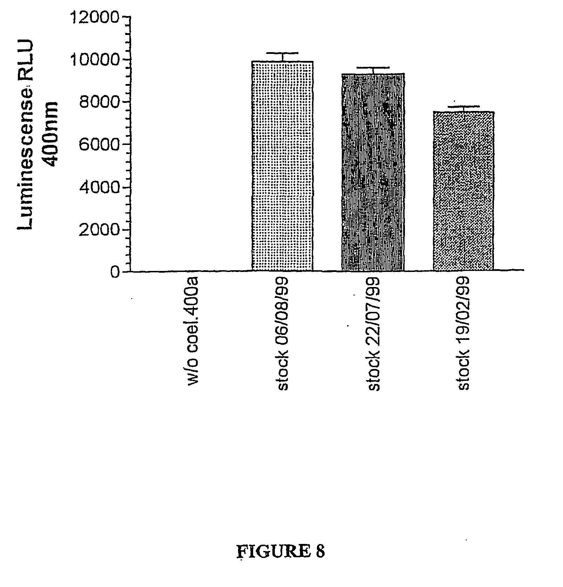

Coelenterazine stability: Three different stock solutions of coelenterazine 400a (dissolved in anhydrous ethanol), prepared at different times, were tested using the BRETCount. CHO cells were transiently transfected with pCDNA3.1-Rluc only. Forty-eight hours later, 50 000 cells in complete BRET buffer were distributed in wells (96-well white Optiplate). Coelenterazine 400a from the various stocks were then added to start the bioluminescent reaction. Figure 8 shows the luminescent signal for the various stocks immediately after addition of the substrate. A reduction of 22 % is observed for the older stock (made Feb.19-1999) compared to a stock freshly made (August 6-1999). Stock made July 22nd 1999 did not demonstrate significant luminescence reduction.

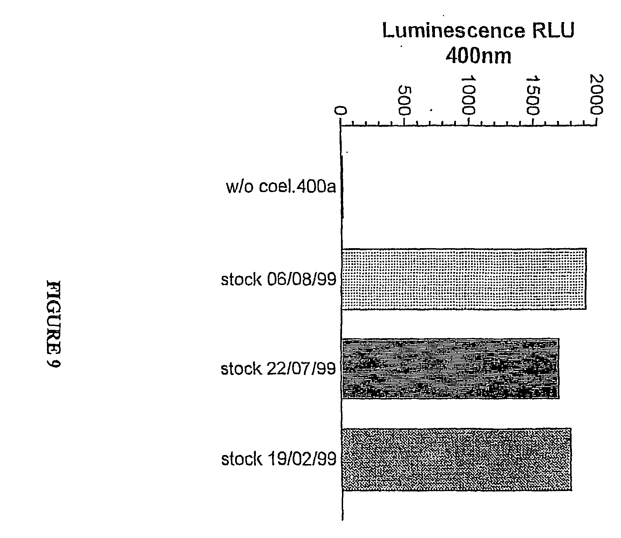

Figure 9 represents the same bioluminescent reactions as in Figure 8 but at t = 5 min after addition of the coelenterazines. In this case no significant difference was observed between the various coelenterazine stocks.

This example demonstrates that coe1400a, once dissolved in ethanol, does not show a significant loss in luminescent potential over one month. Coe1400a stored for a longer period of time (6 months), does show a loss of potential but it remains acceptable and workable for the BRET system.

The Coel400a can be used as a substrate for BDP as part of a BRET system exhibiting a broad spectral separation between BDP and FAM emission wavelengths. The BDP and FAM moieties can be modified in order to improve the light output levels and thereby increase the ease of detection. Other modifications include codon humanisation of GFPuv and Rluc.

## EXAMPLE V: Modification and adaptation of the BDP (Rluc) and FAM (GFP) moieties for the coel400A

According to Forster's law, three intrinsic parameters are important in order to efficiently transfer energy from the donor to the acceptor moieties: 1) the quantum yield (QE) of the donor; 2) the extinction coefficient of the acceptor; and 3) the overlap between emission of the donor and excitation of the acceptor. During the development of the present BRET system it was recognised that total light output was low compared to the basic system. This problem comes from the fact that Rluc when used with the new coelenterazine (coe1400) has a much lower quantum yield (> 100X lower) than coelenterazine *h*. Furthermore, the acceptor moiety (GFP class 1) in the present BRET system has an extinction coefficient 3 times lower than the EYFP. Hence, even if the overlap between donor emission and acceptor excitation is almost perfect, this BRET system energy transfer is not more efficient than for basic BRET. The BRET system of this invention has the advantage of broadened resolution between the donor and acceptor emission wavelengths.

The lowered energy transfer efficiency could mean that longer detection times may be necessary. One solution to attenuate the low light output, is to increase cellular sensor concentration by increasing the expression of the proteins. This was achieved by: 1) humanising the Rluc and the GFP class 1 (GFPwt or GFPuv) and 2) testing various GFP mutants which would be more soluble and thermo-stable, and less sensitive to dimerisation and photoisomerisation.

GFP mutants that could be tested are mentioned in Table 4. The effects of the different mutations tested are described in Table 5.

Another way to improve the system is to find mutations in both the donor and the acceptor that would increase energy transfer efficiencies. A GFP (GFP10 see below) was found that acted as a better acceptor.

Rluc humanisation: tThe cDNA of Rluc was codon humanised using methods previously described (patent # US5874304 and Zolotukhin *et al, J. Virol.* **70**: 4646-4654, 1996). Briefly, humanised Rluc (hRluc) was synthesised using a series of ligated polymerase extended oligonucleotides. hRluc sequence confirmed by DNA sequencing. hRluc was subcloned in pCDNA3.1 zeo (+). Expression of hRluc was compared to Rluc in transiently transfected mammalian cells, at 24 and 48 h post-transfection, using a GFP as internal standard to normalise the transfections.

GFP mutants and humanisation: The construction of all the GFP mutants was based on a commercially available GFP sequence. Mutations were introduced using an *in vitro* site-directed mutagenesis kit, QuickChange™ from Stratagene. EGFP was purchased already codon humanised and having a valine residue after the first methionine to create a Kozak consensus sequence in the encoding nucleic acid. All mutations were carried out using the original EGFP vector (pEGFP-N1, Clontech) as the source of parent nucleic acid. A T65S mutation was first introduced to obtain GFP1. From this mutant three additional mutations (F99S, M153T V163A) necessary for the GFPuv phenotype (GFP4), were incorporated. S202F T203I S147P and L64F were then introduced in both intermediates. GFPuv used for the comparison was purchased from Clontech.

The various GFP mutants were first tested using spectrofluorometric analyses. CHO cells were co-transfected with the mutant DNA and with the pRL-CMV vector as an internal control. Transfections were performed in 100 mm dishes using the LipofectAMINE reagent using 7.5 $\mu$g of mutant DNA and 0.5 $\mu$g of pRL-CMV. Forty-eight hours after transfection, cells were harvested and counted before measuring Rluc activities in the LumiCount using coelenterazine h. Cells were then normalised by dilution according to their specific Rluc activity. The same volume of cells having the same Rluc activity was used in the spectrofluorometer for the spectral analysis. The excitation spectrum was obtained using the following parameters: scan 300-505 nm, em 530 nm slits ex:2 em:5 scan speed 2 nm in time 0.5 sec. For the emission spectrum the following parameters were used: scan 430-600 nm, ex 400 nm slits ex:5 em:2 scan speed 2 nm int time 0.5 sec Photoisomerisation experiments were performed by measuring emission intensity of constant illumination at 400 nm for 30 minutes. After that period, excitation scans were performed on the previously illuminated sample.

GFP mutants 1, 6, 9, 10 and 11 were also tested for their ability to accept energy transfer from the Rluc. They were subcloned in pCDNA3.1 Rluc::EYFP (see PCT WO 99/66324) where EYFP gene was replaced with the GFP mutants using the BamHI-NotI restriction sites, such that all GFP mutants were subcloned in frame with a Rluc gene to form a fusion protein having the structure: Rluc:linker:GFP mutant. The linker region found in between the Rluc and the GFP mutant was the same (9 amino acids in length and composition) for all constructs. CHO-K1 cells were transfected using the DNA constructs and LipofectAMINE (BRL/Gibco) in 100 mm dishes (according to the manufacturer's protocol). Forty-eight hours post-transfection, transfected cells were washed, harvested and diluted in BRET buffer (PBS $Ca^{2+}/Mg^{2+}$, glucose, aprotinin with or without 10 mM DTT). Transfected cells were seeded in white Optiplate (Packard BioScience, Meriden CT) at a density of 50 000 cells per well. Bioluminescent reactions were analysed using the BRETCount with appropriate filters. An aliquot of coelenterazine derivative was added at a concentration of 20 $\mu$M (final 5 $\mu$M) to each well to start the bioluminescent reaction.

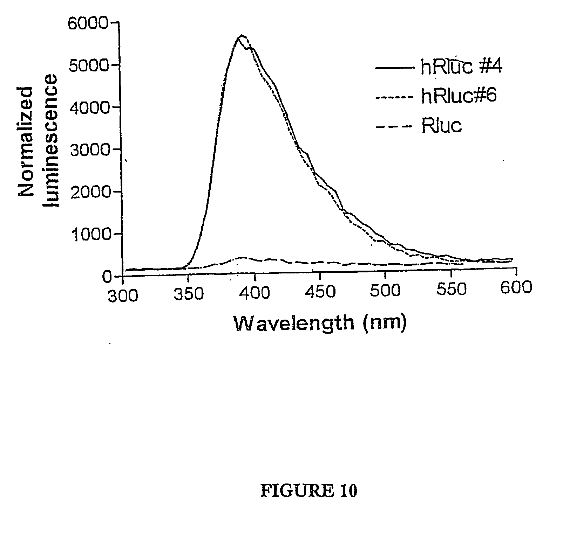

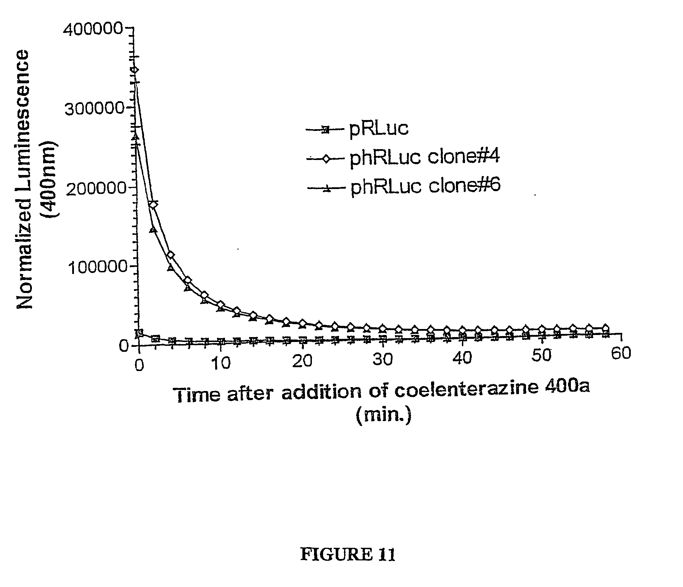

The results of Rluc codon humanisation: After sequencing analyses of the hRluc genes (clone 4 and 6), comparison experiments were performed in live cells (CHO) by transfecting mammalian vectors expressing the Rluc gene or hRluc gene. For each vector, a reporter vector (expressing constitutively a GFP) was co-transfected in order to normalise transfection efficiencies (using the FluoroCount). After normalisation, cells were analysed using the SPEX spectrophotometer and in the BRETCount. Figure 10 shows results obtained with the SPEX. No difference was observed in the position of the emission signal (immediately following addition of coel400a) between the two hRluc clones (#4 and 6) tested and wtRluc. In all three cases, the luminescence signals peak at approximately 400 nm. The same result was observed using the coelenterazine derivative h instead of the Coel400a (data not shown). This experiment was repeated using the BRETCount in order to quantify the increase in signal generated when using hRluc in place of Rluc (Figure 11). At 1 minute following addition of coel400a, a 30 fold difference in gene expression, in favor of hRluc compared to Rluc, was observed.

The results of the GFP mutants: Using standard site-directed mutagenesis techniques and a commercially available GFP as starting material, various GFP mutants were generated (see Table 4). Comparison experiments were performed between mutants in order to determine which one exhibits the best characteristics for the BRET system. Three parameters were taken account: i) energy transfer efficiency; ii) relative brightness (which includes intrinsic fluorescence + cellular expression) ; and iii) photoisomerisation.

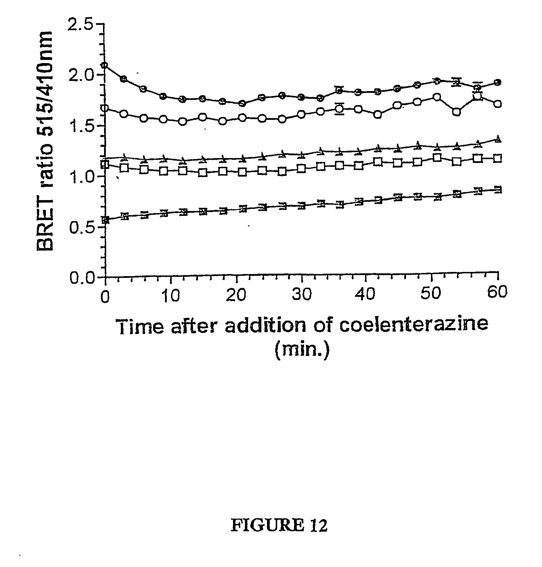

Emission and excitation scans were performed using a spectrofluorometer to identify the GFP mutants having the higher brightness when expressed in live cells both at 25 °C or 37 °C (data not shown). This demonstrated that the GFP mutants were expressed at acceptable levels in live cells. Three GFP mutants GFP1, 6, and 9 (and later 10, 11 see below) were further tested to determine whether they acted as good FAMs. GFP mutant #9 (F64L, S147P, S202F and T203I) was found to be a very good FAM (see Figure 12).

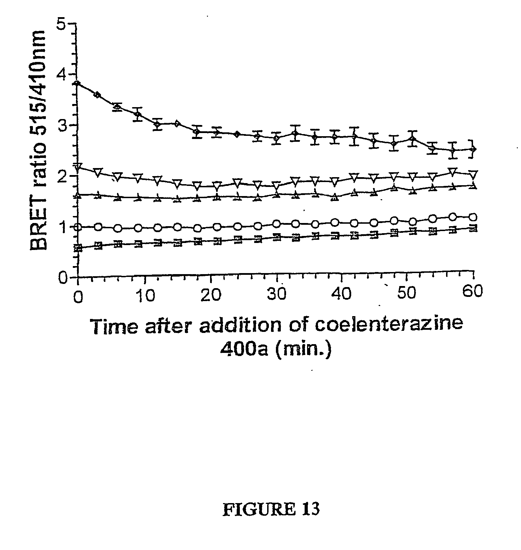

An additional two GFP mutants harbouring a mutation at either 202 or 203 (GFP mutant #10: F64L, S147P and S202F and GFP mutant # 11: F64L, S147P and T203I) were prepared. As described above, each mutant was introduced, in frame with Rluc gene, in an expression vector. The additional sequences were identical to those of the previously prepared GFP mutant containing vectors (i.e. promoter, linker between Rluc and the GFP, etc). The vectors were transfected in CHO cells using Lipofectamine. At 48 hours post-transfection, cells were normalised for their transfection efficiencies by determining Rluc expression following coelenterazine h addition in the LumiCount. Figure13 shows results from the BRETCount analysis. GFP mutant #10 gave the highest ratio among all compared to Rluc alone (negative control) (almost 8 fold at time zero). GFP #11 gave a ratio much lower than GFP#10 and even lower than GFP#9. A list of GFP mutants made is presented in Table 4.

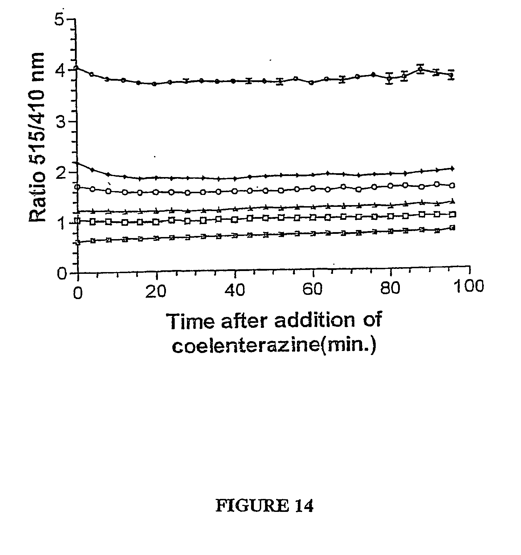

Figure 14 presents the same experiment as for BRETCount analysis of the GFP mutants of Figure 13 except 40 mM DTT was added to help stabilise the ratio over time (see PCT WO 99/66324). For all expressed constructs, addition of DTT helps to stabilise the ratio.

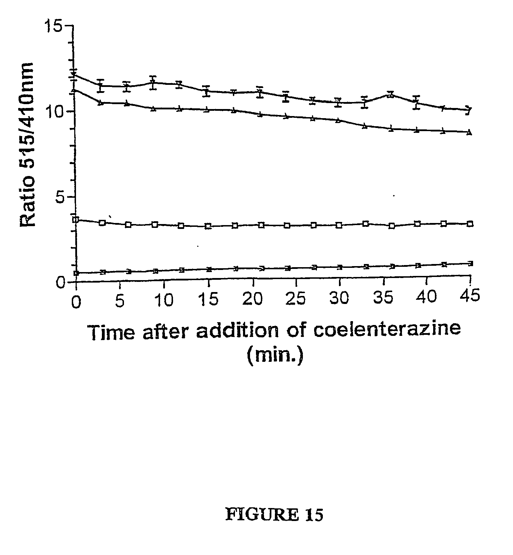

Other coelenterazine derivatives can be used with a BRET system containing a GFP mutant. The only one commercially available and having suitable luminescence properties following processing by Rluc, is the *hcp* derivative, which generates light at 445 nm. Figure 15 shows an experiment using this derivative in CHO cells transiently expressing the Rluc alone or the fusion Rluc::GFP10. Data using *hcp* derivative were compared to data obtained with coel400a derivative. Using *hcp* derivative, a difference in ratio of Rluc and the fusion construct Rluc::GFP10 was observed. This difference was smaller than when the coel400a was used. This is not surprising since the *hcp* coelenterazine generates light at 445 nm which about 45 nm higher than that generated by coe1400a. Therefore, a significant quantity of light is detected by the acceptor channel (through the acceptor filter) resulting in an increase background.

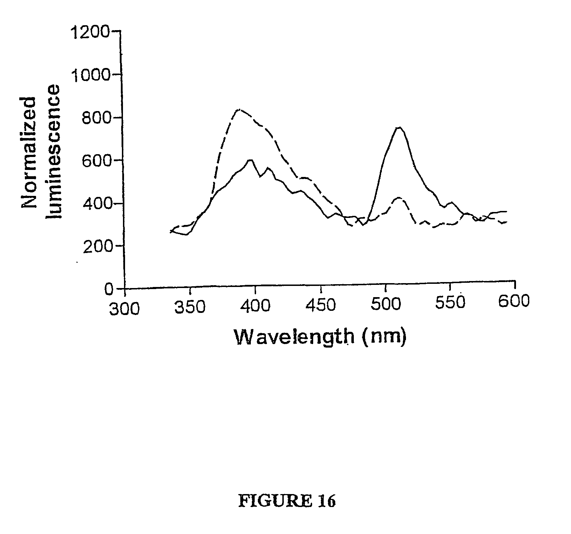

Analysis of GFP10 mutant: During the development of the BRET system of the present invention one GFP mutant (GFP10) was found to be better that the other mutants in accepting energy transfer for the Rluc. Spectral analysis of the fusion construct Rluc::GFP10 is shown in Figure 16. When compared to Rluc::GFP11, Rluc::GFP12 has about 3 - 4 fold difference in accepting energy from Rluc. In comparing BDP and FAM peak heights from GFP11 and GFP10 constructs, the FAM peak height (515 nm) for GFP10 is even higher then for the BDP peak.

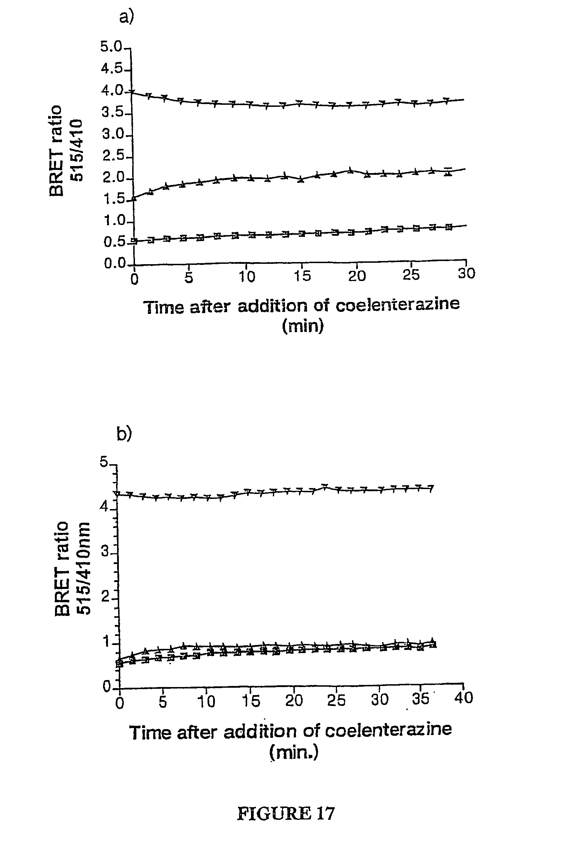

In order to rule out the possibility that this is the result of spontaneous association between Rluc and GFP10, the Rluc::GFP10 construct was expressed in CHO cells and compared to expression of Rluc alone or Rluc + GFP10 (not fused together) in CHO cells. Figure 17a and 17b demonstrate that GFP10 does not interact specifically with Rluc. Figure 17a demonstrates that the ratio of Rluc + GFP10 was higher than for Rluc alone which indicates that spontaneous interaction can occur between the two moieties. This interaction, however, can occur as a result of overexpression of GFP10 in comparison to Rluc protein. When cells were lysed using a small amount of detergent (NP-40 0.1%) and a Dounce potter to open the cells (visual inspection showed more than 95% of lysed cells), the ratio of Rluc + GFP10 decreased to almost the level of Rluc and the ratio of the fusion Rluc::GFP10 didn't change.

## EXAMPLE VI: Protein-protein interaction assays

The previous examples demonstrate that the BRET system of this invention, comprising the GFP mutant, Rluc and Coe1400, performs better than systems in the prior art (using Rluc and EYFP) in terms of signal-to-base ratio (S/B) and DR when the Rluc is fused to the GFP mutant (fusion construct Rluc::GFP). This BRET system was then used in a protein-protein interaction assay. GFP mutants were fused to kai A and B protein and co-transfected along with Rluc-kaiB in BHK cells. Subclonings were performed as for the constructs described in PCT Application No. WO 99/66324. BHK cell line was transfected using the DNA constructs and LipofectAMINE (BRL/Gibco) in 100 mm dishes (according to the manufacturer's protocol). Forty-eight hours post-transfection, transfected cells were washed, harvested and diluted in BRET buffer (PBS Ca$^{2+}$/Mg$^{2+}$, glucose, aprotinin 2 $\mu$g/ml with or without 10 mM DTT). Transfected cells were seeded in white Optiplate (Packard BioScience, Meriden CT) at a density of 50 000 cells per well. Bioluminescent reactions were analysed using the BRETcount. Coel400a derivative, at a concentration of 20$\mu$M, was added in each well to start the bioluminescent reaction (final coelenterazine concentration was 5 $\mu$M).

Figure18a and 18b demonstrate that, as expected, Rluc-kaiB interacts with GFP-kaiB. Kai B is known to interact with itself (see Xu *et al*, 1999) and to kai A, at a much lower levels (EMBO J. 18: 1137-1145, 1999). Cells expressing Rluc:kaiB and GFP:kaiB had a much higher ratio than cells expressing Rluc:kaiB and GFP:kaiA. As expected, cells expressing Rluc:kaiB alone had the lowest ratio. The use of DTT did not cause a significant change in the assay results: the ratios were relatively stable in absence of DTT. The use of DTT depends on the type of assay. These results show that the BRET system using Rluc and the GFP mutant along with coel400a can be used successfully in protein-protein interaction assays.

**EXAMPLE VII: calculating energy transfer efficiencies - quantification of BDP and FAM emission lights**

A 2-detector TopCount was modified in order to measure BRET. This prototype was later referred to as BRETCount. Bandpass interference filters were positioned under each TopCount PMT (between the PMT and the detector mounting plate). Furthermore, the TopCount software was modified to allow each well of a microplate to be read by each PMT. These modifications allow the sequential detection of light at specific wavelengths emitting from the samples. Therefore, for each well (or sample) two light output values were obtained that corresponded to each light filter. TopCount is a temperature controlled instrument which is particularly useful when performing cell-based assays or time-course experiments.

For the examples presented herein, the bandpass interference filters were designed based on emission spectra of BDP and FAM, as indicated below: 1) for the acceptor emission (fluorophore): 410DF80 (Omega Optical Inc, Brattleboro VT); and 2) for the donor emission (bioluminescent protein): 515/50 nm (Chroma Technology Corp., Brattleboro VT). Alternative light filter combinations can be designed, depending on the BDP and FAM used in the assay.

In this example, BRET is measured by dividing the light output for the 515 nm filters by the 410 nm filters (acceptor/donor). The instrument parameters used for the BRETCount were: 1) assay temperature: 25 °C; 2) Single Photon Counting mode; and 3) read length of 2 sec for each PMT. Cells (CHO-K1) were transfected using LipofectAMINE (Life Technologies, Rockville MD) with either pRL-CMV DNA (that constitutively expresses Rluc) or a vector pCDNA3.1/Rluc::GFPuv) constitutively expressing the fusion construct Rluc::GFPuv (GFPuv comes from Clontech, Palo Alto CA). Two days post-transfection, cells were harvested, counted and distributed into 96-well plates (white Optiplate from Packard Instruments, Meriden CT) at a density of 50 000 cells per well in PBS + $Ca^{2+}$ + $Mg^{2+}$ + glucose + 2 $\mu$g/ml aprotinin.

Typically transfection efficiencies were in the range of 50 - 60 % (calculated by counting GFP fluorescent cells under a fluorescence microscope) when the manufacturer's protocol was used for transfection. Coelenterazine 400a was then added (final concentration 5 $\mu$M) to each well to initiate the bioluminescent reaction. Light outputs were determined using the BRETCount with the 400 nm light filter (donor emission) and the 510 nm light filter (acceptor emission) and using the normal TopCount (without the use of any filter), which will determine light output for all wavelengths (open spectrum).

Eight measurements were made for each type of transfected cell and for each type of measurement for a total of 48 measurements per assay. Table 6 shows the mean values for the different assay conditions. Instrumental parameters were: for BRETCount 25 °C, read time of 2 sec, photon counting mode; for the TopCount 19 °C, read time of 1 sec, photon counting mode.

**Example VIII: Altering emission filters to improve spectral performance of the BRET system**

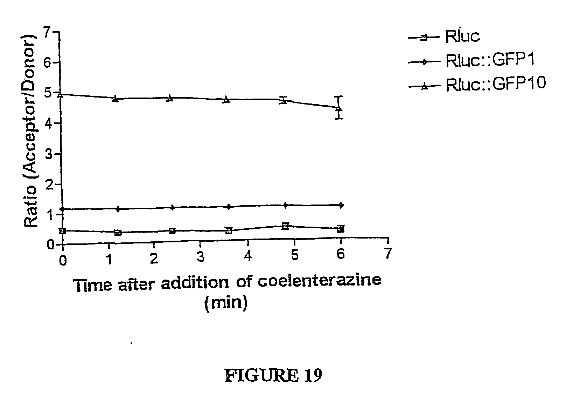

CHO cells were transfected using LipofectAMINE™ (Life technologies) using the manufacturer's protocol in 100 mm dishes, with DNA vectors expressing either Rluc, the Rluc::GFP1 fusion or the Rluc::GFP10 fusion (see Figure 19). Except for the FAM (GFP), both fusion constructs were similar in structure. They had the same linker between Rluc and GFP and they were subcloned in the same expression vector (pCDhTA3.1/zeo; Invitrogen) using the same restriction sites. Forty-eight hours post-transfection, cells were washed with BRET buffer (PBS with $Ca^{2+}$, $Mg^{2+}$, glucose, 2 $\mu$g/ml aprotinin and 10mM DTT), harvested, counted and resuspended in BRET buffer at a density of 500, 000 cells per ml. Transfected cells were then distributed into white 96-well plates (Optiplate, Packard Instrument Company) at a density of 50,000 cells per well. Fifty microliters of Coel400a (diluted at 20 $\mu$M) was added in each well to start the bioluminescent reaction (final concentration of Coel400a: 5 $\mu$M). Total volume was 200 $\mu$L.

Microplates were then introduced into a Victor2 instrument (Perkin Elmer). When used in dual luminescence mode, this instrument is capable of detecting the BRET signals. Instrument parameters were: 2 second read time per filter, normal aperture, focus at 8mm. In the experiment demonstrated in Figure 19, the D410/80 (Chroma Technology Corp.) and GG475 long pass (Chroma Technology Corp. Brattleboro, VT) filters were used for the detection of donor and acceptor emissions. The spectral characteristics at the filters (blocking agent, bandpass, transmission peak, etc.) were chosen according to the emission spectrum of the system (Example III). The filters were installed in a Victor2 instrument.

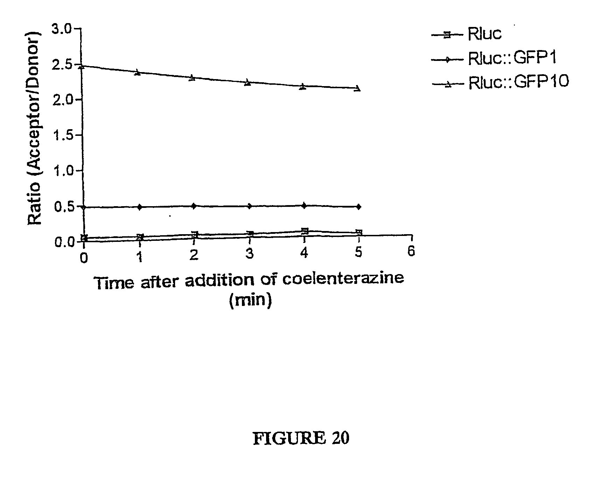

The Rluc, Rluc::GFP1 and Rluc::GFP10 fusion constructs generated BRET ratios of 0.46, 1.17 and 4.94, respectively (at time 0). Therefore, S/Bs (fold increase in the BRET ratio of the fusion construct over the Rluc alone) were 2.5 and 10.7 for the Rluc::GFP1 and Rluc::GFP10, respectively. In the experiment demonstrated in Figure 48, the same transfected cells were then used again with a different filter pair: D410/80 (Chroma Technology Corp.) for the donor and D515/30 (Chroma Technology Corp.) for the acceptor emission. All the other parameters were kept the same as described for Figure 20. Using this new filter pair, we obtained BRET ratios of 0.057, 0.49 and 2.48 for the Rluc, Rluc::GFP1 and Rluc::GFP10, respectively. S/Bs were then 8.6 and 43.7 for the Rluc::GFP1 and Rluc::GFP10 fusion constructs, respectively. These results demonstrate that optimisation/adaptation of the filter spectral characteristics to the donor and acceptor emissions can significantly improve the overall performance of the BRET system.

**Example IX: Constitutive protein-protein interaction assay.**

CHO-K1 cell line was transiently transfected using the DNA constructs and LipofectAMINE™ (BRL/Gibco) in 100 mm dishes as described in the manufacturer's protocol. Forty-eight hours post-transfection, transfected cells were washed, harvested and diluted in BRET buffer (PBS $Ca^{2+}$/$Mg^{2+}$ + glucose + 2 $\mu$g/ml aprotinin) at a density of 2,000,000 cells per ml. Bioluminescent reactions were analysed using the BRETCount with appropriate filters. BRETCount is a modified TopCount capable of measuring light coming of a well at specific wavelength (for the donor and acceptor). Since the BRETCount used in this example did not have optimised emission filters for this BRET system, the BRET ratio was corrected using an algorithm (Angers, S. *et al* (2000) *Proc. Natl. Acad. Sci. USA* **97**: 3684-3689).

Transfected cells were added (50,000 cells, in BRET buffer, per well) to the wells in white 96-well plates (Optiplate, Packard Instrument). The analysis was performed at 25 °C, integration time: 2 sec per wavelength in luminescence mode. The filters used were: for Rluc emission 410DF80 (from Omega); for GFP D515/50M (from Chroma). Coel400a, at a working concentration of 40 $\mu$M (final 5$\mu$M), was added in each well to start the bioluminescent reaction. The final reaction volume was 50 $\mu$l. Assays were performed in quadruplicate.

The DNA constructs used to transfect the cells were: pCDNA3.1/Rluc-kaiB, pCDNA3.1/GFP-kaiB, pCDNA3.1/GFP-kaiA and the positive control was pBRET+. Cells were transfected with either pCDNA3.1/Rluc-kaiB or pBRET+ alone or co-transfected with pCDNA3.1/Rluc-kaiB + pCDNA3.1/GFP+kaiA or pCDNA3.1/Rluc-kaiB+ pCDNA3.1/GFP-kaiB, using equal amounts of DNA in each case.

Kai proteins are clock proteins from cyanobacteria involved in circadian rhythm. KaiB is known to interact with itself but not with kaiA (Xu, Y. *et al* (1999) *Cell Biol.*, **96:** 151-156). The protein domain of kaiB responsible for this homodimerisation, was fused to either Rluc or GFP. KaiA was fused to GFP only. All fusion constructs were subcloned into pCDNA 3.1/zeo mammalian expression vector (Invitrogen). Cells were transfected with the various DNA (see protocol) and analysed forty-eight hours after transfection. Cells expressing only the Rluc-kaiB fusion generated a very low BRET ratio since no GFP was present to accept the energy transfer from the Rluc. Cells expressing Rluc-kaiB and GFP-kaiA also had a low BRET ratio but higher than Rluc-kaiB alone. As indicated above, kaiB does not interact with kaiA and should not generate a signal. Cells expressing the Rluc-kaiB and GFP-kaiB exhibited a very high energy transfer efficiency (BRET ratio) because of the kaiB homodimerisation which brings the Rluc into proximity with the GFP. These results are summarised in Figure21.

**EXAMPLE X: G protein-coupled receptor - $\beta$-arrestin interaction assay.**

A BRET assay was developed to monitor G protein-coupled receptor (GPCR) activation. This assay uses the natural interaction of $\beta$-arrestin for GPCRs. When a ligand binds to a GPCR, it triggers downstream effectors (adenylate cyclase, $IP_3$/DAG), which modulate cellular second messenger levels. After its activation, the GPCR is desensitised by the addition of phosphorylation group(s) at its C-terminus and/or its third intracellular loop. $\beta$-arrestin recognises these phosphorylated sites and binds to the receptor to start the internalisation process. Internalisation is important to uncouple the ligand from the receptor once internalised and to recycle the receptor to the plasma membrane. Currently, over eighty percent of known GPCRs use this process.

The Rluc (BDP) was fused to the C-terminus of the GPCR and the GFP (FAM) was fused to the C-terminus of $\beta$-arrestin. Therefore, shortly after ligand binding (5-30 minutes) the BRET ratio should increase due to binding of $\beta$-arrestin to the GPCR, which brings the GFP in proximity to Rluc. This assay is an excellent tool for GPCR orphan screening.

HEK293 cell line were transiently transfected using the DNA constructs and LipofectAMINE™ 2000 (BRL/Gibco) in 100 mm dishes as described in the manufacturer's protocol. Forty-eight hours post-transfection, transfected cells were washed, harvested and diluted in BRET buffer (PBS +$Ca^{2+}$/$Mg^{2+}$ + glucose + 2 $\mu$g/ml aprotinin) at a density of 2 000 000 cells per ml. Bioluminescent reactions were analysed using the BRETCount with appropriate filters. BRETCount is a modified TopCount capable of measuring light coming of a well at specific wavelength (for the donor and acceptor. Since the BRETCount used in this example did not have optimised emission filters for this BRET system, the BRET ratio was corrected using an algorithm (Angers, S. *et al* (2000) *Proc. Natl. Acad. Sci. USA* **97**: 3684-3689).

Transfected cells were added (50 000 cells (in BRET buffer) per well) to the wells in white 96-well plates (Optiplate, Packard Instrument). Agonists (isoproterenol (iso) or Arg[8]-vasopressin (AVP)) were then added in half of the wells. In the other half, BRET buffer, alone, was added. Cells were then incubated for 10-20 minutes at room temperature before adding the coel400a. The analysis was performed at 25 °C, integration time 2 sec per wavelength in luminescence mode. The filters used were: for Rluc emission 410DF80 (from Omega); for GFP D515/50M (from Chroma). Coe1400a, at a working concentration of 40 $\mu$M (final 5 $\mu$M), was added to each well to start the bioluminescent reaction. The final reaction volume was 50 $\mu$l. Assays were done in quadruplicate.

The DNA constructs used to transfect the cells were: pCDNA3.1/β2AR:-Rluc (h) (codon humanised Rluc), pCDNA3.1/β3AR-Rluc (h), pCDNA3.1/V2-Rluc (h) and pCDNA3.1/β-arrestin2-GFP. Cells were co-transfected with one of the Rluc vectors and the pCDNA3.1/β-arrestin2-GFP vector using a DNA ratio of 8 $\mu$g:42 $\mu$g (Rluc vector:GFP vector). For all fusion constructs, a 6 amino acid linker was inserted, between the Rluc or GFP and the receptor (β2, β3 or V2) or β-arrestin2.

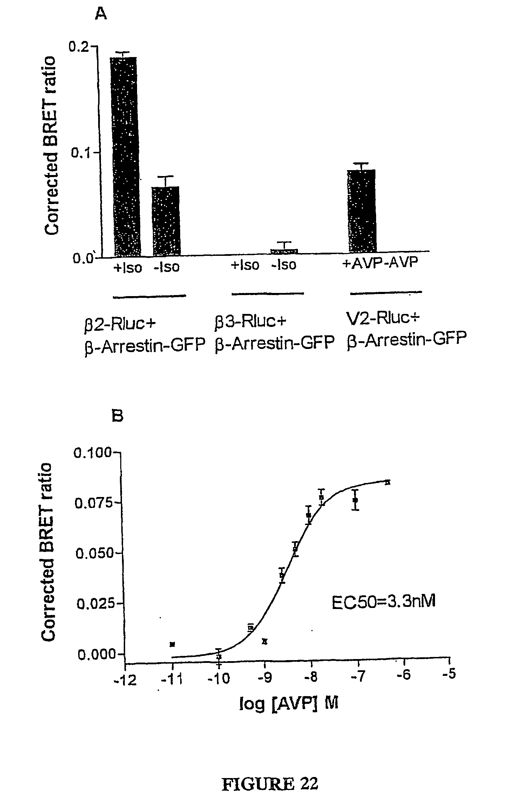

HEK-293 cells were co-transfected with various combinations of Rluc and GFP fusion constructs. The agonists isoproterenol (Iso) and AVP were used to activate their respective receptor (β-arrestin receptor or V2 receptor). Receptor activation eventually led to desensitisation and internalisation of the receptor as described above. As expected, the BRET ratio increased in the presence of the agonists but remained low in their absence, except for β3-arrestin receptor (β3-AR)-Rluc (see Figure 22). The β3-AR is known to not bind the β-arrestin used in this assay and was therefore used as a negative control. The BRET ratio for β2-AR-Rluc in the absence of isoproterenol was high. This was attributed to the fact that in this particular assay the cells had not been starved or it may be attributed to spontaneous receptor activation. Figure 22b shows a dose response curve obtained from cells transfected with V2-Rluc and β-arrestin-GFP and stimulated by AVP. An $EC_{50}$ of 3.3 nM was observed, which matches with values found in the literature. Taking these results into consideration in combination with the fact that eighty percent of currently known GPCRs interact with β-arrestin, this interaction assay presents a powerful tool to screen orphan receptors.

## EXAMPLE XI: The Use of a Coelenterazine *hcp* derivative in BRET

A BRET assay was designed using Rluc as the BDP and GFP1 as the FAM which makes use of different coelenterazine derivatives. Coelenterazine derivatives *h, hcp* and DeepBlueC (DBC also called Coel400a) were compared in this example. Derivative *hcp* and *h* generate light with an emission peak at 445 nm and 470-480 nm, respectively, when catalyzed by Aequorin and *Renilla* luciferase (Shimomura *et al., Biochem. J.* **261,** 913-920, 1989; Hart *et al., Biochemistry* **18,** 2204-2210, 1979; Matthews et al., *Biochemistry* **16,** 5217-5220, 1977; Shimomura *et al., Biochem. J.* **296,** 549-551, 1993; Matthews *et al., Biochemistry* **16,** 85-91, 1977). DBC generates light with an emission peak at approximately 390- 400nm (Hart *et al., Biochemistry* **18,** 2204-2210, 1979).

CHO cells were transfected with pRluc::GFP1 construct (positive control (+)) and the pRluc construct (negative control (-)) using LipofectAMINE™ 2000 (Life Technologies) in 100 mm dish according to the manufacturer's protocol. Both positive and negative control DNAs have been made previously and are now commercially available from BioSignal Packard under the name pBRET+ and pRluc-N1 (h), respectively. Forty-eight hours after transfection, cells were washed with PBS+, harvested with versine, counted and suspended in PBS+ at a density of one million cells per ml. Transfected cells were distributed into white 96-well plate (Optiplate, Packard Instrument Company) at a density of 50 000 cells per well in 150 $\mu$l of PBS+. To start the bioluminescent reaction, 50 $\mu$l of DBC (BioSignal Packard, Montreal), *hcp* (Molecular Probes Inc, OR) or *h* derivatives (Molecular Probes Inc, OR) at a working concentration of 20 $\mu$M (in PBS +) were added last in each well to a final concentration of 5$\mu$M. Total assay volume: 200 $\mu$l.

Plates were introduced in the Fusion™ microplate analyzer instrument (Packard Instrument Company) to measure FAM and BDP emissions.

**Instrument settings**: PMT voltage:1100 volts Gain: 25 Read time: 1sec Plate: 96-well Temperature: 25°C. The emission filter for the BDP was 410 nm with a bandpass 80 nm and the emission filter for the FAM was 515 nm with bandpass 30 nm. Both filters were purchased from Chroma Technology Corp. (Brattleboro, VT). The results represent the mean, +/- SEM, of three experiments. Graphs were generated using GraphPad PRISM software. In each case the background emission, from the wells containing only DBC in PBS+, was subtracted from the measured emissions.

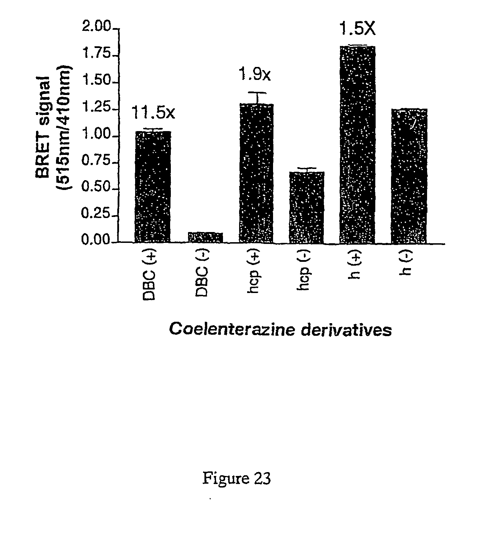

The results presented in Figure 23 demonstrate that coelenterazine derivatives other than DeepBlueC can be used in this BRET assay. The use of DeepBlueC generated higher S/B of ratio (see numbers above bars) in comparison to *hcp* and *h.* S/B ratios were calculated by dividing the value obtained for the positive control ratio by the ratio for the negative control.

## EXAMPLE XII: Rluc Mutants

Liu and Escher (*Gene* **237**, 153-159, 1999; PCT W/O 00/20619)have demonstrated that one secreted Rluc mutant in which a cysteine residue was changed to alanine, generated higher light output than wild type coelenterazine was used as substrate. The cellular secretion properties and biochemical properties was analyzed by measuring relative light intensities of the mutant which was compared to secreted Rluc. It was found that this mutant (C124A; based on Rluc wild type numbering) generated an increased light output in both the secreted Rluc and in Rluc. It was demonstrated that the mutation increased the cellular half-life of the mutant enzyme.

Since both the bioluminescent enzyme and its substrate determine the emission wavelength of the bioluminescent reaction (Inouye and Shimomura, *Biochem. Biophys. Res. Com.* **233**, 349-353, 1997), this example demonstrates that Rluc mutants will still emit light around 390-400nm when utilizing DeepBlueC as for wild type Rluc. Four Rluc mutants were made: C124A, C124S, C124V and C124Y. The mutant C124A has been already described by Liu *et al* (called SRUC3). These mutants were generated using site-directed mutagenesis (QuickChange kit from Stratagene) according to manufacturer protocol and oligonucleotides (Life technologies). Codon humanized Rluc gene in pBlueScriptII vector (Stratagene) subcloned between the XbaI and EcoRI restriction sites (see above for details of subcloning), was used as template for the mutagenesis. Once made, the mutants were subcloned from pBlueScriptII to pCDNA3.1 zeo (-) (Invitrogen) using XbaI and EcoRI. Sequence analyses were performed on each mutant to verify whether they demonstrated the expected DNA sequence (ABI Prism 310 Genetic Analyzer from Perkin Elmer).

The effect of the substitutions at the position 124 on light output in live cells was texted. Wild type Rluc and mutants Rluc (in pCDNA3.1 zeo) were co-transfected along with pGFPend-N1 vector (Packard Instruments Company) at a DNA ratio of 7:1 in CHO cells using LipofectAMINE (according to manufacturer protocol) in 100mm dishes. Forty-eight hours after transfection, transfected cells were washed and harvested in PBS+ (PBS Mg$^{2+}$ 1mM , Ca$^{2+}$ 1mM, glucose 1000g/ml aprotinin 2$\mu$g/ml).

GFPemd fluorescence from transfected cells were determined using a FluoroCount (Packard Instrument Company) using the following instrument settings: 1100 volts, 2sec read length, gain 1, excitation filter 485nm, emission filter 530nm. For this purpose transfected cells were seeded in a black 96-well ViewPlate (Packard Instrument Company) at a density of 50,000 cells per well in 200$\mu$l PBS+ (in triplicate). GFPemd fluorescence was used to normalize transfection efficiencies between transfected cells. Normalized cell quantities (about 100,000 cells) in PBS+ were added in white 96-well Optiplate (Packard Instrument Company) to determine total luminescence output of Rluc wild type and Rluc mutants expressed in cells (in triplicate). DeepBlueC was added in each well (final concentration 5$\mu$M) to start the bioluminescent reaction and luminescence levels were quantified using a LumiCount (Packard Instrument Company). Instrument settings: 1100volts, gain 1, read length 0.5sec. Final assay volume 200$\mu$l. Bioluminescence was determined immediately after addition of DeepBlueC (see Table 8). Rluc mutants C124A gave the highest luminescence (almost 3 times higher compared to Rluc wild type). C124Y mutant generated practically no bioluminescence compared to Rluc wild type and background levels (50-60 RLU). Bioluminescence from C124V was too dimmed to be useful. These two mutants were not further analyzed.

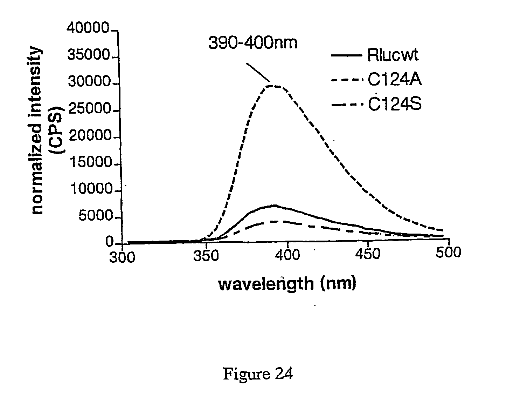

Emission scans were performed to determine the position of emission peaks for Rluc mutants C124A and C124S (see Figure 24). Transfected cells (total of 1.8 X10$^6$) were added in a 2ml cuvette of a spectrofluorometer (FluoroLog3.2 from Instruments S.A., Inc). DeepBlueC (final 5$\mu$M) was added to start the bioluminescent reaction. Emission scans were recorded using the following settings: Scan 300-500 nm, emission slit 10nm, increment: 2nm, integration time 0.2 sec, Excitation lamp off.

Figure 24 shows that the Rluc mutants C124A and C124S were still emitting around 390-400nm when DeepBlueC was used as substrate as for Rluc wild type. Furthermore, as shown by Liu *et al,* the C124A Rluc mutant has a higher emission intensity compared to wild type coelenterazine (about 4.5X when comparing surface under the curves). As determined using the LumiCount, the C124S mutant generated lower light intensity compared to wild type Rluc. These results demonstrate that a single mutation in Rluc primary structure can affect light emission for a given substrate. Finally, these results demonstrate that Rluc mutants can be used with DeepBlueC.

This improvement in light intensity of the mutant C124A can result from an overall increase of cellular expression or an increase in the quantum yield of the mutant or both. In order to demonstrate that the mutation C124A affects the quantum yield of the reaction, the following BRET experiments were peformed. Since BRET is an energy transfer process using dipole-dipole resonance between a donor and acceptor moieties (????8), the efficiency of the energy transfer is dependent of the quantum yield of the donor. If the mutation C124A affects the quantum yield of Rluc, an improved energy transfer (BRET signal) will be generated compared to wild type Rluc. For this purpose, wild type and mutant Rluc genes were fused in frame to an acceptor moiety (GFP1 mutant see above).

Rluc wild type and Rluc mutant genes were subcloned into pGFP-C1 vector (BioSignal Packard) from pCDNA3.1 zeo (see above) using the restriction sites ApaI and BamHI. All final DNA constructs had the same overall GFP::Rluc structure and the same linker (14 amino acid long) between GFP and Rluc moieties. Fusion GFP::Rluc construct vectors were transfected in CHO cells using LipofectAMINE (Life Technologies) using manufacturer protocol in 100mm dishes. Co-transfection of Rluc and GFP was used as negative control. Forty-eight hours post-transfection, cells were harvested in PBS+ and re-suspended at a density of million cells per ml. Fifty thousands cells were distributed in White 96-well Optiplate (in triplicate). DeepBlueC (final concentration of 5$\mu$M) was added on cells to start the bioluminescence reaction. Final assay volume 200$\mu$l.

Bioluminescence levels were determined using a Fusion Microplate analyzer (Packard Instrument Company) with the following settings for BRET2: read length 1 sec per well per channel, PMT 1100 volts, gain 25, temperature 25°C, emission filters donor and acceptor were 410/80 and 515/30 (Chroma Technology Corp.), respectively. BRET signals (ratio of light output at 515nm divided by light output at 410nm) were calculated for each fusion construct and compared to BRET signal for a non-interacting Rluc for GFP (negative control). Results represent the mean +/- SEM. of triplicate wells. Graphs were generated using GraphPad PRISM software. In all cases, background levels (wells with only DBC in PBS+) were subtracted.

BRET signal at time 0 for the fusion construct using the wild type Rluc was at 1.04 and at 1.09 for the fusion construct using the C124S mutant. BRET signal for the negative control (Rluc + GFP) was at 0.068. This difference between BRET signal of wild type Rluc fusion construct and C124A fusion construct is small but significant. These results indicate (taking into account Liu et al of the same mutant) that both the cellular expression properties and quantum yield are modified by the substitution. The BRET signal for the fusion construct using the C124S was at 0.81. These results demonstrate that it is possible to improve the energy transfer properties of a donor-acceptor pair by modifying the primary structure of the donor.

**EXAMPLE XIII: Detection of G protein coupled receptor (GPCR) oligomerization using biological membranes, derived from mammalian cells, by (BRET)**

To study oligomerization of the β2-adrenergic receptor (β2-AR) the Rluc and the GFP10 were genetically fused to the C-terminus end of the β2-AR as described in Angers et al (*Proc. Natl. Acad Sci.* USA **97**, 7:3684-3689). β2-AR: Rluc and β2-AR:GFP10 constructs were transfected a DNA ratio of 1:1 in HEK 293T using standard $Ca^{2+}$ phosphate transfection procedure (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press). Forty-eight hours after transfection, crude membranes were prepared. Cells from a 100mm dish were washed twice with PBS and lysed in 10 ml of lysis buffer (5 mM tris 2 mM EDTA pH 7.4 supplemented with protease inhibitor mixture consisting of 5 μg/ml leupeptin, 10 ug/ml benzamidine, and 5 ug/ml soybean trypsin inhibitor) for 10 mins at 4 °C, the lysate was then subjected to mechanical stress using a Polytron. Homogenates were centrifuged at $500 \times g$ for 5 min at 4 °C and the supernatant was centrifuged at $45,000 \times g$ for 20 min afterwhich pellets were washed twice in the same buffer.

BRET assays were performed in white 96-well plate (Optiplate, Packard Instrument Company) using fifteen μg of crude purified membrane per well in PBS. Fifty μl of coelenterazine h derivative (Molecular Probes, Eugene, OR) at a concentration of 20μM was added in each well to start the bioluminescence reaction, wherein the total assay volume was 200μl. Rluc and GFP10 emissions were quantified using a microtiterplate reader (BRETCount instrument see Angers, S. *et al* (2000) *Proc. Natl. Acad Sci. USA* **97**: 3684-3689) set-up with filters specific for the GFP10 fluorescence and the Rluc emission. The BRET signal was determined using the algorithm described in Angers, S. *et al* (2000) *Proc. Natl. Acad Sci. USA* **97**: 3684-3689.

A low BRET signal (> 0.05) was obtained when membrane from cells singly transfected with either β2AR-Rluc or β2AR-GFP were used upon addition of coelenterazine *h* derivative (not shown). However, when both receptor fusion constructs were expressed, a robust BRET signal was obtained which is best explained by the constitutive presence of β2-AR oligomers in HEK293T cells. In an attempt to control for the specificity of the interaction, two different distantly related GPCRs, the chemokine CCR5 receptor and the GABA-R2, were fused to Rluc and used in the same kind of experiments with the β2AR-GFP10. When two distantly related receptors were studied, little hetero- interactions could be measure in membrane prepared from cells indicating that oligomerization of GPCR is selective.

**EXAMPLE XIV: Alternative Apoptosis Sensor**

In this example, a BRET apoptosis sensor was prepared by introducing a caspase-3 recognition site in the linker region of the GFP-Rluc fusion construct. Upon induction of apoptosis, caspase-3 recognizes and cleaves the linker region, thereby separating Rluc from EGFP. Therefore, induction of apoptosis will decrease the BRET ratio over time.

pGFP1::Rluc was made by introducing the codon humanized Rluc gene from the pCDNA3.1/Rluc (h) vector into the pGFP1-C2 vector (see Technical data sheet from BioSignal Packard , Montreal). GFP1 is a mutant of the Green Fluorescent Protein (GFP) having a unique mutation at position 64 (amino acid positioning is relative to the GFP wild type) where the phenylalanine has been replaced by a leucine. pCDNA3.1/Rluc (h) was digested with Apal and BamHI. After digestion, products were separated on an agarose gel. A band corresponding to Rluc (h) gene (around 920 base pair in length) was cut from the agarose gel and purified using the Qiaquick spin kit (Qiagen). The purified band was then subcloned in the pGFP1-C2 vector digested with Apal and BamHI. The linker between GFP1 and Rluc was then shortened by digesting the pGFP1::Rluc vector with BspE1 followed by a fill-in reaction using Klenow enzyme to blunt the end. After the Klenow reaction, the product was purified using Qiaquick spin kit, digested with EcoRV, purified another time (Qiaquick) and then ligated using ligase. This procedure removed 47 nucleotides from the original pGFP1-C2 vector and creates the following linker between GFP1 and Rluc:

TCCGGATCAAGCTTGCGGTACCGCGGGCCCTCTAGAGCCACCATG .

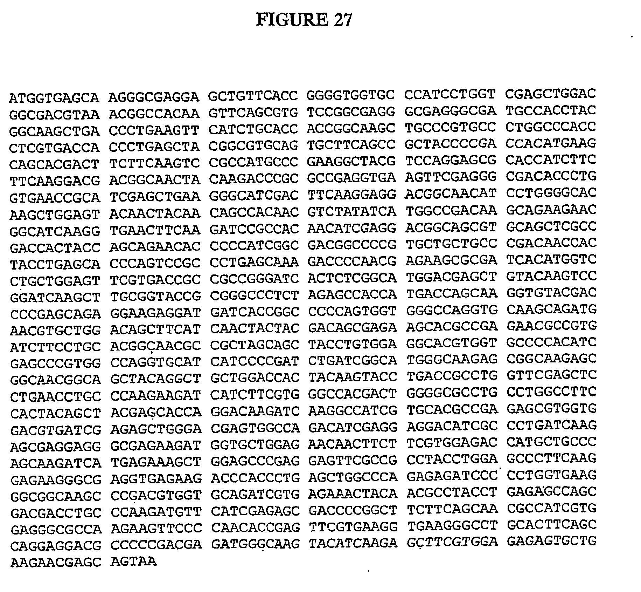

This linker contains convenient unique restriction site that can be used to subclone fragments between the GFP1 and Rluc genes. These restriction sites are (5' to 3' orientation): BspE1, HindIII, KpnI, SacII, ApaI and XbaI. Figure 27 shows a DNA sequence encoding the GFP:Rluc fusion protein containing a unique 14 amino acid linker region between the GFP and the Rluc.

The caspase-3 site was introduced in the pGFP1::Rluc vector using annealed complementary oligonucleotides. We annealed two complementary oligonucleotides encoding the following caspase-3 site: Gly-Asp-Glu-Val-Asp-Gly. Only the sequence Asp-Glu-Val-Asp is needed for recognition by caspase-3. Caspase-3 cuts between the Asp and Gly residues.

Sense oligonucleotide: 5' AGCTTGGGCGACGAGGTGGACGGCGGGCC 3'

(SEQ ID NO: )

Antisense oligonucleotide: 5' ACCCGCTGCTCCACCTGCCGC 3' (SEQ ID

NO:)

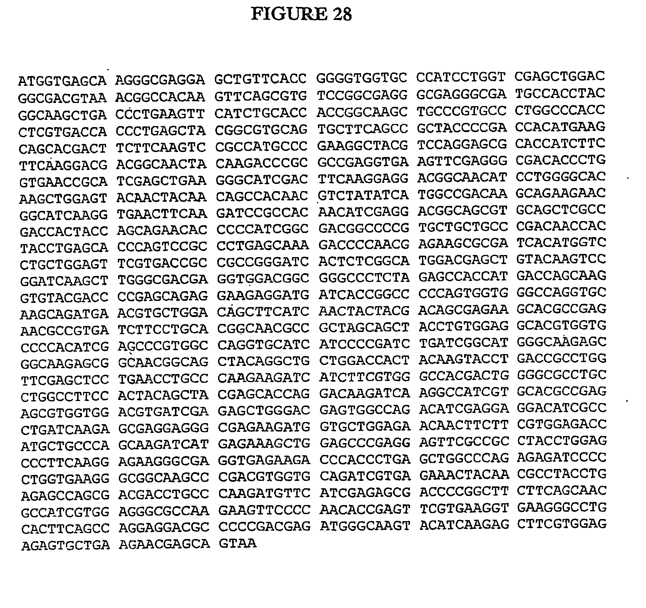

The above two oligonucleotides (sense and antisense) were synthesized (by BRL/Gibco-Life Technologies). The two oligonucleotides are complementary to each other and were annealed together using standard molecular biology techniques (Ausubel, F.M. et al.: Current protocols in molecular biology, Vol. 1 (1995) John Wiley & Sons, Inc.; Sambrook, J. Fritsch, E.F. & Maniatis, T.: Molecular Cloning: A laboratory manual, 2nd ed. (1989) Cold Spring Harbor Laboratory Press). The oligonucleotides were engineered in order to generate cohesive ends after their annealing. HindIII is found at the 5' end and ApaI is found at the 3' end. The double stranded product generated after annealing was introduced into the GFP1::Rluc construct digested using HindIII and ApaI. HindIII and ApaI are unique restriction sites found in the linker region between the GFP and Rluc genes. The final structure is GFP1:caspase-3:Rluc and as shown in Figure 28. Since originally the GFP::Rluc was already in a expression vector compatible for transfection and expression of the protein in mammalian cells (pGFP background), no further modification was required. The pGFP1:caspase-3:Rluc plasmid DNA used for the transfections was purified using the Maxi-Prep kit from Qiagen.

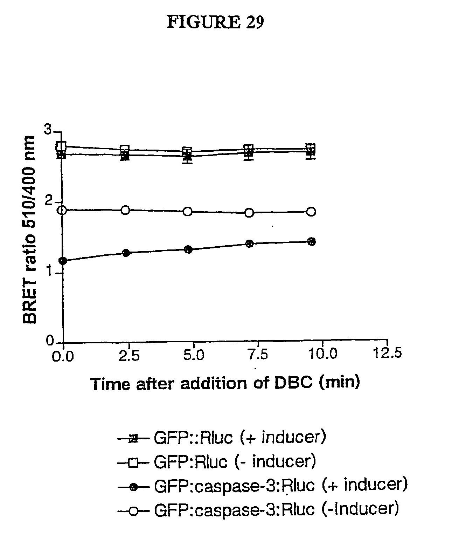

PGFP1:caspase-3:Rluc DNAs and the original plasmid pGFP1::Rluc (without the caspase-3 site) were transfected into HeLa cell using LipofectAMINE (BRL/Gibco-Life technologies) in 100mm dishes and 8 $\mu$g of plasmid DNA as described in the manufacturer's protocol. Twenty-four hours post-transfection transfected cells were harvested and distributed into 96-well microtiterplate (white Optiplate from Packard) at a density of 30 000 cells per well. The following day, cells were washed with PBS and were induced to undergo apoptosis by adding 100$\mu$l of Iscove media without serum and phenol red (BRL/Life Technologies) containing staurosporine at a final concentration of 1$\mu$M (+ inducer; see Figure 29) or 100$\mu$l of Iscove alone (-inducer). Cells were incubated for 5 hours at 37°C. After this period, 50 $\mu$l of BRET buffer (PBS Ca$^{2+}$/Mg$^{2+}$ + glucose without aprotinin) and 50$\mu$l of DeepBlueC (20 $\mu$M) were added to start the bioluminescence reaction. Total assay volume was 200$\mu$l. Light emissions were detected using the BRETCount (read length of 1 sec. for each filter, SPC mode, temperature 25°C). The results represent the mean +/- SEM of quadruplicate wells. The graph in Figure 29 was generated using GraphPad PRISM software. In all cases, background levels (wells with only DBC in PBS+) were subtracted.

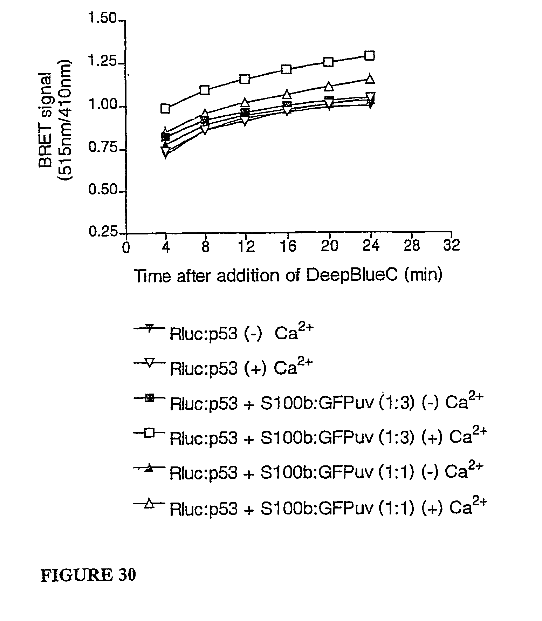

Figure 30 represents the ratio changes in HeLa cells after 5 hours of apoptosis induction by staurosporine. A significant BRET ratio change (0.72 unit at time zero) occured when cells were transfected with the apoptosis sensor (pGFP1:caspase-3:Rluc) and then induced to undergo apoptosis by staurosporine. As expected, the ratio decreased indicating that cellular caspase-3 recognized and cut the cleavage site between the GFP1 and Rluc moieties. No change in BRET ratio was observed in cells transfected with the pGFP::Rluc or in cells transfected with the pGFP:caspases-3: Rluc without staurosporine (-inducer).

The: GFP1::Rluc construct was sequenced in its entirety using an ABI automated sequencer (ABI Prism 310 Genetic Analyzer from Perkin Elmer).

**EXAMPLE XVI: Calcium Sensor**

Calcium is an important second messenger molecule used by G-protein couple receptor (GPCR) to activate downstream effectors. Many GCRPs can generate cellular calcium influx via G$\alpha$q G-protein upon activation by their ligand. Amplitude of calcium influx is often measured to determine GPCR activation. Many fluorescent compounds such as Fluo-3 and Fura-2 have been engineered to bind calcium (Methods in cell biology: A practical guide to the study of calcium in living cells. Vol.4 (1994)) Academic Press, Inc.). Binding of calcium onto these molecules changes their fluorescence properties. Even if these compounds could be very sensitive, they suffer from the disadvantage that they have to be loaded into the cells before performing the assay. Furthermore, not all the cells can be loaded with enough compounds for calcium measurement.

In the following, we describe the generation of a BRET calcium sensor based on the specific interaction of p53 and S100b proteins. These proteins can be synthesized by cells therefore removing the need to load cells with a chemical compound prior performing the assay. S100B is part of the calmodulin family. Members of this family are known to bind calcium using the EF-hand domain (Allore et al., *J. Biol. Chem.* **265** 15537-15543, 1990). When S100B binds calcium, it undergoes structural changes, which permits its binding to the tumor suppressor protein p53. Delphin et al, (*J. Biol. Chem.* **274,** 10539-10544, 1999), have characterized the protein domains on both p53 and S100b involved in the their interaction. These domains have been isolated using recombinant technology and fused to donor and acceptor BRET moieties. Amino acids of p53 involved in the interaction with S100b are localized at the C-terminus of the protein between amino acid 320-393. This domain has been amplified using RT-PCR (see below) and subcloned at the C-terminus of the Rluc. S100b protein has been cloned by RT-PCR and subcloned at the N-terminus of the acceptor GFPuv. These fusion constructs were amplified and purified from bacteria using a suitable expression vector. Purified fusion constructs were used in cell-free assays.

Amplification by PCR and subcloning of p53 domain needed for the interaction with S100b: The human p53 domain was cloned and the p53 domain responsible for binding to S100b (amino acids 320-393) was amplified by PCR (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press). The template used for PCR was pCMV-p53 (from Clontech Cat# K6004-1). PCR amplification was performed using the following primers (Life Technologies):

Sense primer: 5'-GGC<u>GGATCC</u>AAAGAAGAAACCACTGGAT-3'

               BamHI                 p53 coding sequence

Antisense primer: 5'-GCC<u>TCTAGA**TCA**GTCTGAGTCAGGCCC</u>-3'

                 XbaI        p53 coding sequences (reverse strand)

The stop codon of p53 is shown in bold. The primers were designed with unique restriction sites (BamHI and XbaI) in their sequences in order to facilitate subcloning. The primers were designed according to the p53 DNA sequence found in Genebank under the access number: U94788. PCR were performed in 50μl using a GeneAmp PCR system 9600 instrument from Perkin Elmer (see Table 9 for PCR conditions).

PCR reactions were carried out as follows:

Denaturation step at 95°C for 2 min.
Hot start at 75°C

| | | |
|---|---|---|
| 5 cycles of | denaturation step | 94°C 20 sec. |
| | annealing step | 50°C 20sec. |
| | elongation step | 72°C 30sec. |
| 25 cycles of | denaturation step | 94°C 20 sec. |
| | annealing +elongation step | 72°C 30sec. |

Final elongation step 72°C 10min

Amplified product was separated by standard agarose gel electrophoresis and the band corresponding to p53 domain (228 base pairs in length) was gel purified using the Qiaquick spin kit (Qiagen). The purified band was then subcloned into an intermediate vector pCDNA3.1/Rluc::EYFP (see PCT/CA99/00561) by replacing the EYFP with the amplified p53 domain. For this purpose, pCDNA3.1 /Rluc::EYFP was digested with BamHI and XbaI to remove EYFP and the vector band was gel purified using Qiaquick.

Amplified p53 domain was subcloned in frame with the Rluc gene into the linearized pCDNA31./Rluc vector to make the final Rluc::p53 fusion protein. The fusion gene was entirely sequenced using BigDye ABI Prism kit (PE Applied Biosystems, Foster City, CA) and ABI Prism 310 Genetic Analyzer (Perking Elmer) to ensure no mismatch compared to original sequences. The amino acid sequence of the linker between Rluc and p53 was RARDP (single amino acid code). The Rluc:p53 fusion gene was retrieved from the pCDNA3.1/Rluc:p53 vector using restriction digests (NheI and ApaI), gel purified using Qiaquick, blunted with Klenow enzyme (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press) and subcloned into pQE 32 vector linearized with SmaI to make the final pQE32/Rluc::p53 vector. Once subcloned into pQE32, the Rluc::p53 fusion gene was automatically fused to a His-tag sequence at the N-terminus of Rluc::p53 gene. The His-tag sequence was used to purify the Rluc::p53 fusion gene using Ni-column (The QIAexpressionist: A handbook for high-level expression and purification of 6xHis-tagged proteins. July 1998. Qiagen). After transformation of pQE32/Rluc:p53 in bacteria (M15), IPTG was added to induce expression of the His-tag Rluc::p53. The fusion protein was then purified with Ni-bead column according to the manufacturer's protocol (The QIAexpressionist: A handbook for high-level expression and purification of 6xHis-tagged proteins. July 1998. Qiagen). Protein determinations were performed using the BioRad Protein assay (from BioRad). Purity of the His-tag purified fusion protein was achieved by standard SDS-PAGE. Purified His-tag Rluc::p53 fusion protein was used in BRET assay (see below).

RT-PCR amplification and subcloning of s100b protein: human S100b cDNA has been cloned without its stop codon by RT-PCR (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press). The template used for the RT-PCR was single-strand cDNAs (first strand) made using the SuperscriptII kit (Life Technologies) according to the manufacturer's protocol and 1$\mu$g of human brain mRNA (Clontech). A first RT-PCR amplification using the cDNAs as template has been performed using the following primers (synthesized by Life Technologies) :

Sense primer: 5'-CGGCGCTAGC**ATG**TCTGAGCTGGAGAAG

Nhe I        (h) S100b

The start codon of the S100b is shown in bold.

Antisense primer: 5'-GCCGGTACCGTCTCATGTTCAAAGAACTCG

Kpn I  (h) S100b coding sequence (reverse strand)

The primers were designed according to the sequence of S100B found in Genbank under the access number: M59486. PCR were performed in 50$\mu$l using a GeneAmp PCR system 9600 instrument from Perkin Elmer (see Table 10 for PCR conditions). A second amplification has been performed using the reaction mix of the first RT-PCR as template.
PCR reactions were carried out as follow:
Denaturation step at 95°C for 2 min.
Hot start at 75°C

| 5 cycles of | denaturation step | 94°C 20 sec. |
| | annealing step | 49°C 20sec. |
| | elongation step | 72°C 30sec. |
| | | |
| 25 cycles of | denaturation step | 94°C 20 sec. |
| | annealing +elongation step | 72°C 30sec. |

Final elongation step 72°C 10min
Amplified product was resolved by standard agarose gel electrophoresis and the band corresponding to S100b (278 base pairs in length) was gel purified using the Qiaquick spin kit. The purified band was then subcloned into an intermediate vector pCDNA3.1 hyg/GFPuv at the N-terminus of the GFPuv. GFPuv is a mutant GFP sold by Clontech. For this purpose, pCDNA3.1hyg/GFPuv was linearized with NheI and KpnI enzymes and gel purified with Qiaquick. Amplified S100b was subcloned in frame with GFPuv gene into the linearized pCDNA3.1 hyg/GFPuv vector to make the final S100b:GFPuv fusion protein. The fusion gene was entirely sequenced using BigDye ABI Prism kit (PE Applied Biosystems, Foster City, CA) and ABI Prism 310 Genetic Analyzer (Perking Elmer) to ensure no mismatch compared to original sequence. The amino acid sequence of the linker between S100b and GFPuv genes was TVPVEK (single amino acid code). The S100b:GFPuv fusion gene was retrieved from the pCDNA3.1hyg/S100b:GFPuv vector using restriction digests (NheI and ApaI), gel purified using Qiaquick, blunted with Klenow enzyme (4,5) and subcloned into pQE 31 vector linearized with SmaI to make the pQE31/S100b:GFPuv. Once subcloned in pQE31, S100b:GFPuv fusion gene was automatically fused to a His tag sequence at the N-terminus of S100b:GFPuv fusion gene. His-tag sequence was used to purified the S100b:GFPuv fusion gene using Ni-column (7). After transformation of pQE31/S100b:GFPuv in bacteria (M15), IPTG was added on cells to induce expression of the His-tag S100b:GFPuv. Fusion protein was then purified with Ni-bead column according to manufacturer protocol (7). Protein determinations were performed using the BioRad Protein assay (from BioRad). His-tag purified fusion protein was obtained by standard SDS-PAGE. Purified His-tag S100b:GFPuv was used in BRET assay.

In vitro BRET assay: His-tag purified Rluc::p53 (320ng of protein) was mixed with His-tag purified S100b:: GFPuv (260ng or 780ng of protein) in 150$\mu$l of PBS (without $Ca^{2+}$ and $Mg^{2+}$) containing 10mM DTT in white 96-well Optiplate (Packard Instrument Company). In presence of $Ca^{2+}$ (see symbol (+) in Figure 30), $CaCl_2$ was added at a final concentration of 1mM. Fifty $\mu$l of DeepBlueC (also called Coel400a) at a concentration of 20$\mu$M were added into wells to start the bioluminescent reactions. Final concentration of DeepBlueC was 5$\mu$M. Total assay volume was 200$\mu$l. Donor and acceptor light emissions were quantified using the BRETCount with the following instrument settings: read length 1sec per well per filter, SPC mode, temperature:25°C, donor filter: 410DF80 (Omega Opticals Inc), acceptor filter: D515/50m (Chroma Technology Corp,). Results represents the mean +/- SEM of quadruplate wells. The graph in Figure 30 was generated using Graphpad PRISM software.

Figure 30 shows that in the presence of $Ca^{2+}$, an interaction occurred between S100B and p53. In absence of $Ca^{2+}$, the BRET signals of Rluc:p53 + S100b:GFPuv (protein ratio 1:1) and Rluc:p53 + S100b:GFPuv (protein ratio 1: 3) are similar to the BRET signal of Rluc:p53 alone indicating that Rluc:p53 does not interact with S100b:GFPuv in absence of $Ca^{2+}$. In the presence of 1mM $Ca^{2+}$, the BRET signal for the Rluc:p53 + S100b:GFPuv (1:1) increased significantly (open triangle) compared to the condition in the absence of $Ca^{2+}$ (closed triangle). A similar result was obtained for the Rluc:p53 + S100b:GFPuv (1:3) (compared open and closed squares). The BRET signal for the Rluc: p53 + S100b:GFPuv (1:3) in presence of $Ca^{2+}$ was higher compared to the Rluc:p53 + S100b:GFPuv(1:1) meaning that not all the Rluc:p53 were titered by the S100b:GFPuv in the Rluc:p53 + S100b:GFPuv 1:1 condition. Since GFP mutants and Rluc do not interact together even in presence of $Ca^{2+}$ and since S100b is known to bind p53 in presence of $Ca^{2+}$, the increase in the BRET signal indicates that an interaction is occurring between S100B and p53. This experiment demonstrates also that GFPuv can be used as an acceptor in BRET assay based on the use of DeepBlueC.

**EXAMPLE XX: β-Arrestin**

To demonstrate the structural versatility of BRET, BRET signals generated using the GFP:β -arrestin2 were compared with β-Arrestin2:GFP in BRET GPCR-Arrestin assays.

*β-arrestin2:GFP1 fusion construct*

Cloning of the human β-arrestin2

Human β-arrestin2 cDNA was cloned without its stop codon by RT-PCR (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press). The template used for the RT-PCR was single-stranded cDNA (first strand) prepared using the SuperscriptII kit (Life Technologies) according to manufacturer's protocol and 1 $\mu$g of human brain mRNA (Clontech). The first RT-PCR amplification using the cDNAs as template was performed using the following primers.

**Sense primer: 5′ GAG GCT GCG AGC GAG CCG GAA 3′ (SEQ ID NO:)**

**Antisense primer: 5′ CCC CCA TTG ACA GTG GGG ATC TT 3′ (SEQ ID NO:)**

A second amplification (nested PCR) was performed using the amplified product of the first amplification as template and the following primers (synthesized by Life Technologies) :

Sense primer: 5'-<u>GCT AGC</u> GCC ACC *ATG GGG GAG AAA CCC GGG* (SEQ ID NO:)

The Nhe I restriction site is underlined and the β-Arrestin2 coding sequence is in italics. Start codon of the β-arrestin2 is shown in bold.

Antisense primer: 5' <u>GGT ACC</u> GGA GCC *GCA GAG TTG ATC ATC ATA GTC* 3' (SEQ ID NO:)

The Kpn I restriction site is underlined and the β-Arrestin2 coding sequence (reverse strand) is in italics.

The primers for the RT-PCR were designed according to the sequence of β-arrestin2 found in Genbank under the access number: NM004313. RT-PCR reactions were performed in 50 μl using a GeneAmp PCR system 9600 instrument from Perkin Elmer (see Table 11 for PCR conditions).

PCR reactions were carried out as follows:

• FIRST RT-PCR

Denaturation step at 94 °C for 2 min
Hot start at 75 °C

| 25 cycles of | denaturation | 94 °C 20 sec |
| | annealing + elongation steps | 72 °C 1.5 min |

Final elongation step 72 °C 10 min

• SECOND PCR

Denaturation step at 94 °C for 2 min.
Hot start at 75 °C

| 5 cycles of | denaturation step | 94 °C 20 sec |
| | annealing step | 57 °C 20 sec |
| | elongation step | 72 °C 1.5 min |
| 25 cycles of | denaturation step | 94 °C 20 sec. |
| | annealing +elongation step | 72 °C 1.5 min |

Final elongation step 72 °C 10 min

The amplified DNA product of the second amplification was resolved by standard agarose gel electrophoresis and the band corresponding to β-arrestin2 (1230 base pairs in length) was gel purified using the Qiaquick spin kit (Qiagen). The purified band was then subcloned in the shuttle vector pCR from the Zero Blunt TOPO cloning kit (Invitrogen Cat#K2800-20) according to the manufacturer's protocol.

The recombinant vector was used to tranform bacteria using a standard protocol. Positive clones were identified by restriction digest of isolated vector DNA, using the restriction sites engineered in the PCR primers (NheI and KpnI) as identifiers. Two clones were entirely sequenced using BigDye ABI Prism kit (PE Applied Biosystems, Foster City, CA) and ABI Prism 310 Genetic Analyzer (Perking Elmer) to ensure that there was no mismatch compared to the original sequence. One positive clone in the pCR vector was digested with NheI and KpnI enzymes. Digestion products were separated using agarose gel electrophoresis and the band corresponding in length to the β-arrestin2 coding sequence was gel purified (Qiaquick). The purified band was then used to make the final pCDNA3.1hyg /β-arrestin2:GFP1 vector as described below.

Final β-arrestin-2:GFP fusion construct: the pCDNA3.1/hyg β-arrestin2 (rat):GFP1 mammalian expression vector harboring the β-arrestin2 (rat):GFP1 fusion construct was digested with NheI and KpnI to remove the β-arrestin2 (rat) gene. Digestion products were separated by agarose gel electrophoresis and the band corresponding to the vector was excised, purified (Qiaquick) and used to subclone the purified β-arrestin2 DNA (see above) at the NheI and KpnI sites. The resulting pCDNA3.1hyg\ β-arrestin2 (human):GFP1 vector was then used to transfect mammalian cells for use in a BRET assay. The β-arrestin2:GFP1 fusion construct had a linker, encoding six amino acids (GSGTGS), between the β-arrestin2 and GFP1 genes. The pCDNA3.1 hyg/ β-arrestin2:GFP1 vector DNA was amplified in bacteria and purified using the Maxi-prep DNA kit from Qiagen.

*GFP1:β-arrestin2 fusion construct*

**Introduction of a stop codon in the human β-arrestin2 by PCR**

Since previously the human β-arrestin2 gene was amplified without its stop codon, we re-amplified this gene again by PCR in order to introduce a stop codon at the C-terminus (Ausubel, F.M. et al.: Current protocols in molecular biology, Vol. 1 (1995) John Wiley & Sons, Inc.; Sambrook et al: Molecular Cloning: A laboratory manual, 2nd ed. (1989) Cold Spring Harbor Laboratory Press). The template used for the PCR was vector pCR TOPO/β-arrestin2 made previously (see above). Primers (from Life Technologies) were designed based on the human β-arrestin2 gene found in Genebank (access number: NM004313). A stop codon has been added in the antisense primer in order to introduce this feature in the final construct. Unique restriction sites have been engineered in the primer sequences to simplify subcloning procedure of the amplified product. PCR were performed in 50 μl using a GeneAmp PCR system 9600 instrument from Perkin Elmer (see Table 12 for PCR conditions).

*Sense primer:* 5'-<u>GGTACC</u>GGATCC**ATG**GGGGAGAAACCCGGG

       KpnI                   (h) β Arrestin2 coding sequence

The start codon of the β arrestin2 is shown in bold.

*Antisense primer:* 5'-GCC<u>TCTAGA</u>**CTA**GCAGAGTTGATCATCATAGTC

              XbaI            (h) β Arrestin2 coding sequence (reverse strand)

The stop codon of the β-arrestin2 is shown in bold.
PCR reactions were carried out as follows:
Denaturation step at 94°C for 2 min.
Hot start at 75°C

| | | |
|---|---|---|
| 5 cycles of | denaturation step | 94°C 20 sec. |
| | annealing step | 55°C 20sec. |
| | elongation step | 72°C, 1.3min. |
| 25 cycles of | denaturation step | 94°C, 20 sec. |
| | annealing +elongation step | 72°C, 1.3min. |

Final elongation step 72°C, 10min
PCR amplified product was resolved by standard agarose gel electrophoresis and the band corresponding to β-arrestin2 (around 1230 base pair in length) was gel purified using the Qiaquick spin kit (Qiagen). The purified band was then subcloned into the shuttle vector pCR (Invitrogen) according to the manufacturer's protocol. After transformation in bacteria using standard protocols, positive clones were identified by restriction digest using the site engineered in the PCR primers (KpnI and XbaI). Two clones were entirely sequenced using BigDye ABI Prism kit (PE Applied Biosystems, Foster City, CA) and ABI Prism 310 Genetic Analyzer (Perking Elmer) to ensure no mismatch compared to the original sequence. One positive clone in the pCR vector was digested with KpnI and XbaI enzymes. Digestion products were separated using an agarose gel and the band corresponding in length to β-arrestin2 was gel purified (Qiaquick). The purified band was then used to make the final pCDNA3.1hyg /GFP1:β-arrestin2.

PCR amplification of the GFP1 without its stop codon: GFP1 cDNA has been amplified by PCR (1,2) without its stop codon using as template the commercially available pGFP - C1 vector (BioSignal Packard). Primers (from Life Technologies) were designed based on the GFP1 sequence provided with the vector. Unique restriction sites have been engineered in the primer sequences to simplify subcloning procedure of the amplified product. PCR were performed in 50µl using a GeneAmp PCR system 9600 instrument from Perkin Elmer (see Table 13 for PCR conditions).

Sense primer: 5'-<u>GCTAGC</u>GCCACC**ATG**<u>GTGAGCAAGGGCGAG</u> (SEQ ID NO:)

        NheI                           GFP1

Start codon of theGFP1 is shown in bold.

Antisense primer:  5'-<u>GGTACC</u>GGAGCCC<u>TTGTACAGCTCGTCCATG</u>  (SEQ ID NO:)

                       KpnI                             GFP1

PCR reactions were carried out as follows:
Denaturation step at 94°C for 2 min.
Hot start at 75°C

| 5 cycles of | denaturation step | 94°C, 20 sec. |
| | annealing step | 53°C, 20sec. |
| | elongation step | 72°C, 1.3min. |
| 25 cycles of | denaturation step | 94°C, 20 sec. |
| | annealing +elongation step | 72°C, 1.3min. |

Final elongation step 72°C, 10min

Amplified GFP1 DNA was was gel purified using the Qiaquick spin kit (Qiagen) and subcloned in the shuttle vector pCR (Invitrogen) according to manufacturer protocol. After transformation in bacteria using standard protocol, positive clones were found by restriction digest using the restriction site engineered in the PCR primers (NheI and KpnI). Two clones were then entirely sequenced using BigDye ABI Prism kit (PE Applied Biosystems, Foster City, CA) and ABI Prism 310 Genetic Analyzer (Perking Elmer) to ensure no mismatch compared to the original sequence. One positive clone in pCR vector was digested with NheI and KpnI enzymes. Digestion products were separated using an agarose gel and the band corresponding in length to GFP1 was gel purified (Qiaquick). The purified band was then used to make the final pCDNA3.1hyg /GFP1:β-arrestin2.

GFP:β-arrestin2 fusion construct: The pCDNA3.1/hyg expression vector (Invitrogen) was first digested with NheI and KpnI. Digested vector was gel purified and was used to subclone the amplified GFP1 (without stop codon) to make the pCDNA3.1 hyg/ GFP1 vector. Clones were screened by restriction digest (KpnI and NheI). One positive clone was digested with KpnI and XbaI in order to subclone the amplified β-arrestin2 gene to make the final pCDNA3.1 hyg/ GFP1:β-arrestin2 vector. Clones were screened again by restriction digest to find positive clones. The final GFP1: β-arrestin2 fusion construct had a linker between the β-arrestin2 and GFP1 genes composed of six amino acids (sequence in single letter code: GSGTGS). The pCDNA3.1 hyg/ GFP1:β-arrestin2 vector DNA was amplified in bacteria and purified using the Maxi-prep DNA kit from Qiagen.

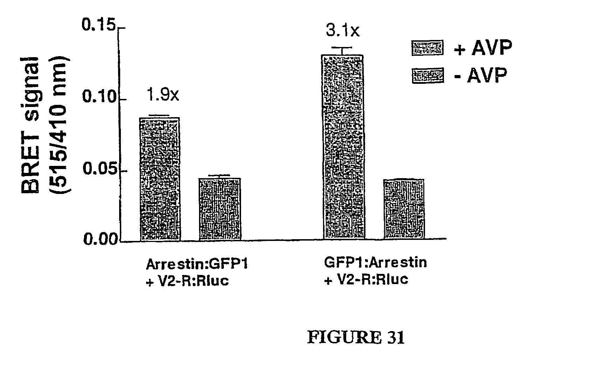

BRET assays: HEK293 cell line were transiently co-transfected using the donor and acceptor fusion construct DNAs and LipofectAMINE 2000 (Life Technologies) in 100mm dishes as described in the manufacturer's protocol. For the transfections, a DNA ratio of 4µg :46µg (donor fusion construct: acceptor fusion construct) was used. The donor fusion constructs were the V2-R:Rluc (h) fusion constructs and the acceptor fusion construct was either the pCDNA3.1hyg/GFP1:β-arrestin2 or the pCDNA3.1hyg/β-arrestin2:GFP1. Forty-eight hours post-transfection, transfected cells were washed, harvested and diluted in BRET buffer (PBS Ca$^{2+}$ 0.9mM/Mg$^{2+}$ 0.5mM, glucose 1000g/l, pyruvate 36mg/l (Life Technologies Cat. # 18287) + 2μg/ml aprotinin) at a density of two million cells per ml. Fifty thousand transfected cells per well were added to white 96-well plates (Optiplate, Packard Instrument Company). The agonist Arg$^8$-vasopressin (AVP) from Bachem was added in half of the wells (see Figure 31: +AVP) to a final saturating concentration of 50nM. In the other half, BRET buffer was added (-AVP). Cells were then incubated for 20 minutes at room temperature in a total volume of 37.5μl. After the incubation period, 12.5μl of DeepBlueC at 20μM (also called Coe1400a) was added in each well (final concentration of 5μM). Final assay volume was 50μl. Donor and acceptor light outputs were quantified with the Fusion microtiterplate analyzer (Packard Instrument Company) using the following instrument setting:

PMT: 1100volts
Gain: 100
Temperature: 25°C
Dual luminescence mode
Read length : 1sec per well per filter.
Filters:

Donor:       D410/80m (from Chroma Technology Corp.)

Acceptor:    D515/30m (from Chroma Technology Corp)

Results represent the mean +/- SEM of quadruplicate wells. The graph of Figure 31 was generated using GraphPad PRISM software using experimental data at the 10min time point after addition of DeepBlueC. In all cases, background levels (wells with only DBC in PBS+) were subtracted. Numbers above bars represent fold increased (condition +AVP divided by -AVP).

Figure 31 demonstrates that both configurations of the β-arrestin2 fusion constructs (β-arrestin2:GFP1 and GFP1:βarrestin2) generated a BRET signal when co-transfected with V2-R:Rluc fusion construct upon addition of the V2-R ligand AVP. Both configurations gave similar BRET signals (0.042) in absence of AVP (-AVP). In presence of AVP (+AVP) GFP1:β-arrestin2 fusion construct generated a higher BRET signal compared to the other configuration (0.129 vs 0.087). Numbers above bars represent the fold increase in BRET signals in presence of AVP over in absence of AVP for a specific condition. The example shows the structural versatility of BRET assay.

## Table 1

| | Coelenterazine | Donor | Acceptor |
|---|---|---|---|
| BRET | wt, h | Rluc | EYFP or Topaz |
| BRET$^2$ | Coel$_{400}$A | Rluc | GFPwt, Sph or GFPuv |

# Table 2

| BDP | Substrate | Substrate wavelength (peak)* | FAM | Acceptor Wavelengths (Ex/Em) |
|---|---|---|---|---|
| Rluc | hcp coelenterazine | 445 | CFP or ECFP | 443 (453)/ 475(501) nm |
| Rluc | Wt coelenterazine | 480 | dsRED | 558 (480)/ 583 nm |
| Rluc | Coelenterazine Wild type | 470nm | Fluorescein | 490/525 nm |
| Rluc | Coelenterazine Wild type | 470nm | Acridine yellow | 470/550 nm |
| Rluc | Coelenterazine Wild type | 470nm | Nile red | 485/525 nm |
| Rluc | Coelenterazine cp | 442nm | Lucifer yellow | 428/540 nm |
| Rluc | Coelenterazine 400 | 400nm | Quin-2 | 365/490 nm |
| Rluc | Coelenterazine 400 | 400nm | Dansyl chloride | 380/475 nm |
| Firefly luciferase | luciferin | 560nm | Texas red | 590/615 nm |

# Table 3

| Species | Name | Organism | MW kDa × 10⁻³ | Emission (nm) | Substrate |
|---|---|---|---|---|---|
| Insect | FFluc | Photinus pyralis (north american Firefly) | ~61 | 560 | Benzothiazole (luciferin) |
| Insect | FF'luc | Luciola cruciata (Japanese Firefly) | | 560-590 (many mutants) | Luciferin |
| Insect | Clluc | Pyrophorus plagiophthalamus (click beetle) | | 546, 560, 578 and 593 | Luciferin |
| Jellyfish | Aequorin | Aequorea | 44.9 | 460-470 | Coelenterazine |
| Sea pansy | Rluc | Renilla Reniformis | 36 | 480 | Coelenterazine |
| Sea pansy | Rmluc | Renilla mullerei | 36.1 | ~480 | Coelenterazine |
| Sea pansy | | Renilla kollikeri | | | |
| Crustacea (shrimp) | Vluc | Vargula hilgendorfil* | ~62 | ~460 | coelenterazine** |
| Crustacea | | Cypridina (sea firefly) | 75 | 460 | coelenterazine** |
| Dino-flagellate (marine alga) | | Gonyaulax polyedra | 130 | ~475 | Tetrapyrrole |
| Mollusc | | Latia (fresh water limpet) | 170 | 500 | Enol formate, terpene, aldehyde |

| Species | Name | Organism | MW kDa x 10⁻³ | Emission (nm) | Substrate |
|---|---|---|---|---|---|
| Hydroid | | Obelia bicuspidata | ~20 | ~470 | Coelenterazine |
| Shrimp | | Oplophorus gracilorostris | 31 | 462 | Coelenterazine |
| Others | Ptluc | Ptilosarcus | | ~490 | Coelenterazine |
| | Gluc | Gaussia | ~20 | ~475 | Coelenterazine |
| | Plluc | Pleuromamma | 22.6 | ~475 | Coelenterazine |

## Table 4

| GFP name | Mutations |
|---|---|
| GFP12 (h) ** | |
| GFP1 (h) | F64L |
| GFP8 (h) | F64L S202F, T203I |
| GFP9 (h) | F64L S202F, T203I S147P |
| EGFP⁴ | F64L S65T |
| GFPuv | F99S M153T V163A |
| GFPuv (h) | F99S M153T V163A |
| GFP4 (h) | F99S M153T V163A F64L |
| GFP5 (h) | F99S M153T V163A F64L S202F T203I |
| GFP7 (h) | F99S M153T V163A F64L S202F T203I S147P |
| GFP10 | F64L S202F |

## Table 5

| Mutation | Effect |
|---|---|
| F64L | increase solubility |
| S72A | presumably helps the folding, less effective than F64L (patented) |
| S147P | improve fluorescence at 37°C |
| S175G | increase thermo-tolerance |
| S202F | attenuate the second excitation peak (470nm) and photoisomerization |
| T203I | attenuate the second excitation peak (470nm) and photoisomerization |

| F99S | one of the three mutations found in GFPuv |
|---|---|
| M153T | one of the three mutations found in GFPuv |
| V163A | one of the three mutations found in GFPuv; increase thermo-tolerance |

## Table 6

| Constructs | Intensity* (CPS) at 400nm | Intensity* (CPS) at 510nm | Intensity* (CPS) open spectrum |
|---|---|---|---|
| Rluc (pRL-CMV) | 1933 | 738 | 9153 |
| Rluc::GFPuv (pcDNA3.1/Rluc::GFPuv) | 1008 | 944 | 15770 |

## Table 7

| Constructs | Ratio eq.1 | Ratio eq.2 | Ratio eq.3 | Ratio eq.4 |
|---|---|---|---|---|
| Rluc (pRL-CMV) | 0.382 | 2.619 | 0.0877 | 3.734 |
| Rluc::GFPuv (pcDNA3.1/Rluc::GFPuv) | 0.936 | 1.068 | 0.0637 | 14.637 |

## Table 8

| Rluc mutant | Bioluminescence (RLU) | Fold difference (compared to Rluc wild type) |
|---|---|---|
| Rluc wild type | 19204 | 1 |
| Rluc C124A | 55561 | 2.9 |
| Rluc C124S | 5838 | 0.3 |
| Rluc C124V | 788 | 0.04 |
| Rluc C124Y | 80 | 0.004 |

Table 9

| Parameters | Final quantity or final concentration (first amplification) |
|---|---|
| DNA template | 10ng |
| *PFU* buffer 10X (from Stratagene) | 1X |
| DMSO | 5% (final) |
| DNTPs 10mM | 250µM |
| Primer sense (1mM) | 500µM |
| Primer antisense (1mM) | 500µM |
| Milli-Q grade water | complete to 50µl |
| *PFU enzyme** | 2.5U |

● added during hot start step

## Table 10

| Parameters | Final quantity or final concentration (first amplification) | Final quantity or final concentration (second amplification) |
|---|---|---|
| DNA template | 2 μl (single-strand cDNA) | 2μl (from the first amplification) |
| *PFU* buffer 10X (from Stratagene) | 1X | 1X |
| DMSO | 5% (final) | 5% (final) |
| dNTPs 10mM | 250μM | 250μM |
| Primer sense (1mM) | 500μM | 500μM |
| Primer antisense (1mM) | 500μM | 500μM |
| Milli-Q grade water | complete to 50μl | complete to 50μl |
| *PFU enzyme** | 2.5U | 2.5U |

**ADDED DURING HOT START STEP.**

## Table 11

| Parameters | Final quantity or final concentration (first amplification) | Final quantity or final concentration (second amplification) |
|---|---|---|
| DNA template | 2 µl | 2 µl |
| PFU buffer 10 × (from Stratagene) | 1 × | 1 × |
| DMSO | 5% (final) | 5% (final) |
| dNTPs 10mM | 250 µM | 250 µM |
| Primer sense (1mM) | 500 µM | 500 µM |
| Primer antisense (1mM) | 500 µM | 500 µM |
| Milli-Q grade water | complete at 50 µl | complete at 50 µl |
| PFU enzyme* | 2.5 U | 2.5 U |

*added during hot start step.

## Table 12

| Parameters | Final quantity or final concentration (first amplification) |
|---|---|
| DNA template | 10 ng |
| PFU buffer 10 × (from Stratagene) | 1 × |
| DNTPs 10 mM | 250 µM |
| Primer sense (1mM) | 500 µM |
| Primer antisense (1mM) | 500 µM |
| Milli-Q grade water | Complete at 50 µl |
| PFU enzyme* | 2.5 U |

*added during hot start step.

## Table 13

| Parameters | Final quantity or final concentration (first amplification) |
|---|---|
| DNA template | 10ng |
| PFU buffer 10 × (from Stratagene) | 1 × |
| dNTPs 10 mM | 250 µM |
| Primer sense (1 mM) | 500 µM |
| Primer antisense (1 mM) | 500 µM |
| Milli-Q grade water | Complete at 50 µl |
| PFU enzyme* | 2.5 U |

*added during hot start step.

### Claims

1. A bioluminescence resonance energy transfer (BRET) system comprising:

   (a) a bioluminescent donor protein (BDP) attached to a first molecule or modulator;
   (b) a fluorescent acceptor molecule (FAM) attached to a second molecule or modulator, wherein said FAM can accept the energy from the BDP when they are associated, in the presence of the appropriate substrate; and

   wherein a physical change in the modulator(s) influences the energy transfer efficiency between the BDP and the FAM and wherein said system has a broad spectral resolution of at least 80 nm between the peaks of the BDP and FAM emission spectra.

2. The BRET system according to claim 1, wherein the modulator comprises two separate molecular entities attached to the FAM and BDP, respectively.

3. The BRET system according to claim 2, wherein the two separate molecular entities are the same.

4. The BRET system according to claim 2, wherein the two separate molecular entities are distinct.

5. The BRET system according to any one of claims 2, 3 or 4 wherein one or both of the molecular entities is a receptor, receptor subunit or fragment thereof.

6. The BRET system according to claim 5, wherein said receptor is an orphan receptor.

7. The BRET system according to claim 2 or claim 4, wherein one of the molecular entities is a receptor binding molecule that will bind to an activated receptor and the other is a receptor, receptor subunit or fragment thereof.

8. The BRET system according to claim 7 wherein said receptor binding molecule is β-arrestin, a G-protein or ubiquitin.

9. The BRET system according to claim 1 wherein the modulator, FAM and BDP comprise one fusion molecule.

10. The BRET system according to claim 2 or claim 9, wherein interaction between the modulator, FAM and BDP is dependent on the activity of another molecule.

11. The BRET system according to claim 2, wherein interaction between said molecules is constitutive.

12. The BRET system according to claim 1, additionally comprising an instrument for detecting BRET signal capable of determining specifically BDP and/or FAM emissions.

13. The BRET system according to claim 12, wherein one or more optical filters are incorporated into said instrument to reduce overlap between said emission spectra and produce said broad spectral resolution.

14. The BRET system according to claim 12, wherein the instrument is a microplate plate reader, or an imaging system.

15. The BRET system according to claim 12, wherein one or more monochromators or prisms are incorporated into said instrument to reduce overlap between said emission spectra and produce said broad spectral resolution.

16. The BRET system according to claim 12, wherein the instrument is spectrophotometer or a spectrofluorometer.

17. The BRET system according to claim 12, wherein an algorithm is used to mathematically subtract BDP emission present in the FAM peak and produce said broad spectral resolution.

18. The BRET system according to claim 1, wherein said BDP has luciferase activity in the presence of an appropriate substrate.

19. The BRET system according to claim 18, wherein said luciferase is *Renilla* luciferase or firefly luciferase, Gaussia luciferase, Aequorin.

20. The BRET system according to claim 19, wherein a broad spectral resolution is attained by using a non-naturally occurring luciferase.

21. The BRET system according to claim 1, additionally comprising an appropriate substrate to activate the luminescent activity of the BDP.

22. The BRET system according to claim 1, wherein said broad spectral resolution is attained by using a non-naturally occurring substrate.

23. The BRET system according to claim 22, wherein said substrate is a derivative of coelenterazine.

24. The BRET system according to claim23, wherein said derivative of coelenterazine is coe1400a.

25. The BRET system according to any one of claims 1, 12, 20, or 22 wherein said broad spectral resolution is attained by using a non-naturally occurring FPAM.

26. The BRET system according to claim 25, wherein said FAM is a mutant green fluorescent protein or a mutant red fluorescent protein.

27. The BRET system according to claim 26, wherein said mutant green fluorescent protein comprises the following substitutions:

    (a) phenylalanine-64 to leucine;
    (b) phenylalanine-64 to leucine, serine-147 to proline;
    (c) phenylalanine-64 to leucine and serine-202 to phenylalanine
    (d) phenylalanine-64 to leucine, serine-147 to proline, and serine-202 to phenylalanine;
    (e) phenylalanine-64 to leucine, serine-147 to proline, and tyrosine-203 to isoleucine; or

(f) phenylalanine-64 to leucine, serine-147 to proline, serine-202 to phenylalanine and tyrosine-203 to isoleucine.

**28.** Use of the BRET system of claim 1 to monitor protein-protein interactions *in vitro.*

**29.** A method of producing a BRET system as in claim 1 comprising:

(a) selecting a bioluminescent donor protein (BDP) and a fluorescent acceptor molecule (FAM) such that the FAM can accept the energy from the BDP when they are associated, in the presence of the appropriate substrate; and
(b) either

(i) using an algorithm to mathematically subtract BDP emission from the FAM peak,
(ii) reducing overlap between the BDP and FAM emission spectra by using optical filters,
(iii) using site-directed mutagenesis to alter the emission spectrum of the BDP,
(iv) using a coelenterazine derivative, luciferin or bioluminescent substrate that causes a shift in the BDP emission spectrum, or
(v) using site-directed mutagenesis to alter the excitation and/or emission spectrum of the FAM,

wherein said system has a broad spectral resolution of at least 80nm between the peaks of the BDP and FAM emission spectra.

**Patentansprüche**

**1.** Biolumineszenz-Resonanz-Energie-Transfer (BRET) -System, das Folgendes aufweist:

(a) ein biolumineszierendes Donorprotein (BDP), das an ein erstes Molekül oder einen ersten Modulator angelagert ist;
(b) ein fluoreszierendes Akzeptormolekül (FAM), das an ein zweites Molekül oder einen zweiten Modulator angelagert ist, wobei das FAM in Gegenwart eines geeigneten Substrats die Energie von dem BDP entgegennehmen kann, wenn sie miteinander in Verbindung gebracht sind, und wobei eine physikalische Veränderung bei dem (den) Modulator(en) die Effizienz des Energietransfers zwischen dem BDP und dem FAM beeinflusst und wobei das System eine große spektrale Auflösung von mindestens 80 nm zwischen den Peaks des BDP-Emissionsspektrums und des FAM-Emissionsspektrums aufweist.

**2.** BRET-System nach Anspruch 1, wobei der Modulator zwei getrennte Moleküleinheiten aufweist, die an das FAM bzw. an das BDP angelagert sind.

**3.** BRET-System nach Anspruch 2, wobei die zwei getrennte Moleküleinheiten gleich sind.

**4.** BRET-System nach Anspruch 2, wobei die zwei getrennten Moleküleinheiten verschieden sind.

**5.** BRET-System nach einem der Ansprüche 2, 3 oder 4, wobei eine der Moleküleinheiten ein Rezeptor, eine Rezeptor-Untereinheit oder ein Fragment davon ist oder beide Moleküleinheiten Rezeptoren, Rezeptor-Untereinheiten oder Fragmente davon sind.

**6.** BRET-System nach Anspruch 5, wobei der Rezeptor ein Orphan-Rezeptor ist.

**7.** BRET-System nach Anspruch 2 oder Anspruch 4, wobei eine der Moleküleinheiten ein rezeptorbindendes Molekül ist, das an einen aktivierten Rezeptor binden wird, während die andere ein Rezeptor, eine Rezeptor-Untereinheit oder ein Fragment davon ist.

**8.** BRET-System nach Anspruch 7, wobei das rezeptorbindende Molekül β-Arrestin, ein G-Protein oder Ubiquitin ist.

**9.** BRET-System nach Anspruch 1, wobei der Modulator, das FAM und das BDP ein Fusionsmolekül umfassen.

**10.** BRET-System nach Anspruch 2 oder Anspruch 9, wobei eine Interaktion zwischen dem Modulator, dem FAM und dem BDP von der Aktivität eines weiteren Moleküls abhängig ist.

11. BRET-System nach Anspruch 2, wobei die Interaktion zwischen den Molekülen konstitutiv ist.

12. BRET-System nach Anspruch 1, das außerdem ein Gerät zur BRET-Signal-Erfassung aufweist, das fähig ist, speziell BDP und/oder FAM-Emissionen zu bestimmen.

13. BRET-System nach Anspruch 12, wobei eine oder mehrere optische Filter in das Gerät eingebaut sind, um die Überlappung zwischen den Emissionsspektren zu vermindern und eine große spektrale Auflösung zu erzeugen.

14. BRET-System nach Anspruch 12, wobei das Gerät ein Mikroplattenleser oder ein Abbildungssystem ist.

15. BRET-System nach Anspruch 12, wobei in das Gerät ein oder mehrere Monochromatoren oder Prismen eingebaut sind, um die Überlappung zwischen den Emissionsspektren zu vermindern und eine große spektrale Auflösung zu erzeugen.

16. BRET-System nach Anspruch 12, wobei das Gerät ein Spektrophotometer oder ein Spektrofluorometer ist.

17. BRET-System nach Anspruch 12, wobei ein Algorithmus benutzt wird, um rechnerisch die in dem FAM-Peak vorhandene BDP-Emission abzuziehen und die große spektrale Auflösung zu erzeugen.

18. BRET-System nach Anspruch 1, wobei das BDP in Gegenwart eines geeigneten Substrats Luciferaseaktivität aufweist.

19. BRET-System nach Anspruch 18, wobei die Luciferase *Renilla*-Luciferase oder Leuchtkäfer-Luciferase, Gaussia-Luciferase, Aequorin ist.

20. BRET-System nach Anspruch 19, wobei eine große spektrale Auflösung durch Verwendung einer nicht natürlich vorkommenden Luciferase erreicht wird.

21. BRET-System nach Anspruch 1, welches zusätzlich ein geeignetes Substrat, das die Lumineszenzaktivität des BDP aktiviert, aufweist.

22. BRET-System nach Anspruch 1, wobei die große spektrale Auflösung durch Verwenden eines nicht natürlich vorkommenden Substrats erreicht wird.

23. BRET-System nach Anspruch 22, wobei das Substrat ein Derivat von Coelenterazin ist.

24. BRET-System nach Anspruch 23, wobei das Coelenterazin-Derivat coe1400a ist.

25. BRET-System nach einem der Ansprüche 1, 12, 20 oder 22, wobei die große spektrale Auflösung durch Verwenden eines nicht natürlich vorkommenden FAM erreicht wird.

26. BRET-System nach Anspruch 25, wobei das FAM ein mutiertes grün fluoreszierendes Protein oder ein mutiertes rot fluoreszierendes Protein ist.

27. BRET-System nach Anspruch 26, wobei das mutierte grün fluoreszierende Protein die folgenden Substitutionen aufweist:

    (a) Phenylalanin-64 zu Leucin,
    (b) Phenylalanin-64 zu Leucin, Serin-147 zu Prolin;
    (c) Phenylalanin-64 zu Leucin und Serin-202 zu Phenylalanin;
    (d) Phenylalanin-64 zu Leucin, Serin-147 zu Prolin und Serin-202 zu Phenylalanin;
    (e) Phenylalanin-64 zu Leucin, Serin-147 zu Prolin und Tyrosin-203 zu Isoleucin; oder
    (f) Phenylalanin-64 zu Leucin, Serin-147 zu Prolin, Serin-202 zu Phenylalanin und Tyrosin-203 in Isoleucin.

28. Verwendung des BRET-Systems nach Anspruch 1, um Protein-Protein-Interaktionen *in vitro* zu überwachen.

29. Verfahren zum Herstellen eines BRET-Systems nach Anspruch 1, das folgende Schritte aufweist:

(a) Auswählen eines biolumineszierenden Donorproteins (BDP) und eines fluoreszierenden Akzeptormoleküls (FAM), derart, dass das FAM in Gegenwart eines geeigneten Substrats die Energie von dem BDP entgegennehmen kann, wenn sie miteinander in Verbindung gebracht sind; und
(b) entweder

(i) Anwenden eines Algorithmus, um rechnerisch die BDP-Emission von dem FAM-Peak abzuziehen,
(ii) Vermindern der Überlappung zwischen dem BDP-Emissionsspektrum und dem FAM-Emissionsspektrum mit optischen Filtern;
(iii) Anwenden einer ortsspezifischen Mutagenese, um das Emissionsspektrum des BDP zu verändern;
(iv) Verwenden eines Coelenterazin-Derivat-, Luciferin- oder biolumineszierenden Substrats, das eine Verschiebung bei dem BDP-Emissionsspektrum bewirkt; oder
(v) Anwenden einer ortsspezifischen Mutagenese, um die Anregung und/oder das Emissionsspektrum des FAM zu verändern,

wobei das System eine große spektrale Auflösung von mindestens 80 nm zwischen den Peaks des BDP-Emissionsspektrums und des FAM-Emissionsspektrums aufweist.

**Revendications**

1. Système de transfert d'énergie par résonance bioluminescente (BRET) comprenant :

(a) une protéine donneur bioluminescente (BDP) attachée à une première molécule ou modulateur;
(b) une molécule accepteur fluorescente (FAM) attachée à une deuxième molécule ou modulateur, ladite FAM pouvant accepter l'énergie provenant de la BDP lorsqu'elles sont associées, en présence d'un substrat approprié; et

dans lequel une modification physique du(des) modulateur(s) influence l'efficacité du transfert d'énergie entre la BDP et la FAM et ledit système présentant une large résolution spectrale d'au moins 80 nm entre les pics des spectres d'émission de la BDP et de la FAM.

2. Système de BRET selon la revendication 1, dans lequel le modulateur comprend deux entités moléculaires séparées attachées à la FAM et à la BDP, respectivement.

3. Système de BRET selon la revendication 2, dans lequel les deux entités moléculaires séparées sont identiques.

4. Système de BRET selon la revendication 2, dans lequel les deux entités moléculaires séparées sont distinctes.

5. Système de BRET selon l'une quelconque des revendications 2, 3 ou 4, dans lequel l'une des entités moléculaires, ou les deux, est un récepteur, une sous-unité d'un récepteur ou un fragment de celui-ci.

6. Système de BRET selon la revendication 5, dans lequel ledit récepteur est un récepteur orphelin.

7. Système de BRET selon la revendication 2 ou la revendication 4, dans lequel l'une des entités moléculaires est une molécule de liaison à un récepteur qui se liera à un récepteur activé et l'autre est un récepteur, une sous-unité d'un récepteur ou un fragment de celui-ci.

8. Système de BRET selon la revendication 7, dans lequel ladite molécule de liaison à un récepteur est la β-arrestine, une protéine G ou l'ubiquitine.

9. Système de BRET selon la revendication 1, dans lequel le modulateur, la FAM et la BDP comprennent une molécule de fusion.

10. Système de BRET selon la revendication 2 ou la revendication 9, dans lequel l'interaction entre le modulateur, la FAM et la BDP dépend de l'activité d'une autre molécule.

11. Système de BRET selon la revendication 2, dans lequel l'interaction entre lesdites molécules est constitutive.

**12.** Système de BRET selon la revendication 1, comprenant en outre un dispositif de détection du signal de BRET pouvant déterminer de manière spécifique les émissions de la BDP et/ou de la FAM.

**13.** Système de BRET selon la revendication 12, dans lequel on inclut un ou plusieurs filtres optiques dans ledit dispositif pour diminuer le recouvrement entre lesdits spectres d'émission et produire ladite large résolution spectrale.

**14.** Système de BRET selon la revendication 12, dans lequel le dispositif est un lecteur de microplaques ou un système d'imagerie.

**15.** Système de BRET selon la revendication 12, dans lequel on inclut un ou plusieurs monochromateurs ou prismes dans ledit dispositif pour diminuer le recouvrement entre lesdits spectres d'émission et produire ladite large résolution spectrale.

**16.** Système de BRET selon la revendication 12, dans lequel le dispositif est un spectrophotomètre ou un spectrofluorimètre.

**17.** Système de BRET selon la revendication 12, dans lequel on utilise un algorithme pour soustraire mathématiquement l'émission de la BDP présente dans le pic de la FAM et produire ladite large résolution spectrale.

**18.** Système de BRET selon la revendication 1, dans lequel ladite BDP présente une activité luciférase en présence d'un substrat approprié.

**19.** Système de BRET selon la revendication 18, dans lequel ladite luciférase est la luciférase de *Renilla* ou la luciférase de luciole, la luciférase de *Gaussia,* l'aequorine.

**20.** Système de BRET selon la revendication 19, dans lequel on atteint une large résolution spectrale en utilisant une luciférase qui n'existe pas dans la nature.

**21.** Système de BRET selon la revendication 1, comprenant en outre un substrat approprié pour activer l'activité luminescente de la BDP.

**22.** Système de BRET selon la revendication 1, dans lequel on atteint ladite large résolution spectrale en utilisant un substrat qui n'existe pas dans la nature.

**23.** Système de BRET selon la revendication 22, dans lequel ledit substrat est un dérivé de la coelentérazine.

**24.** Système de BRET selon la revendication 23, dans lequel ledit dérivé de la coelentérazine est le coe1400a.

**25.** Système de BRET selon l'une quelconque des revendications 1, 12, 20 ou 22, dans lequel on atteint ladite large résolution spectrale en utilisant une FAM qui n'existe pas dans la nature.

**26.** Système de BRET selon la revendication 25, dans lequel ladite FAM est une protéine fluorescente verte mutante ou une protéine fluorescente rouge mutante.

**27.** Système de BRET selon la revendication 26, dans lequel ladite protéine fluorescente verte mutante comprend les substitutions suivantes :

(a) la phénylalanine-64 en leucine;
(b) la phénylalanine-64 en leucine, la sérine-147 en proline;
(c) la phénylalanine-64 en leucine et la sérine-202 en phénylalanine;
(d) la phénylalanine-64 en leucine, la sérine-147 en proline et la sérine-202 en phénylalanine;
(e) la phénylalanine-64 en leucine, la sérine-147 en proline et la tyrosine-203 en isoleucine; ou
(f) la phénylalanine-64 en leucine, la sérine-147 en proline, la sérine-202 en phénylalanine et la tyrosine-203 en isoleucine.

**28.** Utilisation du système de BRET selon la revendication 1 pour surveiller les interactions protéine-protéine *in vitro.*

**29.** Procédé de production d'un système de BRET selon la revendication 1 comprenant :

(a) la sélection d'une protéine donneur bioluminescente (BDP) et d'une molécule accepteur fluorescente (FAM) de sorte que la FAM puisse accepter l'énergie provenant de la BDP lorsqu'elles sont associées, en présence d'un substrat approprié; et

(b) soit

(i) l'utilisation d'un algorithme pour soustraire mathématiquement l'émission de la BDP du pic de la FAM,

(ii) la diminution du recouvrement entre les spectres d'émission de la BDP et de la FAM en utilisant des filtres optiques,

(iii) l'utilisation de la mutagenèse dirigée pour modifier le spectre d'émission de la BDP,

(iv) l'utilisation d'un dérivé de la coelentérazine, de la luciférine ou d'un substrat bioluminescent qui provoque un déplacement dans le spectre d'émission de la BDP, soit

(v) l'utilisation de la mutagenèse dirigée pour modifier le spectre d'excitation et/ou d'émission de la FAM,

dans lequel ledit système présente une large résolution spectrale d'au moins 80 nm entre les pics des spectres d'émission de la BDP et de la FAM.

FIGURE 1

FIGURE 2

470-480nm

525-530nm

Energy transfer
(non-radiative)

Rluc

site

EYFP

Coelenterazine

Coelenteramide

FIGURE 3

FIGURE 4

FIGURE 5

FIGURE 6

FIGURE 7

**FIGURE 8**

**FIGURE 9**

FIGURE 10

FIGURE 11

**FIGURE 12**

FIGURE 13

FIGURE 14

**FIGURE 15**

**FIGURE 16**

a)

Time after addition of coelenterazine
(min)

b)

Time after addition of coelenterazine
(min.)

FIGURE 17

FIGURE 18

**FIGURE 19**

**FIGURE 20**

FIGURE 21

A

B

FIGURE 22

Figure 23

Figure 24

Figure 25

**FIGURE 26**

FIGURE 27

```
ATGGTGAGCA AGGGCGAGGA GCTGTTCACC GGGGTGGTGC CCATCCTGGT CGAGCTGGAC
GGCGACGTAA ACGGCCACAA GTTCAGCGTG TCCGGCGAGG GCGAGGGCGA TGCCACCTAC
GGCAAGCTGA CCCTGAAGTT CATCTGCACC ACCGGCAAGC TGCCCGTGCC CTGGCCCACC
CTCGTGACCA CCCTGAGCTA CGGCGTGCAG TGCTTCAGCC GCTACCCCGA CCACATGAAG
CAGCACGACT TCTTCAAGTC CGCCATGCCC GAAGGCTACG TCCAGGAGCG CACCATCTTC
TTCAAGGACG ACGGCAACTA CAAGACCCGC GCCGAGGTGA AGTTCGAGGG CGACACCCTG
GTGAACCGCA TCGAGCTGAA GGGCATCGAC TTCAAGGAGG ACGGCAACAT CCTGGGGCAC
AAGCTGGAGT ACAACTACAA CAGCCACAAC GTCTATATCA TGGCCGACAA GCAGAAGAAC
GGCATCAAGG TGAACTTCAA GATCCGCCAC AACATCGAGG ACGGCAGCGT GCAGCTCGCC
GACCACTACC AGCAGAACAC CCCCATCGGC GACGGCCCCG TGCTGCTGCC CGACAACCAC
TACCTGAGCA CCCAGTCCGC CCTGAGCAAA GACCCCAACG AGAAGCGCGA TCACATGGTC
CTGCTGGAGT TCGTGACCGC CGCCGGGATC ACTCTCGGCA TGGACGAGCT GTACAAGTCC
GGATCAAGCT TGCGGTACCG CGGGCCCTCT AGAGCCACCA TGACCAGCAA GGTGTACGAC
CCCGAGCAGA GGAAGAGGAT GATCACCGGC CCCCAGTGGT GGGCCAGGTG CAAGCAGATG
AACGTGCTGG ACAGCTTCAT CAACTACTAC GACAGCGAGA AGCACGCCGA GAACGCCGTG
ATCTTCCTGC ACGGCAACGC CGCTAGCAGC TACCTGTGGA GGCACGTGGT GCCCCACATC
GAGCCCGTGG CCAGGTGCAT CATCCCCGAT CTGATCGGCA TGGGCAAGAG CGGCAAGAGC
GGCAACGGCA GCTACAGGCT GCTGGACCAC TACAAGTACC TGACCGCCTG GTTCGAGCTC
CTGAACCTGC CCAAGAAGAT CATCTTCGTG GGCCACGACT GGGGCGCCTG CCTGGCCTTC
CACTACAGCT ACGAGCACCA GGACAAGATC AAGGCCATCG TGCACGCCGA GAGCGTGGTG
GACGTGATCG AGAGCTGGGA CGAGTGGCCA GACATCGAGG AGGACATCGC CCTGATCAAG
AGCGAGGAGG CGGAGAAGAT GGTGCTGGAG AACAACTTCT TCGTGGAGAC CATGCTGCCC
AGCAAGATCA TGAGAAAGCT GGAGCCCGAG GAGTTCGCCG CCTACCTGGA GCCCTTCAAG
GAGAAGGGCG AGGTGAGAAG ACCCACCCTG AGCTGGCCCA GAGAGATCCC CCTGGTGAAG
GGCGGCAAGC CCGACGTGGT GCAGATCGTG AGAAACTACA ACGCCTACCT GAGAGCCAGC
GACGACCTGC CCAAGATGTT CATCGAGAGC GACCCCGGCT TCTTCAGCAA CGCCATCGTG
GAGGGCGCCA AGAAGTTCCC CAACACCGAG TTCGTGAAGG TGAAGGGCCT GCACTTCAGC
CAGGAGGACG CCCCCGACGA GATGGGCAAG TACATCAAGA GCTTCGTGGA GAGAGTGCTG
AAGAACGAGC AGTAA
```

# FIGURE 28

```
ATGGTGAGCA AGGGCGAGGA GCTGTTCACC GGGGTGGTGC CCATCCTGGT CGAGCTGGAC
GGCGACGTAA ACGGCCACAA GTTCAGCGTG TCCGGCGAGG GCGAGGGCGA TGCCACCTAC
GGCAAGCTGA CCCTGAAGTT CATCTGCACC ACCGGCAAGC TGCCCGTGCC CTGGCCCACC
CTCGTGACCA CCCTGAGCTA CGGCGTGCAG TGCTTCAGCC GCTACCCCGA CCACATGAAG
CAGCACGACT TCTTCAAGTC CGCCATGCCC GAAGGCTACG TCCAGGAGCG CACCATCTTC
TTCAAGGACG ACGGCAACTA CAAGACCCGC GCCGAGGTGA AGTTCGAGGG CGACACCCTG
GTGAACCGCA TCGAGCTGAA GGGCATCGAC TTCAAGGAGG ACGGCAACAT CCTGGGGCAC
AAGCTGGAGT ACAACTACAA CAGCCACAAC GTCTATATCA TGGCCGACAA GCAGAAGAAC
GGCATCAAGG TGAACTTCAA GATCCGCCAC AACATCGAGG ACGGCAGCGT GCAGCTCGCC
GACCACTACC AGCAGAACAC CCCCATCGGC GACGGCCCCG TGCTGCTGCC CGACAACCAC
TACCTGAGCA CCCAGTCCGC CCTGAGCAAA GACCCCAACG AGAAGCGCGA TCACATGGTC
CTGCTGGAGT TCGTGACCGC CGCCGGGATC ACTCTCGGCA TGGACGAGCT GTACAAGTCC
GGATCAAGCT TGGGCGACGA GGTGGACGGC GGGCCCTCTA GAGCCACCAT GACCAGCAAG
GTGTACGACC CCGAGCAGAG GAAGAGGATG ATCACCGGCC CCCAGTGGTG GGCCAGGTGC
AAGCAGATGA ACGTGCTGGA CAGCTTCATC AACTACTACG ACAGCGAGAA GCACGCCGAG
AACGCCGTGA TCTTCCTGCA CGGCAACGCC GCTAGCAGCT ACCTGTGGAG GCACGTGGTG
CCCCACATCG AGCCCGTGGC CAGGTGCATC ATCCCCGATC TGATCGGCAT GGGCAAGAGC
GGCAAGAGCG GCAACGGCAG CTACAGGCTG CTGGACCACT ACAAGTACCT GACCGCCTGG
TTCGAGCTCC TGAACCTGCC CAAGAAGATC ATCTTCGTGG CCACGACTG GGGCGCCTGC
CTGGCCTTCC ACTACAGCTA CGAGCACCAG GACAAGATCA AGGCCATCGT GCACGCCGAG
AGCGTGGTGG ACGTGATCGA GAGCTGGGAC GAGTGGCCAG ACATCGAGGA GGACATCGCC
CTGATCAAGA GCGAGGAGGG CGAGAAGATG GTGCTGGAGA ACAACTTCTT CGTGGAGACC
ATGCTGCCCA GCAAGATCAT GAGAAAGCTG GAGCCCGAGG AGTTCGCCGC CTACCTGGAG
CCCTTCAAGG AGAAGGGCGA GGTGAGAAGA CCCACCCTGA GCTGGCCCAG AGAGATCCCC
CTGGTGAAGG GCGGCAAGCC CGACGTGGTG CAGATCGTGA GAAACTACAA CGCCTACCTG
AGAGCCAGCG ACGACCTGCC CAAGATGTTC ATCGAGAGCG ACCCCGGCTT CTTCAGCAAC
GCCATCGTGG AGGGCGCCAA GAAGTTCCCC AACACCGAGT TCGTGAAGGT GAAGGGCCTG
CACTTCAGCC AGGAGGACGC CCCCGACGAG ATGGGCAAGT ACATCAAGAG CTTCGTGGAG
AGAGTGCTGA AGAACGAGCA GTAA
```

# FIGURE 29

—■— GFP::Rluc (+ inducer)

—□— GFP:Rluc (- inducer)

—●— GFP:caspase-3:Rluc (+ inducer)

—○— GFP:caspase-3:Rluc (-Inducer)

Time after addition of DeepBlueC (min)

—▽— Rluc:p53 (-)  $Ca^{2+}$

—▽— Rluc:p53 (+)  $Ca^{2+}$

—■— Rluc:p53 + S100b:GFPuv (1:3) (-)  $Ca^{2+}$

—□— Rluc:p53 + S100b:GFPuv (1:3) (+)  $Ca^{2+}$

—▲— Rluc:p53 + S100b:GFPuv (1:1) (-)  $Ca^{2+}$

—△— Rluc:p53 + S100b:GFPuv (1:1) (+)  $Ca^{2+}$

FIGURE 30

FIGURE 31

## Schema I

Synthesis of aminopyrazine starting from chloropyrazine

**FIGURE 32**

Schema II

Synthesis of benzylglyoxal starting from phenylacetylchloride

benzylglyoxal

FIGURE 33

Schema III

EtOH, conc. HCl
80 C, 4.5 h

**FIGURE 34**